(19) Europäisches Patentamt
European Patent Office
Office européen des brevets

(11) Numéro de publication : **0 038 271**
**B1**

# (12) FASCICULE DE BREVET EUROPÉEN

(45) Date de publication du fascicule du brevet : **04.07.84**

(51) Int. Cl.³ : **C 07 C121/75**, A 23 K 1/16, A 61 K 31/22, A 01 N 53/00

(21) Numéro de dépôt : **81400611.0**

(22) Date de dépôt : **16.04.81**

(54) **Nouveaux dérivés de l'acide cyclopropane carboxylique, leur préparation, leur application à la lutte contre les parasites des végétaux et des animaux, les compositions les renfermant et les nouveaux intermédiaires obtenus.**

(30) Priorité : **16.04.80 FR 8008491**
**24.09.80 FR 8020478**

(43) Date de publication de la demande :
**21.10.81 Bulletin 81/42**

(45) Mention de la délivrance du brevet :
**04.07.84 Bulletin 84/27**

(84) Etats contractants désignés :
**AT BE CH DE FR GB IT LI LU NL SE**

(56) Documents cités :
**FR-A- 2 100 835**
**FR-A- 2 143 820**
**FR-A- 2 185 612**
**JOURNAL OF THE CHEMICAL SOCIETY, Perkin Transactions I, 1974 M. ELLIOTT et al.: "The pyrethrins and related compounds. Part XVIII. Insecticidal 2,2-dimethylcyclopropanecarboxylates with new unsaturated 3-substituents", pages 2470-2474**

(73) Titulaire : **ROUSSEL-UCLAF**
**35, boulevard des Invalides**
**F-75007 Paris (FR)**

(72) Inventeur : **Martel, Jacques**
**15, rue Douvillez**
**F-93140 Bondy (FR)**
Inventeur : **Tessier, Jean**
**30, rue Jean Moulin**
**F-94300 Vincennes (FR)**
Inventeur : **Teche, André**
**15, rue Godot de Mauroy**
**F-75009 Paris (FR)**

(74) Mandataire : **Vieillefosse, Jean-Claude et al**
**ROUSSEL-UCLAF Boîte postale no. 9**
**102, route de Noisy**
**F-93230 Romainville (FR)**

EP 0 038 271 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne de nouveaux dérivés de l'acide cyclopropane carboxylique, leur procédé de préparation, leur application à la lutte contre les parasites des végétaux et des animaux, les compositions les renfermant et les nouveaux intermédiaires obtenus.

L'invention a plus précisément pour objet des dérivés de l'acide cyclopropane carboxylique, portant en position 3 une chaîne latérale insaturée de géométrie Z et de structure A :

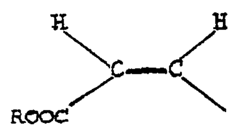

(A)

On connaissait certes des dérivés de l'acide cyclopropane carboxylique portant en position 3 une chaîne latérale de structure B :

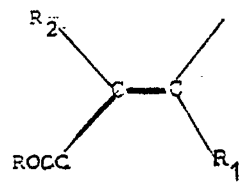

(B)

mais dont la géométrie était essentiellement E. Citons par exemple les dérivés de l'acide pyréthrique ($R = CH_3$, $R_1 = H$, $R_2 = CH_3$). Quelques dérivés pour lesquels $R_1 = R_2 = H$ ont également été décrits : Voir à ce sujet le brevet français 2.185.612 ainsi que J. Chem. Soc. Perkin I, page 2470, 1974 et Pest. Sci. *7,* page 499, 1976. Toutefois, la géométrie de la chaîne latérale de ces composés est E de façon prédominante. En effet, le procédé de synthèse utilisé pour préparer ces dérivés ne fournissait presqu'exclusivement que la géométrie E. (Voir Agr. Biol. Chem. *34,* page 1119, 1970). Pour ces composés, dont la géométrie de la chaîne latérale était essentiellement E, aucune propriété intéressante n'a pu être mise en évidence.

Or, on vient de découvrir que certains produits, dont la chaîne latérale insaturée est de structure A mais dont la géométrie est Z, présentent des propriétés pesticides inattendues, notamment des propriétés insecticides exceptionnelles.

L'invention a pour objet les composés de formule (I)

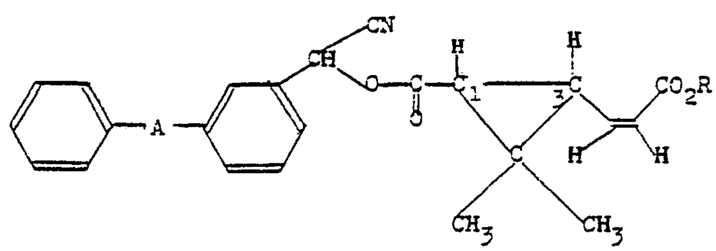

(I)

dans laquelle A représente un atome d'oxygène, un groupement méthylène ou un groupement carbonyle, et R représente un radical alcoyle linéaire, ramifié ou cyclique, saturé ou insaturé, renfermant de 1 à 8 atomes de carbone, dans laquelle la double liaison de la chaîne latérale en 3 a la géométrie Z, et la copule cyclopropanique est de structure IR cis sous toutes leurs formes stéréoisomères possibles, ainsi que les mélanges de ces stéréoisomères.

La formule (I) comporte 3 centres asymétriques, à savoir les carbones 1 et 3 du cyclopropane et le carbone portant la fonction nitrile. La formule I comporte donc 8 stéréoisomères.

Dans la formule I, R représente un radical alcoyle dérivant d'un alcool primaire, secondaire ou tertiaire.

Lorsque R représente un radical alcoyle cyclique, il s'agit de préférence d'un radical cyclopropyle, cyclobutyle, cyclopentyle ou cyclohexyle, d'un radical alcoyle linéaire ou ramifié portant un de ces radicaux ou d'un radical cyclopropyle, cyclobutyle, cyclopentyle ou cyclohexyle substitué par un ou plusieurs radicaux alcoyles, et dont la liaison avec le groupement —COO— est située sur l'un quelconque

de ses sommets, par exemple le radical 1-méthylcyclobutyle, 1-méthyl cyclopentyle, 1-méthylcyclohexyle ou 2,2,3,3,-tétraméthylcyclopropyle.

Lorsque R représente un radical alcoyle insaturé, il s'agit d'un radical éthylénique, comme par exemple le radical vinyle, allyle ou 1,1-diméthylallyle, ou d'un radical acétylénique comme par exemple le radical éthynyle ou propynyle.

Lorsque R représente un radical alcoyle saturé, linéaire ou ramifié, il s'agit de préférence d'un radical méthyle, éthyle, n-propyle, isopropyle, n-butyle, isobutyle, sec-butyle, tert-butyle, n-pentyle, tert-pentyle, néo-pentyle ou n-hexyle.

L'invention a plus particulièrement pour objet les composés de formule (I) pour lesquels A représente un atome d'oxygène.

L'invention a tout spécialement pour objet les composés de formule (I) pour lesquels la copule alcoolique de l'ester en position 1 du cyclopropane est le reste de l'alcool (S) α-cyano 3-phénoxybenzylique.

Parmi les composés préférés de l'invention, on peut citer tout particulièrement les composés de formule (I) pour lesquels R représente un radical méthyle, ainsi que ceux pour lesquels R représente un radical éthyle, propyle, isopropyle ou tert-butyle.

Parmi ces derniers composés, on peut citer tout particulièrement :

— le (1R cis) 2,2-diméthyl 3-/(Z) 2-(méthoxy carbonyl) éthényl/cyclopropane carboxylate de (S) α-cyano 3-phénoxybenzyle, ainsi que,

— le (1R cis) 2,2-diméthyl 3-/(Z) 2-(éthoxycarbonyl) éthényl/cyclopropane carboxylate de (S) α-cyano 3-phénoxy benzyle,

— le (1R cis) 2,2-diméthyl 3-/(Z) 2-(isopropyloxy carbonyl) éthényl/cyclopropane carboxylate de (S) α-cyano 3-phénoxy benzyle,

— le (1R cis) 2,2-diméthyl 3-/(Z) 2-(cyclopropylméthoxycarbonyl) éthényl/cyclopropane carboxylate de (S) α-cyano 3-phénoxybenzyle,

— le (1R cis) 2,2-diméthyl 3-/(Z) 2-(cyclopropyloxycarbonyl) éthényl/cyclopropane carboxylate de (S) α-cyano 3-phénoxybenzyle,

— le (1R cis) 2,2-diméthyl 3-/(Z) 2-(terbutoxycarbonyl) éthényl/cyclopropane carboxylate de (S) α-cyano 3-phénoxybenzyle.

Les composés de formule (I) présentent d'intéressantes propriétés qui permettent leur utilisation dans la lutte contre les parasites ; il peut s'agir par exemple de la lutte contre les parasites des végétaux, les parasites domestiques et les parasites des animaux à sang chaud. C'est ainsi que l'on peut utiliser les produits de l'invention pour lutter contre les insectes, les nématodes et les acariens parasites des végétaux et des animaux.

L'invention a donc pour objet l'application des composés de formule (I) tels que définis précédemment à la lutte contre les parasites des végétaux, les parasites domestiques, et les parasites des animaux à sang chaud.

Les produits de formule (I) peuvent donc être utilisés notamment pour lutter contre les insectes dans le domaine agricole, pour lutter par exemple contre les pucerons, les larves de lépidoptères et les coléoptères. Ils sont utilisés à des doses comprises entre 10 g et 300 g de matière active à l'hectare.

Les produits de formule (I) présentent d'excellentes propriétés qui permettent de les utiliser pour lutter contre les insectes : ils présentent en particulier un bon pouvoir létal et un excellent pouvoir de knock-down.

Les produits de formule (I) sont de plus photostables et ne sont pas toxiques pour les mammifères.

L'ensemble de ces propriétés fait des produits de formule (I) des produits qui correspondent parfaitement aux exigences de l'industrie agrochimique moderne : ils permettent de protéger les récoltes tout en préservant l'environnement.

Le produit de l'exemple 1 est particulièrement remarquable.

Les produits de formule (I) peuvent également être utilisés pour lutter contre les insectes dans le domaine domestique, pour lutter notamment contre les mouches, les moustiques et les blattes.

Les produits de formule (I) peuvent aussi être utilisés pour lutter contre les acariens et les nématodes parasites des végétaux.

La partie expérimentale exposée ci-après met clairement en évidence l'activité du produit de l'exemple 6 pour lutter contre le Tetranychus Urticae.

Les composés de formule (I) peuvent encore être utilisés pour lutter contre les acariens parasites des animaux, pour lutter par exemple contre les tiques et notamment les tiques de l'espèce Boophilus, ceux de l'espèce Hyalomnia, ceux de l'espèce Amblyomnia et ceux de l'espèce Rhipicephalus, ou pour lutter contre toutes sortes de gales et notamment la gale sarcoptique, la gale psoroptique et la gale chorioptique.

L'invention a donc également pour objet les compositions destinées à la lutte contre les parasites des animaux à sang chaud, les parasites domestiques et les parasites des végétaux, caractérisées en ce qu'elles renferment au moins l'un des produits définis ci-dessus.

L'invention a notamment pour objet les compositions insecticides renfermant comme principe actif au moins l'un des produits définis ci-dessus.

L'invention a tout spécialement pour objet les compositions renfermant comme principe actif,

— le (1R cis) 2,2-diméthyl 3-/(Z) 2-(méthoxycarbonyl) éthényl/cyclopropane carboxylate de (S) α-cyano 3-phénoxybenzyle.

L'invention a par ailleurs plus particulièrement pour objet les compositions renfermant comme principe actif,

— le (1R cis) 2,2-diméthyl 3-/(Z) 2-(éthoxycarbonyl) éthényl/cyclopropane carboxylate de (S) α-cyano 3-phénoxybenzyle,

— le (1R cis) 2,2-diméthyl 3-/(Z) 2-(isopropyloxycarbonyl) éthényl/cyclopropane carboxylate de (S) α-cyano 3-phénoxybenzyle,

— le (1R cis) 2,2-diméthyl 3-/(Z) 2-(cyclopropylméthoxycarbonyl) éthényl/cyclopropane carboxylate de (S) α-cyano 3-phénoxybenzyle,

— le (1R cis) 2,2-diméthyl 3-/(Z) 2-(cyclopropyloxycarbonyl) éthényl/cyclopropane carboxylate de (S) α-cyano 3-phénoxybenzyle,

— le (1R cis) 2,2-diméthyl 3-/(Z) 2-(terbutoxycarbonyl) éthényl/cyclopropane carboxylate de (S) α-cyano 3-phénoxybenzyle.

Les compositions selon l'invention sont préparées par les procédés usuels de l'industrie agrochimique, de l'industrie vétérinaire ou de l'industrie des produits destinés à la nutrition animale.

Dans les compositions destinées à l'usage agrochimique et domestique la ou les matières actives peuvent être additionnées éventuellement d'un ou plusieurs autres agents pesticides. Ces compositions peuvent se présenter sous forme de poudres, granulés, suspensions, émulsions, solutions, solutions pour aérosols, bandes combustibles, appâts ou autres préparations employées classiquement pour l'utilisation de ce genre de composés.

Outre le principe actif, ces compositions contiennent, en général, un véhicule et/ou un agent tensio-actif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre tel que le talc, les argiles, les silicates, le kieselguhr ou un solide combustible.

Les compositions insecticides selon l'invention contiennent de préférence de 0,005 % à 10 % en poids de matière active.

Selon un mode opératoire avantageux, pour un usage domestique, les compositions selon l'invention sont utilisées sous forme de compositions fumigantes.

Les compositions selon l'invention peuvent alors être avantageusement constituées, pour la partie non active, d'un serpentin insecticide (ou coil) combustible, ou encore d'un substrat fibreux incombustible. Dans ce dernier cas, le fumigant obtenu après incorporation de la matière active est placé sur un appareil chauffant tel qu'un électromosquito destroyer.

Dans le cas où l'on utilise un serpentin insecticide, le support inerte peut être, par exemple, composé de marc de pyrèthre, poudre de Tabu (ou poudre de feuilles Machilus Thumbergii), poudre de tige de pyrèthre, poudre de feuille de cèdre, poudre de bois, (telle que de la sciure de pin) amidon et poudre de coque de noix de coco.

La dose de matière active peut alors être, par exemple, de 0,03 à 1 % en poids.

Dans le cas où l'on utilise un support fibreux incombustible, la dose de matière active peut alors être, par exemple, de 0,03 à 95 % en poids.

Les compositions selon l'invention pour un usage domestique peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95 % en poids.

Les compositions insecticides selon l'invention, comme les compositions acaricides et nématicides, peuvent être additionnées éventuellement d'un ou plusieurs autres agents pesticides. Les compositions acaricides et nématicides peuvent se présenter notamment sous forme de poudre, granulés, suspensions, émulsions, solutions.

Pour l'usage acaricide, on utilise de préférence des poudres mouillables, pour pulvérisation foliaire contenant de 1 à 80 % ou des liquides pour pulvérisation foliaire contenant de 1 à 500 g/l de principe actif. On peut également employer des poudres pour poudrage foliaire contenant de 0,05 à 3 % de matière active.

Pour l'usage nématicide on utilise de préférence des liquides pour traitement des sols contenant de 300 à 500 g/l de principe actif.

Les composés acaricides et nématicides selon l'invention sont utilisés, de préférence à des doses comprises entre 1 et 100 g de matière active à l'hectare.

Pour exalter l'activité biologique des produits de l'invention on peut les additionner à des synergistes classiques utilisés en pareil cas tel que le 1-(2,5,8-trioxadodecyl) 2-propyl 4,5-méthylènedioxy benzène (ou butoxyde de pipéronyle) ou la N-(2-éthyl heptyl) bicyclo [2,2-1]-5-heptène-2,3-di carboximide, ou la pipéronyl-bis-2-(2'-n-butoxy éthoxy) éthylacétal (ou tropical).

Lorsqu'il s'agit de lutter contre les acariens parasites des animaux, on incorpore très souvent les produits de l'invention dans des compositions alimentaires en association avec un mélange nutritif adapté à l'alimentation animale. Le mélange nutritiel peut varier selon l'espèce animale, il peut renfermer des céréales, des sucres et des grains, des tourteaux de soja, d'arachide et de tournesol, des farines

d'origine animale, par exemple des farines de poissons, des acides aminés de synthèse, des sels minéraux, des vitamines et des antioxydants.

L'invention a donc pour objet les compositions alimentaires définies ci-dessus.

Les composés de formule (I) présentent une excellente tolérance générale, et l'invention a donc également pour objet les produits de formule (I), à titre de médicaments, pour lutter notamment contre les affections créées par les tiques et les gales.

Les médicaments de l'invention peuvent être utilisés tant en médecine humaine qu'en médecine vétérinaire.

Les médicaments de l'invention sont notamment utilisés en médecine humaine pour lutter contre les poux à titre préventif ou curatif et pour lutter contre la gale. Ils peuvent également être utilisés comme anthelmintiques.

Les médicaments de l'invention peuvent être administrés par voie externe, par vaporisation, par bain ou badigeonnage.

Les médicaments de l'invention à usage vétérinaire peuvent être également administrés par badigeonnage de l'épine dorsale selon la méthode dite méthode « pour-on ». Ils peuvent être également administrés par voie digestive ou parentérale.

L'invention a donc pour objet les compositions pharmaceutiques renfermant comme principe actif au moins l'un des médicaments définis précédemment.

On peut indiquer également que les produits de l'invention peuvent être utilisés comme biocides ou comme régulateur de croissance.

L'invention a également pour objet les associations douées d'activité insecticide, acaricide ou nématicide, caractérisées en ce qu'elles contiennent comme matière active, d'une part un au moins des composés de formule générale (I), et d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcools α-cyano 3-phénoxy benzyliques des acides 2,2-diméthyl 3-(2,2-dichlorovinyl) cyclopropane-1-carboxyliques, par les esters d'alcools α-cyano 3-phénoxy benzyliques d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolones, d'alcool 3,4,5,6-tétrahydrophtalimidométhylique, d'alcool 5-benzyl 3-furyl méthylique, d'alcool 3-phénoxy benzylique et d'alcools α-cyano 3-phénoxy benzyliques des acides 2,2-diméthyl 3-(1,2,2,2-tétrahalo) cyclopropane-1-carboxyliques, dans lesquels « halo » représente un atome de fluor, de chlore ou de brome, étant entendu que les composés I peuvent exister sous toutes leurs formes stéréoisomères possibles de même que les copules acides et alcools des esters pyréthrinoïdes ci-dessus.

Les associations selon l'invention présentent notamment l'intérêt soit de permettre de combattre, par la polyvalence de leur action, une gamme de parasites, plus étendue, soit de manifester, dans certains cas, un effet de synergie.

L'invention a également pour objet un procédé de préparation des composés de formule (I) caractérisé en ce que l'on fait réagir un acide de formule

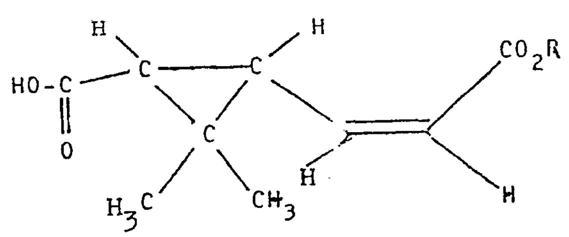

(II)

dans laquelle la double liaison a la géométrie Z et la copule cyclopropanique est de structure IR cis et R conserve la même signification que précédemment ou un dérivé fonctionnel de cet acide avec un alcool de formule

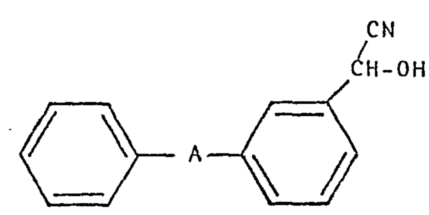

(III)

dans laquelle A conserve la même signification que précédemment.

L'invention a plus spécialement pour objet un procédé caractérisé en ce que l'acide de départ utilisé répond à la formule (II) pour laquelle R représente un radical méthyle, et l'alcool utilisé répond à la formule (III) pour laquelle A représente un atome d'oxygène. Le dérivé fonctionnel d'acide utilisé peut être par exemple un chlorure d'acide.

La réaction d'estérification peut être réalisée selon d'autres procédés. On peut par exemple faire réagir l'acide de formule (II) avec l'alcool de formule (III) en présence de dicyclohexylcarbodiimide ou de diisopropyl carbodiimide. Comme le montre la partie expérimentale ce procédé est l'un des modes de préparation préféré du procédé de l'invention.

Les composés de formule (II) sont des produits chimiques nouveaux, et l'invention a donc pour objet les composés de formule (II) à titre de produits chimiques nouveaux.

Parmi les composés de formule (II), on peut citer tout particulièrement les composés dont la préparation est donnée plus loin dans la partie expérimentale.

L'invention a plus particulièrement pour objet les produits de formule (II) à titre de produits intermédiaires nécessaires à la mise en œuvre du procédé de l'invention, notamment ceux dont la préparation est donnée plus loin dans la partie expérimentale.

L'invention a également pour objet un procédé de préparation tel que défini précédemment, caractérisé en ce que le composé de formule (II) utilisé est préparé en faisant réagir un composé de formule (IV) :

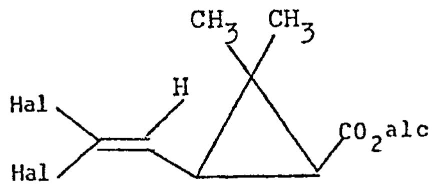

(IV)

dans laquelle la copule cyclopropanique est de structure IR cis et Hal représente un atome d'halogène et alc représente un radical alcoyle renfermant de 1 à 8 atomes de carbone, dans un premier temps avec un agent alcalin capable d'arracher les atomes d'halogène et dans un deuxième temps,

— soit avec un agent capable d'introduire le groupement carboxylique pour obtenir le composé de formule (V)

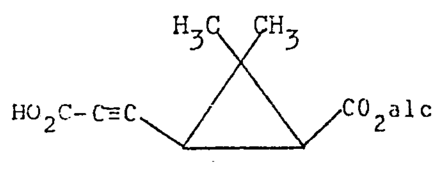

(V)

que l'on soumet à l'action d'un agent d'estérification, pour obtenir le composé de formule (VI) :

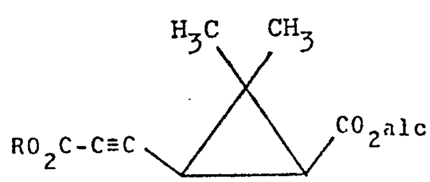

(VI)

dans lequel R conserve la même signification que précédemment,

— soit avec un chloroformiate d'alcoyle de formule

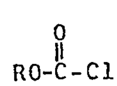

(V')

dans laquelle R conserve la signification précédente, pour obtenir directement le composé de formule (VI)

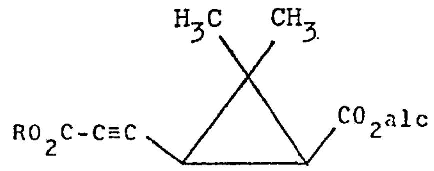

(VI)

puis en soumettant le composé de formule (VI) obtenu, à l'action d'un agent d'hydrogénation ménagée, pour obtenir le composé de formule

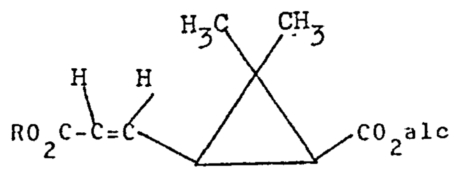

(VII)

dans laquelle la double liaison a la géométrie Z, que l'on soumet à l'action d'un agent d'hydrolyse acide capable de cliver sélectivement la fonction ester en 1 du cyclopropane, pour obtenir le composé de formule (II) correspondant :

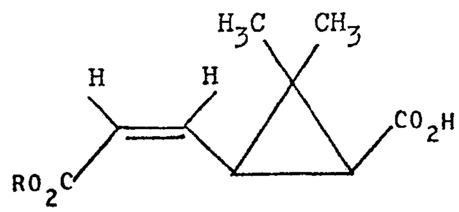

(II)

Dans un mode de réalisation préféré du procédé de l'invention,
— Hal représente un atome de brome ou de chlore,
— alc représente un radical ter-butyle, benzyle ou trityle,
— l'agent alcalin capable d'arracher les halogènes vinyliques est le butyl lithium,
— l'agent capable d'introduire le groupement carboxylique est le dioxyde de carbone,
— l'agent d'hydrogénation ménagée est l'hydrogène en présence d'un catalyseur, comme le palladium en présence de traces de quinoléine,
— l'agent d'hydrolyse acide est l'acide paratoluène sulfonique.

Le procédé qui vient d'être décrit conduit donc aux produits de l'invention, c'est-à-dire à des produits pour lesquels la double liaison a la géométrie Z, avec d'excellents rendements comme le montre clairement la partie expérimentale exposée ci-après.

Le procédé ci-dessus comporte une variante évidente pour le chimiste, dans laquelle la réduction du composé (V) précède l'estérification. L'invention a donc également pour objet un procédé tel que défini précédemment, caractérisé en ce que l'on soumet le composé de formule (V) :

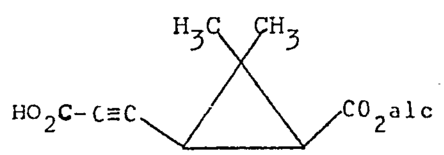

(V)

dans laquelle la copule cyclopropanique est de structure IR cis et alc conserve sa signification précédente, d'abord à l'action d'un agent d'hydrogénation ménagée, pour obtenir un composé de formule :

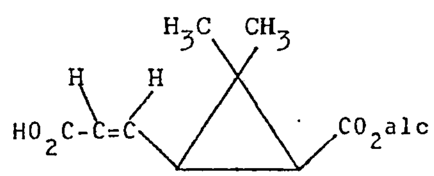

(VIII)

dans laquelle la double liaison a la géométrie Z, que l'on soumet à l'action d'un agent d'estérification, pour obtenir le composé de formule (VII) correspondant :

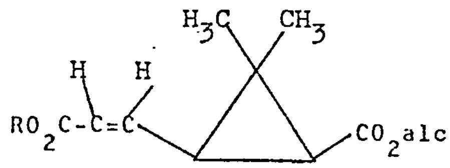

(VII)

Le procédé ci-dessus comporte une seconde variante évidente, dans laquelle l'ordre de certains stades est modifié.

L'invention a donc également pour objet un procédé tel que défini précédemment, caractérisé en ce que l'on soumet un composé de formule (VI) :

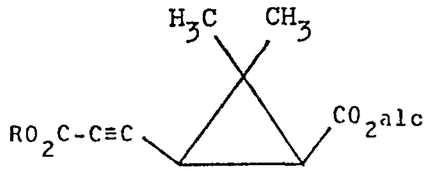

(VI)

dans laquelle R et alc sont définis comme précédemment, à l'action d'un agent d'hydrolyse acide capable de cliver sélectivement la fonction ester en 1 du cyclopropane, pour obtenir le composé de formule (IX) :

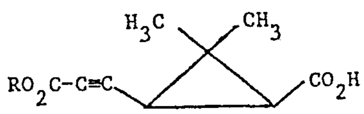

(IX)

dans laquelle la copule cyclopropanique est de structure IR cis et R est défini comme précédemment, que

— soit l'on soumet, le cas échéant, sous forme d'un dérivé fonctionnel, à l'action d'un alcool de formule (III) :

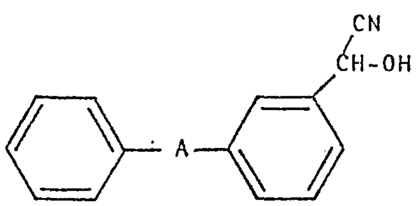

(III)

dans laquelle A conserve la même signification que précédemment, pour obtenir un composé de formule (X) :

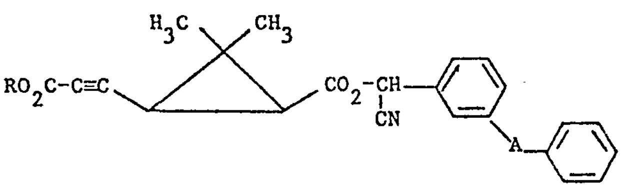

(X)

dans laquelle R et A conservent la même signification que précédemment, que l'on soumet à l'action d'un agent d'hydrogénation ménagée, pour obtenir le composé de formule (I),

— soit l'on soumet d'abord à l'action d'un agent d'hydrogénation ménagée, pour obtenir le composé de formule (II) :

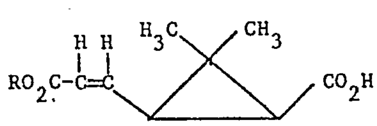

(II)

dans laquelle R est défini comme précédemment et la double liaison a la géométrie Z, puis, le cas échéant sous forme d'un dérivé fonctionnel, à l'action d'un alcool (III), pour obtenir le composé de formule I.

Les conditions préférentielles d'exécution du procédé ci-dessus sont identiques à celles qui ont été définies précédemment pour les opérations analogues.

Les composés de formule (V), (VI), (VII), (VIII), (IX) et (X) obtenus lors de la mise en œuvre du procédé de l'invention sont des produits nouveaux.

L'invention a donc également pour objet, à titre de produits industriels nouveaux et notamment à titre de produits intermédiaires nécessaires à la mise en œuvre de l'invention, les produits de formule V, VI, VII, VIII, IX et X et, plus particulièrement, ceux dont la préparation est donnée plus loin dans la partie expérimentale.

L'invention a également pour objet un procédé de préparation des produits de formule I, caractérisé en ce que l'on soumet un composé de formule (XI) :

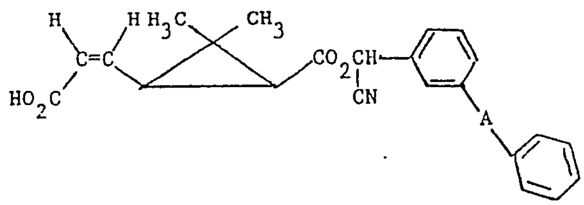

(XI)

dans laquelle la double liaison a la géométrie Z et la copule cyclopropanique est de structure IR cis, à l'action d'un agent d'estérification, pour obtenir le composé de formule (I) correspondant :

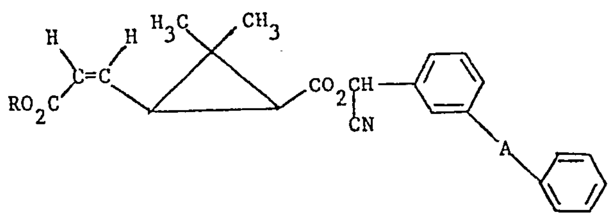

(I)

Dans un mode de réalisation préféré du procédé ci-dessus, l'estérification est effectuée avec un dérivé fonctionnel d'alcool, à savoir un dérivé de la N,N'-diisopropylurée de formule :

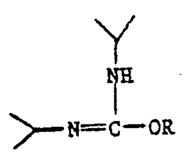

L'invention a également pour objet un procédé tel que défini précédemment, caractérisé en ce que le produit de formule (XI) est préparé en soumettant un acide de formule (V) :

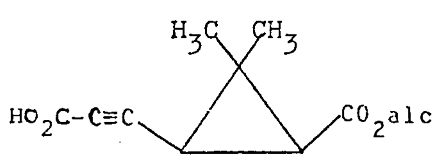

(V)

dans laquelle la copule cyclopropanique est de structure IR cis et alc représente un radical alcoyle renfermant de 1 à 8 atomes de carbone à l'action du 2,2,2-trichloroéthanol pour obtenir le composé de formule (XII) :

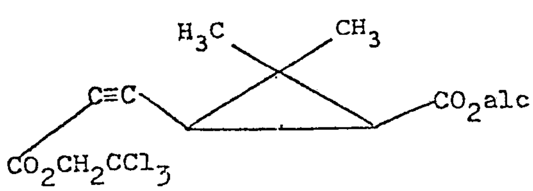

(XII)

que l'on soumet à l'action d'un agent d'hydrolyse acide pour obtenir le composé de formule (XIII) :

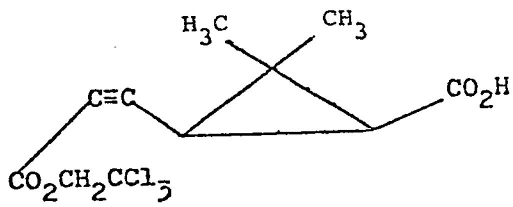

(XIII)

que l'on soumet à l'action d'un alcool de formule (III) :

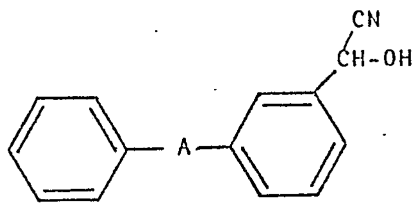

(III)

dans laquelle A conserve la même signification que précédemment, pour obtenir le composé de formule (XIV) :

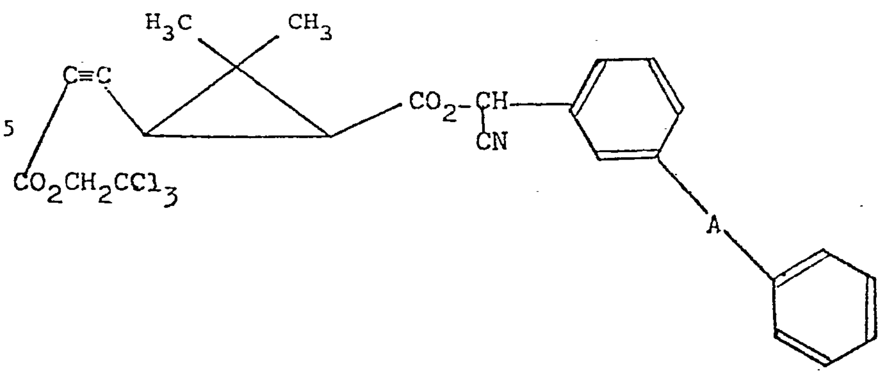

(XIV)

que l'on soumet à l'action d'un agent de clivage de la fonction ester porté par le carbone acétylénique pour obtenir le composé de formule (XV) :

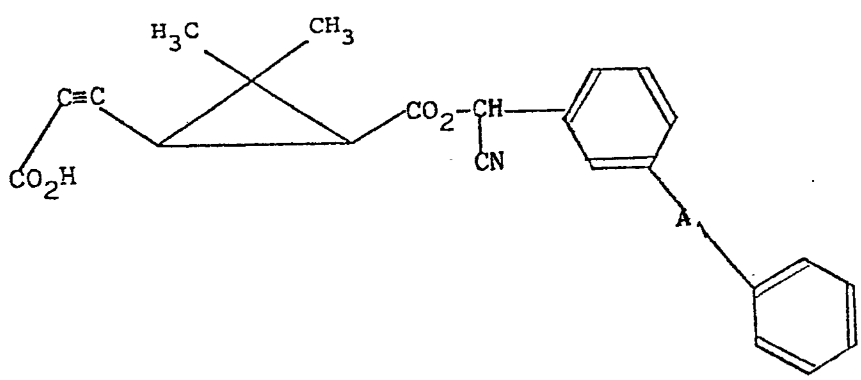

(XV)

que l'on soumet à l'action d'un agent d'hydrogénation ménagée, pour obtenir le composé de formule (XI).

Dans un mode de réalisation préféré du procédé de l'invention ci-dessus,

— alc dans la formule (V) représente un radical terbutyle, benzyle ou trityle ;

— l'agent d'hydrolyse acide est l'acide paratoluènesulfonique ;

— l'estérification du composé (XIII) se fait en faisant réagir le composé (XIII) avec l'alcool (III) en présence de dicyclohexyl carbodiimide ou de diisopropyl carbodiimide ;

— le clivage de l'ester (XIV) se fait en utilisant une poudre métallique par exemple, la poudre de zinc, en milieu acide ;

— l'agent d'hydrogénation ménagée est l'hydrogène en présence d'un catalyseur comme le palladium, en présence de traces de quinoléine.

Le procédé ci-dessus comporte une variante évidente selon laquelle les stades d'hydrogénation et d'estérification sont inversés.

L'invention a ainsi également pour objet un procédé tel que défini précédemment, caractérisé en ce que l'on soumet un composé de formule (XV) :

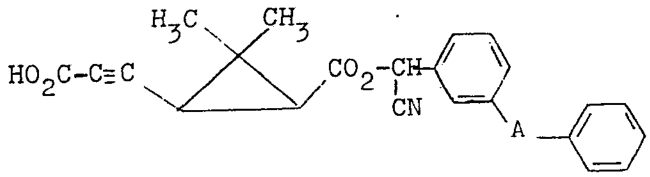

(XV)

dans laquelle la copule cyclopropanique est de structure IR cis et A est défini comme précédemment, à l'action d'un agent d'estérification, pour obtenir le composé de formule (X) :

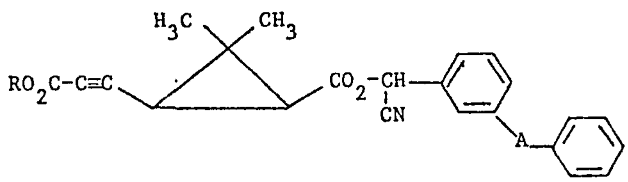

(X)

dans laquelle R et A sont définis comme précédemment, que l'on soumet à l'action d'un agent d'hydrogénation ménagée, pour obtenir le composé de formule I.

Les conditions préférentielles d'exécution du procédé ci-dessus sont identiques à celles qui ont été définies précédemment pour les opérations analogues.

Les produits intermédiaires obtenus lors de la mise en œuvre du procédé de l'invention sont des produits chimiques nouveaux et l'invention a donc également pour objet, à titre de produits industriels nouveaux et notamment à titre de produits intermédiaires nécessaires à la mise en œuvre de l'invention, les produits de formule XI, XII, XIII, XIV, et XV et, plus particulièrement, ceux dont la préparation est donnée plus loin dans la partie expérimentale.

Ainsi qu'il est précisé ci-dessus, les composés de formule (II) sont des produits nouveaux. On vient de découvrir que, de plus, ces produits et notamment l'acide (1R, cis) 2,2-diméthyl 3-[(Z) 2-(méthoxycarbonyl) éthényl] cyclopropane carboxylique, possèdent des propriétés antifongiques intéressantes qui les rendent aptes à être utilisés dans la lutte contre les fungi. Ils peuvent notamment être utilisés pour la lutte contre les fungi parasites des cultures, par exemple, les divers fungi parasites de la vigne, des tomates et des concombres.

Des tests sur Fusarium, Penicillium, Aspergilus, Geotrichum, Trichosporon et Cephalosporium, décrits plus loin dans la partie expérimentale, mettent en évidence l'activité antifongique de ces acides.

On peut donc préparer des compositions antifongiques renfermant, comme principe actif, l'un au moins des composés de formule (II) tels que définis ci-dessus.

Dans ces compositions la ou les matières actives peuvent être additionnées éventuellement d'un ou plusieurs autres agents pesticides. Ces compositions peuvent se présenter sous forme de poudres, granulés, suspensions, émulsions, solutions ou autres préparations employés classiquement pour l'utilisation de ce genre de composés.

Outre le principe actif, ces compositions contiennent en général, un véhicule et/ou un agent tensioactif, non ionique assurant, en outre, une dispersion uniforme des substances constitutives du mélange. Le véhicule utilisé peut être un liquide tel que l'eau, l'alcool, les hydrocarbures ou autres solvants organiques, une huile minérale, animale ou végétale, une poudre telle que le talc, les argiles, les silicates, le kieselguhr.

Ces compositions anti-fongiques renferment, de préférence, pour les poudres pour pulvérisation de 25 à 95 % en poids de matière active, pour les poudres pour poudrage foliaire de 2,5 à 95 % en poids de matière active, pour les poudres ou liquides pour pulvérisation au sol de 10 à 30 % en poids de matière active.

Les composés de formule II ci-dessus sont également doués de propriétés bactéricides qui les rendent aptes à être utilisés comme biocides industriels.

Des tests donnés, ci-après, dans la partie expérimentale, illustrent l'activité biocide de ces acides. Ces tests ont été effectués pour les colles infestées par un mélange complexe de bactéries.

Les composés de formule II précités peuvent donc être utilisés d'une manière générale comme biocides industriels, notamment pour la protection des colles, des charges industrielles, et lors de

l'utilisation des huiles de coupe. Ils peuvent être également utilisés pour prévenir et éliminer la formation de boues microbiennes dans les circuits de papeterie ou pour le traitement des peaux, liqueurs de tannage et cuirs.

On peut aussi préparer des compositions biocides renfermant, comme principe actif, l'un au moins des composés de formule II tels que définis ci-dessus.

Dans ces compositions la ou les matières actives peuvent être additionnées d'un ou plusieurs autres agents pesticides. Ces compositions peuvent se présenter sous des formes analogues aux compositions antifongiques décrites précédemment, c'est-à-dire, sous forme de poudres, suspensions, émulsions ou solutions et peuvent contenir outre le ou les principes actifs un véhicule solide ou liquide et un agent tensio-actif.

Les compositions biocides selon l'invention renfermant de préférence, de 20 à 95 % en poids de matière active.

Préparation 1 : Acide (1R, cis) 2,2-diméthyl 3-[(Z) 2(méthoxy carbonyl) éthényl] cyclopropane carboxylique.

Stade A : (1R, cis) 2,2-diméthyl 3-[2(méthoxy carbonyl) éthynyl] cyclopropane carboxylate de terbutyle :

On introduit 55 g de (1R, cis) 2,2-diméthyl 3-(2,2-dibromovinyl) cyclopropane carboxylate de terbutyle dans 550 $cm^3$ de tétrahydrofurane. On refroidit à – 70 °C et ajoute en 40 minutes, 132 $cm^3$ d'une solution de butyl lithium dans le cyclohexane (à 20 %) et agite 30 minutes à – 65 °C. On ajoute ensuite 12,5 $cm^3$ de chloroformiate de méthyle. Après 2 heures de réaction à – 70 °C, on laisse la température remonter à – 20 °C et verse le mélange obtenu dans une solution aqueuse de phosphate monosodique et extrait à l'éther. On lave, sèche et amène à sec sous pression réduite. On obtient ainsi 38,3 g d'un produit que l'on chromatographie sur silice éluant cyclohexane-acétate d'éthyle (8-2). On obtient ainsi 17,2 g du produit recherché.

Stade B : (1R, cis) 2,2-diméthyl 3-[(Z) 2-(méthoxy carbonyl) éthényl] cyclopropane carboxylate de terbutyle :

On fait une hydrogénation de 12 g du produit préparé au stade A dans 240 $cm^3$ d'acétate d'éthyle, en présence de 2,4 g d'hydroxyde de palladium à 10 % sur sulfate de baryum et 2,4 $cm^3$ de quinoléine. On filtre et sèche. On obtient ainsi 11 g du produit recherché.

Stade C : Acide(1R, cis) 2,2-diméthyl 3-[(Z) 2-(méthoxy carbonyl) éthényl] cyclopropane carboxylique :

On porte au reflux pendant 3 heures une solution renfermant 13,5 g du produit préparé au stade B, 100 $cm^3$ de toluène et 400 mg d'acide paratoluènesulfonique hydraté. On amène à sec sous pression réduite et obtient 11,2 g d'un produit que l'on chromatographie sur silice éluant : cyclohexane-acétate d'éthyle-acide acétique (60-39-1). On amène à sec sous pression réduite et obtient 9,6 g du produit recherché fondant à 110 °C.

$(\alpha)_D = + 75{,}5° \pm 2°$ (c = 1 %, $CHCl_3$)

RMN $CDCl_3$ ppm :

1,3 : protons des méthyles en 2 du cyclopropane

1,86-2 : proton en 1 du cyclopropane

3,1-3,28-3,43 : proton en 3 du cyclopropane

5,8-5,99 : proton éthylénique en $\alpha$ du groupement $CO_2CH_3$

6,42-6,58 }
6,61-6,77 } proton éthylénique en $\beta$ du groupement $CO_2CH_3$

8,63 proton du groupement $CO_2H$

3,71 protons du méthoxy

Préparation 2 : Acide (1R, cis) 2,2-diméthyl [(Z) 3-(éthoxy carbonyl) éthényl] cyclopropane carboxylique.

Stade A : (1R, cis) 2,2-diméthyl 3 [carboxy éthynyl] cyclopropane carboxylate de terbutyle :

On introduit 26 g de (1R, cis) 2,2-diméthyl 3-(2,2-dibromovinyl) cyclopropane carboxylate de terbutyle dans 175 $cm^3$ de tétrahydrofurane anhydre. On ajoute ensuite à – 65 °C 60 $cm^3$ d'une solution de butyl lithium à 20 % dans le cyclohexane. On agite 1 heure à – 60 °C puis fait barboter un courant de dioxyde de carbone pendant 1 heure et demie, verse le mélange réactionnel dans l'eau glacée additionnée de soude N. On lave à l'éther. La phase aqueuse alcaline est acidifiée à pH 4 et extraite à l'éther. On sèche les phases organiques, amène à sec sous pression réduite. On obtient ainsi un produit que l'on recristallise dans l'éther de pétrole (Eb 60-80 °C). On obtient alors 8,3 g du produit recherché fondant à 144 °C.

RMN $CDCl_3$ ppm :

1,22 et 1,37 : protons des méthyles en 2 du cyclopropane

1,78 : proton en 1 et 3 du cyclopropane

1,47 : protons du terbutyle
8,25 : proton du groupement 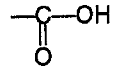

Stade B : (1R, cis) 2,2-diméthyl 3-(éthoxycarbonyl éthynyl) cyclopropane carboxylate de terbutyle :

On introduit 4 g du produit préparé au stade A, 3,4 g de dicyclohexylcarbodiimide et 6 mg de 4-diméthylamino pyridine dans 30 $cm^3$ de chlorure de méthylène. On ajoute ensuite 1,5 $cm^3$ d'éthanol et agite 16 heures à 20 °C. On filtre, et concentre le filtrat sous pression réduite. On obtient ainsi 5,5 g d'un produit que l'on purifie par chromatographie sur silice éluant cyclohexane-acétate d'éthyle (9-1). On obtient ainsi 4,25 g du produit recherché.

RMN $CDCl_3$ ppm :
1,18-1,21 et 1,36-1,47 : protons en 2 du cyclopropane
1,73 et 1,82 : protons en 1 et 3 du cyclopropane
1,47 : protons du terbutyle
1,27-1,38-1,5 }
4,0-4,13-4,25-4,36 } protons de l'éthyle

Stade C : (1R, cis) 2,2-diméthyl 3-[(Z) 2(éthoxy carbonyl) éthényl] cyclopropane de terbutyle :

On fait une hydrogénation de 4,3 g du produit préparé au stade précédent dans 100 $cm^3$ d'acétate d'éthyle en présence de 800 mg de catalyseur de $Pd(OH)_2BaSO_4$ et 0,8 $cm^3$ de quinoléine. On filtre, amène le filtrat à un pH inférieur à 7, avec de l'acide chlorhydrique 2N et lave à l'eau. On sèche et amène à sec sous pression réduite. On obtient 4,6 g d'un produit que l'on chromatographie sur silice éluant cyclohexane-acétate d'éthyle (95-5). On obtient ainsi 2,5 g du produit recherché.

RMN $CDCl_3$ ppm :
1,25 et 1,28 : protons des méthyles en 2 du cyclopropane
1,78-1,93 : proton en 1 du cyclopropane
2,98-3,1-3,2 : proton en 3 du cyclopropane
6,4-6,6-6,8 : proton du carbone éthylénique en $\alpha$ du cyclopropane
5,7-5,9 : proton du carbone éthylénique portant le groupement éthoxy carbonyle
4,0-4,13-4,25-4,36 : proton du méthylène de l'éthoxy

Stade D : Acide (1R, cis) 2,2-diméthyl 3-[(Z) 2(éthoxy carbonyl) éthényl] cyclopropane carboxylique :

On introduit 2,3 g de produit préparé au stade C et 20 mg d'acide paratoluènesulfonique hydraté dans 20 $cm^3$ de toluène. On porte au reflux pendant 40 minutes, amène à sec sous pression réduite et obtient ainsi 2,1 g d'un résidu que l'on chromatographie sur silice éluant cyclohexane-acétate d'éthyle-acide acétique (60-39-1). On isole 1,7 g d'un produit que l'on recristallise dans le cyclohexane. On obtient ainsi 1,5 g du produit recherché fondant à 96 °C.

RMN $CDCl_3$ ppm :
1,3 et 1,32 : protons des méthyles en 2 du cyclopropane
1,86-2,02 }
3,15-3,28 } proton du carbone en 1 du cyclopropane
3,3-3,45 }
6,38-6,53 } proton du carbone en 3 du cyclopropane
6,55-6,73 : proton du carbone éthylénique branché sur le cyclopropane
5,78-5,96 : proton du carbone éthylénique portant le groupement éthoxy carbonyle
1,18-1,3-1,41 }
4,0-4,13 } protons du méthyle du groupement éthoxy carbonyle
4,25-4,36 : protons du méthylène du groupement éthoxy carbonyle.

Préparation 3 : Acide (1R, cis) 2,2-diméthyl 3 [(Z) 2-(n-propoxy carbonyl) éthényl] cyclopropane carboxylique.

Stade A : (1R, cis) 2,2-diméthyl 3 [n-propoxy carbonyl éthynyl] cyclopropane carboxylate de terbutyle :

On introduit 22,8 g de (1R, cis) 2,2-diméthyl 3-(2,2-dibromovinyl) cyclopropane carboxylate de terbutyle, 250 $cm^3$ de tétrahydrofurane puis, à − 60 °C, 55 $cm^3$ d'une solution de butyl lithium dans le cyclohexane à 20 %. On maintient à − 65 °C pendant 1 heure et introduit à − 65 °C en 15 minutes 8 $cm^3$ de chloroformiate de n-propyle. On maintient l'agitation pendant 1 heure à − 65 °C, laisse remonter à la température ambiante en 1 heure, et agite à nouveau pendant 1 heure à la température ambiante. On verse sur une solution aqueuse saturée de phosphate monosodique, agite, extrait à l'éther et lave à l'eau. On sèche et amène à sec sous pression réduite. On obtient ainsi 19,5 g d'une huile que l'on purifie par chromatographie sur silice éluant cyclohexane-acétate d'éthyle (9-1). On obtient ainsi 11,5 g du produit attendu.

Spectre RMN $CDCl_3$ ppm :
1,17 et 1,37 : protons des méthyles en 2 du cyclopropane
1,72 : protons en 1 et 3 du cyclopropane
1,44 : protons du terbutyle
4,0-4,12-4,23 : proton du méthylène en 1 du propoxy carbonyl
0,83-0,95-1,06 : protons du méthyle du propoxy carbonyl

Stade B : (1R, cis) 2,2-diméthyl 3-[(Z) 2-(n-propoxy carbonyl) éthényl] cyclopropane carboxylate de terbutyle :

On hydrogène 7 g de (1R, cis) 2,2-diméthyl 3-(n-propoxy carbonyl éthynyl) cyclopropane carboxylate de terbutyle dans 140 $cm^3$ d'acétate d'éthyle en présence de 1,4 g d'hydroxyde de palladium à 10 % sur sulfate de baryum et de 1,4 $cm^3$ de quinoléine. On lave le filtrat à l'aide d'une solution d'acide chlorhydrique 2N, puis lave à l'eau, sèche et amène à sec sous pression réduite. On obtient ainsi 7,2 g d'un produit que l'on chromatographie sur silice éluant cyclohexane-acétate d'éthyle (95-5). On obtient ainsi 6,1 g du produit recherché.

Spectre RMN $CDCl_3$ ppm :
1,25 et 1,29 : protons des méthyles en 2 du cyclopropane
1,5 à 2,03 : proton du carbone en 1 du cyclopropane
3,03 à 3,35 } proton du carbone en 3 du cyclopropane
6,5-6,66 }
6,69-6,85 : proton du carbone éthylénique relié au cyclopropane
5,82-6,0 : proton du carbone éthylénique portant le groupement propoxy carbonyl
4,02-4,12-4,23 : proton du méthylène en 1 du groupement propoxy carbonyl
0,86-0,98-1,1 : proton du méthyle du groupement propoxy carbonyl.

Stade C : Acide : (1R, cis) 2,2-diméthyl 3-[(Z) 2-(n-propoxy carbonyl) éthényl] cyclopropane carboxylique :

On porte à reflux pendant 1 heure un mélange de 5,8 g du produit préparé au stade B, 200 mg d'acide paratoluène sulfonique hydraté, et 60 $cm^3$ de toluène. On amène à sec sous pression réduite et obtient 5 g d'un produit que l'on chromatographie sur silice éluant cyclohexane-acétate d'éthyle-acide acétique (70-29-1). On obtient 4,2 g du produit recherché.

Spectre RMN $CDCl_3$ ppm :
1,27 et 1,29 : H des méthyles en 2 du cyclopropane
1,86-2 : H en 1 du cyclopropane
3,13 à 3,45 : H en 3 du cyclopropane
5,8-6 : H du carbone éthylénique portant le groupement $CO_2CH_2CH_2CH_3$
6,4-6,56-6,59 : H du carbone éthylénique relié au cyclopropane
3,98-4,08-4,18 : H du méthylène en 1 du groupement propoxy carbonyle
0,83-0,95-1,06 : H des méthyles du groupement propoxy carbonyle

Préparation 4 : Acide (1R, cis) 2,2-diméthyl 3-[(Z) 2-(isopropyloxy carbonyl) éthényl] cyclopropane carboxylique.

Stade A : (1R, cis) 2,2-diméthyl 3-[(Z) 2-carboxy éthényl] cyclopropane carboxylate de terbutyle :

On hydrogène 2 g de (1R, cis) 2,2-diméthyl 3 [2-carboxy éthynyl] cyclopropane carboxylate de terbutyle dans 40 $cm^3$ d'acétate d'éthyle en présence de 0,38 g d'hydroxyde de palladium à 10 % sur sulfate de baryum et 0,4 $cm^3$ de quinoléine. On filtre, lave le filtrat à l'acide chlorhydrique 0,5N puis à l'eau jusqu'à neutralité, sèche, concentre à sec sous pression réduite et obtient 2 g du produit recherché fondant à 94 °C.

Stade B : (1R, cis) 2,2-diméthyl 3-[(Z) 2-(isopropyloxy carbonyl) éthényl] cyclopropane carboxylate de terbutyle :

On mélange 2,7 g de (1R, cis) 2,2-diméthyl 3-[(Z) 2-(carboxy) éthényl] cyclopropane carboxylate de terbutyle, dans 10 $cm^3$ d'acétate d'éthyle, ajoute ensuite 2 g d'O-isopropyl N,N'-diisopropyl isourée et agite pendant 1 heure à température ambiante. On porte au reflux pendant une heure trente, laisse revenir à 20 °C, filtre l'insoluble et amène à sec le filtrat sous pression réduite. On obtient 3,5 g d'une huile que l'on chromatographie sur silice éluant benzène-cyclohexane (7-3). On obtient ainsi 1 g du produit recherché que l'on utilise tel quel dans le stade suivant.

Stade C : Acide (1R, cis) 2,2-diméthyl 3-[(Z) 2-(isopropyloxy carbonyl) éthényl] cyclopropane carboxylique :

On porte à 120 °C sous agitation pendant 2 heures 30, un mélange renfermant 1,4 g de (1R, cis) 2,2

diméthyl 3-[(Z) 2-(isopropyloxy carbonyl) éthényl] cyclopropane carboxylate de terbutyle, 100 mg d'acide paratoluène sulfonique et 14 $cm^3$ de toluène. On amène à sec sous pression réduite. On obtient un résidu que l'on recristallise dans l'éther isopropylique. On glace, essore, sèche et obtient ainsi 900 mg du produit recherché fondant à 98 °C.

Exemple 1 : (1R, cis) 2,2-diméthyl 3-[(Z) 2-(méthoxy carbonyl) éthényl] cyclopropane carboxylate de (S) α-cyano 3-phénoxybenzyle.

On introduit 7 g d'acide (1R, cis) 2,2-diméthyl [(Z) 3-(2-méthoxy carbonyl) éthényl] cyclopropane carboxylique, 7,3 g de dicyclohexyl carbodiimide et 3 $cm^3$ de pyridine dans 50 $cm^3$ de chlorure de méthylène. On ajoute ensuite 8 g d'alcool (S) α-cyano 3-phénoxy benzylique. On maintient le mélange réactionnel sous agitation pendant 16 heures, à température ambiante. On filtre. On amène le filtrat à sec sous pression réduite et obtient 20 g d'un produit que l'on recristallise dans l'éther isopropylique. On obtient ainsi 10,5 g de produit recherché P F = 98 °C.

RMN $CDCl_3$ ppm :
1,23 et 1,26 protons des méthyles en 2
1,93-2,07 proton du carbone en 1
3,2-3,34-3,37-3,50 proton du carbone en 3
6,35 proton du carbone portant le groupement C≡N
5,8 à 6,05 proton du carbone éthylénique portant le groupement $—CO_2CH_3$
6,35-6,51 } proton du carbone éthylénique en α sur le cyclopropane
6,55-6,72 }
3,72 protons du groupement méthoxy carbonyle
6,9 à 7,6 protons aromatiques.

Exemple 2 : (1R, cis) 2,2-diméthyl 3-[(Z) 2-(méthoxy carbonyl) éthényl] cyclopropane carboxylate de (R) α-cyano 3-phénoxy benzyle.

En opérant comme à l'exemple 1, à partir de 1,5 g de l'acide (1R, cis) 2,2-diméthyl 3-[(Z) 2-(méthoxy carbonyl) éthényl] cyclopropane carboxylique, et de 1,9 g d'alcool (R) α-cyano 3-phénoxy benzylique. On obtient 4,3 g du produit recherché brut que l'on chromatographie sur silice (éluant cyclohexane-acétate d'éthyle (9-1)). On obtient ainsi 2,5 g du produit recherché.

$(\alpha)_D$ : + 23,5° ± 2,5° (c = 0,5 % benzène)

RMD $CDCl_3$ ppm :
1,32 protons des méthyles en 2
1,92-2,06 proton du carbone en 1
3,17 à 3,45 proton du carbone en 3
6,3 proton du carbone portant le groupement C≡N
5,8-5,98 proton du carbone éthylénique portant le groupement $CO_2CH_3$
6,3 à 6,7 proton du carbone éthylénique en α du cyclopropane
3,7 protons du groupement métoxycarbonyle
6,9 à 7,6 protons aromatiques.

Exemple 3 : (1R, cis) 2,2-diméthyl 3-[(Z) 2-(éthoxy carbonyl) éthényl] cyclopropane carboxylate de (S) α-cyano 3-phénoxybenzyle

En opérant comme à l'exemple 1, à partir de 1,25 g d'acide (1R, cis) 2,2-diméthyl 3 [(Z) 2-(éthoxy carbonyl) éthényl] cyclopropane carboxylique et de 1,45 g d'alcool (S) α-cyano 3-phénoxy benzylique, on obtient 4,1 g du produit recherché sous forme brute que l'on chromatographie sur silice (éluant : cyclohexane-acétate d'éthyle (9-1)). On obtient ainsi 1,95 g du produit recherché.

$(\alpha)_D$ : + 57°5 ± 3 (c = 0,3 % dans le benzène)

RMN $CDCl_3$ ppm :
1,25-1,27 protons des méthyle en 2 du cyclopropane
1,92-2,06 proton du carbone en 1 du cyclopropane
3,2-3,36 } proton du carbone en 3 du cyclopropane
3,8-3,52 }
5,83-6,03 proton du carbone éthylénique portant le groupement éthoxycarbonyle
6,38-6,73 proton du carbone éthylénique en α du cyclopropane
6,35 proton du carbone portant le groupement C≡N
1,18-1,3-1,41 proton du méthyle de l'éthyle
4,01-4,13-4,25-4,36 proton du méthylène de l'éthyle.

Exemple 4 : (1R, cis) 2,2-diméthyl 3 [(Z) 2-(n-propoxy carbonyl) éthényl] cyclopropane carboxylate de (S) α-cyano 3-phénoxybenzyle.

En opérant comme à l'exemple 1, à partir de 1,5 g d'acide (1R, cis) 2,2-diméthyl 3 [(Z) 2-(n-propoxy

carbonyl) éthényl] cyclopropane carboxylique et de 1,7 g d'alcool (S) α-cyano 3-phénoxy benzylique, on obtient 4,1 g de produit que l'on chromatographie sur silice (éluant n-hexane-éther isopropylique (7-3)). On obtient 2,2 g du produit recherché.

$(\alpha)_D$ : + 52° ± 2,5° (c = 0,5 % benzène)

RMN $CDCl_3$ ppm

1,25-1,28 protons des méthyles en 2 du cyclopropane

1,94-2,03 proton du carbone en 1 du cyclopropane

3,29-3,39-3,49 proton du carbone en 3 du cyclopropane

6,33 proton du carbone portant le groupement C≡N

5,89-6,01 proton du carbone éthylénique portant le groupement propoxycarbonyle

6,41-6,52 }
6,53-6,64 } proton du carbone éthylénique branché sur le cyclopropane

4,02-4,09-4,15 proton de méthylène 1 du groupement propoxy carbonyl

0,88-0,96-1,04 proton du méthyle du groupement propoxy carbonyl

Exemple 5 : (1R, cis) 2,2-diméthyl 3-[(Z) 2-(isopropyloxy carbonyl) éthényl] cyclopropane carboxylate de (S) α-cyano 3-phénoxybenzyle.

En opérant comme à l'exemple 1, à partir de 900 mg d'acide 1R, cis 2,2-diméthyl 3-[(Z) 2-(isopropyloxycarbonyl) éthyl] cyclopropane carboxylique et 900 mg d'alcool (S) (α) cyano 3-phénoxy benzylique, on obtient 1,8 g du produit recherché sous la forme brute que l'on chromatographie sur silice (éluant cyclohexane-acétate d'éthyle (9-1)). On obtient ainsi 1,2 g du produit recherché.

$(\alpha)_D$ : + 54° ± 2° (c = 0,4 % dans le benzène)

RMN $CDCl_3$ ppm

1,25 et 1,27 : protons des méthyles en 2 du cyclopropane

1,92-2,05 : proton du carbone en 1 du cyclopropane

3,25-3,39 }
3,42-3,56 } proton du carbone en 3 du cyclopropane

6,3 : proton du carbone portant le groupement C≡N

5,8-6 : proton du carbone éthylénique portant le groupement isopropyloxy

6,35-6,51 }
6,55-6,71 } proton du carbone éthylénique branché sur le cyclopropane

5,08 : proton de l'isopropyle

1,23-1,34 : proton des méthyles de l'isopropyle

Exemple 6 : (1R, cis) 2,2-diméthyl 3-[(Z) 2-(terbutoxycarbonyl) éthényl] cyclopropane carboxylate de (S) α-cyano 3-phénoxybenzyle.

Stade A : (1R, cis) 2,2-diméthyl 3-(2,2,2-trichloroéthoxycarbonyl éthynyl) cyclopropane carboxylate de terbutyle.

On introduit 6,2 g de dicyclohexylcarbodiimide dans une solution renfermant 7,15 g de (1R, cis) 2,2-diméthyl 3-(2-carboxyéthynyl) cyclopropane carboxylate de terbutyle et 80 mg de diméthylaminopyridine dans 35 $cm^3$ de chlorure de méthylène. On agite le mélange réactionnel pendant 10 minutes et ajoute 4,5 g de 2,2,2-trichloroéthanol. On maintient sous agitation pendant une heure et élimine par filtration le précipité formé. On lave le filtrat à l'acide chlorhydrique N puis à l'eau jusqu'à neutralité, le sèche et l'amène à sec. On obtient 14 g d'une huile que l'on chromatographie sur silice en éluant par le mélange benzène-acétate d'éthyle (97-3). On isole ainsi 9 g du produit recherché fondant à 70-71 °C.

Stade B : acide (1R, cis) 2,2-diméthyl 3(2,2,2-trichloroéthoxycarbonyl éthynyl) cyclopropane carboxylique.

On porte au reflux pendant une heure un mélange renfermant 11,4 g du produit préparé selon le stade A, 120 $cm^3$ de toluène et 300 mg d'acide paratoluène sulfonique. On laisse revenir à la température ambiante, lave le mélange réactionnel à l'eau, le sèche, et l'amène à sec. On obtient ainsi 9,5 g du produit recherché que l'on utilise tel quel dans le stade suivant.

Stade C : (1R, cis) 2,2-diméthyl 3(2,2,2-trichloro éthoxy carbonyl éthynyl) cyclopropane carboxylate de (S) α-cyano 3-phénoxybenzyle.

On ajoute 6,2 g de dicyclohexylcarbodiimide dans une solution renfermant 9,5 g du produit préparé au stade B, 30 $cm^3$ de chlorure de méthylène et 3 $cm^3$ de pyridine. On agite le mélange réactionnel pendant une demi-heure et ajoute 6,8 g d'alcool (S) α-cyano 3-phénoxy benzylique. On maintient sous agitation pendant une heure et demie. On élimine par filtration, l'insoluble formé. On lave le filtrat à l'acide chlorhydrique N puis à l'eau jusqu'à neutralité. On le sèche, le filtre et l'amène à sec. On obtient

ainsi 16,3 g d'une huile que l'on chromatographie sur silice en éluant par le mélange benzène-acétate d'éthyle (97-3). On isole ainsi 12 g du produit recherché fondant à 101 °C.

Stade D : (1R, cis) 2,2-diméthyl 3-(2-carboxy éthynyl) cyclopropane carboxylate de (S) α-cyano 3-phénoxybenzyle.

On introduit 5,9 g de poudre de zinc dans une solution renfermant 6,5 g du produit préparé au stade C, 23,4 $cm^3$ d'acide acétique et 2,6 $cm^3$ d'eau. On maintient le mélange sous agitation pendant une heure. On filtre et décante le filtrat. On lave la phase organique à l'eau et extrait la phase aqueuse au chlorure de méthylène. On réunit les solutions chlorométhyléniques, les sèche, les filtre et les amène à sec. On obtient ainsi 4,7 g de produit brut que l'on utilise tel quel dans le stade suivant.

Stade E : (1R, cis) 2,2-diméthyl 3-[(ΔZ) 2-carboxy éthényl] cyclopropane carboxylate de (S) α-cyano 3-phénoxybenzyle.

On hydrogène 4,7 g du produit préparé au stade D, dans 45 $cm^3$ d'acétate d'éthyle en présence de 500 mg d'hydroxyde de palladium à 10 % sur sulfate de baryum et 6,5 $cm^3$ de quinoléine. On filtre, lave le filtrat à l'acide chlorhydrique N, puis à l'eau jusqu'à neutralité, sèche et amène à sec. On obtient 5,1 g d'une huile que l'on chromatographie sur silice en éluant par le mélange hexane, acétate d'éthyle, acide acétique (70-30-1). On obtient ainsi 3,8 g du produit recherché que l'on utilise tel quel dans le stade suivant.

Stade F : (1R, cis) 2,2-diméthyl 3-[(Z) 2-terbutoxycarbonyl éthényl] cyclopropane carboxylate de (S) α-cyano 3-phénoxybenzyle.

On agite pendant 16 heures à la température ambiante un mélange renfermant 1,2 g du produit préparé au stade E, 6 $cm^3$ d'acétate d'éthyle et 1,2 g de O-terbutyl N,N'-diisopropylurée. On filtre et amène le filtrat à sec. On obtient 1,55 g d'une huile que l'on chromatographie sur silice en éluant par le benzène. On isole ainsi 600 mg du produit recherché fondant à 89 °C.

$(\alpha)_D = +61,5° \mp 2°$ (c = 0,5 % benzène)
RMN $CDCl_3$ ppm
1,9-2,04 proton du carbone en 1 du cyclopropane
3,22 à 3,52 proton du carbone en 3 du cyclopropane
1,23 protons des méthyles en 2 du cyclopropane
6,3 proton du carbone portent le C≡N
6,24 à 6,6 proton du carbone en 1 du radical éthényle
5,7-5,9 proton du carbone en 2 du radical éthényle
1,5 protons du terbutyle

Exemple 7 : (1R, cis) 2,2-diméthyl 3-[(Z) 2-(n-butoxycarbonyl) éthényl] cyclopropane carboxylate de (S) α-cyano 3-phénoxybenzyle.

Stade A : (1R, cis) 2,2-diméthyl 3-(n-butoxycarbonyl éthynyl) cyclopropane carboxylate de terbutyle.

On mélange 4 g de (1R, cis) 2,2-diméthyl 3-(2-carboxy éthynyl) cyclopropane carboxylate de terbutyle, 40 $cm^3$ de chlorure de méthylène et 6 mg de 4-diméthylaminopyridine puis on ajoute 3,4 g de dicyclohexylcarbodiimide. Après 30 minutes d'agitation sous atmosphère inerte, on ajoute, en 5 minutes, 4 $cm^3$ d'un mélange (1-1) de n-butanol et de chlorure de méthylène et maintient l'agitation pendant 3 heures à température ambiante. On filtre la dicyclohexylurée formée, concentre à sec le filtrat sous pression réduite et chromatographie le résidu sur silice, en éluant par un mélange cyclohexane-acétate d'éthyle (9-1). On obtient 4,7 g du produit attendu.

RMN ($CDCl_3$) ppm
1,22 et 1,4 protons des méthyles en 2 du cyclopropane
1,75 : proton en 1 et 3 du cyclopropane
4,15 (t) : proton en 1 du butoxyle
1,48 : protons du terbutyle

Stade B : (1R, cis) 2,2-diméthyl 3-[(Z) 2-(n-butoxycarbonyl) éthényl] cyclopropane carboxylate de terbutyle.

On agite sous hydrogène pendant 15 minutes 800 mg d'hydroxyde de palladium sur sulfate de baryum dans 20 $cm^3$ d'acétate d'éthyle puis on ajoute 4,7 g du produit obtenu ci-dessus dans 50 $cm^3$ d'acétate d'éthyle et 0,8 $cm^3$ de quinoléine et laisse sous hydrogène pendant 30 minutes. On élimine le catalyseur par filtration, lave le filtrat à l'acide chlorhydrique N puis à l'eau, sèche, concentre à sec sous

pression réduite et chromatographie le résidu sur silice en éluant avec un mélange cyclohexane-acétate d'éthyle (95-5). On obtient 3,4 g de produit attendu.

RMN ($CDCl_3$) ppm
1,25 et 1,28 : protons des méthyles en 2 du cyclopropane
1,76 et 1,90 : proton en 1 du cyclopropane
2,96 à 3,3 : proton en 3 du cyclopropane
6,45-6,6 et 6,6 et 6,8 : proton en 1 de la chaîne allylique
5,75 et 5,95 : proton en 2 de la chaîne allylique
4,12 (t) : proton en 1 du terbutoxyle
1,45 : protons du terbutyle

Stade C : Acide (1R, cis) 2,2-diméthyl 3-[(Z) 2-(n-butoxycarbonyl) éthényl] cyclopropane carboxylique.

On agite 3,3 g de produit obtenu ci-dessus avec 350 mg d'acide paratoluènesulfonique dans 40 cm$^3$ de toluène. On chauffe au reflux jusqu'à cessation de dégagement gazeux d'isobutylène soit environ 40 minutes. On concentre à sec sous pression réduite, chromatographie le résidu sur silice en éluant par un mélange cyclohexane-acétate d'éthyle-acide acétique (75-25-1).

On obtient 2 g de produit attendu.

RMN ($CDCl_3$) ppm
1,26 et 1,3 : protons des méthyles en 2 du cyclopropane
1,85-1,99 : proton en 1 du cyclopropane
3,13 à 3,47 : proton en 3 du cyclopropane
6,4-6,57 et 6,59-6,75 : proton en 1 de la chaîne allylique
5,8-5,99 : proton en 2 de la chaîne allylique

Stade D : (1R, cis) 2,2-diméthyl 3-[(Z) 2-(n-butoxycarbonyl) éthényl] cyclopropane carboxylate de (S) α-cyano 3-phénoxybenzyle.

On agite, sous atmosphère inerte, 2 g d'acide (1R, cis) 2,2-diméthyl 3-[(Z) 2-(n-butoxycarbonyl) éthényl] cyclopropane carboxylique, 20 cm$^3$ de chlorure de méthylène et 1,1 cm$^3$ de pyridine puis on ajoute 1,7 g de dicyclohexylcarbodiimide et maintient l'agitation pendant 30 minutes. On ajoute 3 cm$^3$ de chlorure de méthylène contenant 2 g d'alcool (S) α-cyano 3-phénoxybenzylique et agite pendant 16 heures à température ambiante. On filtre la dicyclohexylurée formée, concentre à sec le filtrat, chromatographie le résidu sur silice en éluant avec un mélange n-hexane-éther isopropylique (8-2) et obtient 3,1 g de produit attendu.

$(\alpha)_D = + 51° \pm 2$ (c = 0,4 % dans benzène)
RMN ($CDCl_3$) ppm
1,25-1,27 : protons des méthyles en 2 du cyclopropane
1,92-2,07 : proton en 1 du cyclopropane
3,22-3,37-3,53 : proton en 3 du cyclopropane
6,5 (t) : proton en 1 de la chaîne allylique
5,8-5,85 : proton en 2 de la chaîne allylique
6,35 : proton porté par le même carbone que le CN

Exemple 8 : (1R, cis) 2,2-diméthyl 3-[(Z) 2-(pentyloxycarbonyl) éthényl] cyclopropane carboxylate de (S) α-cyano 3-phénoxybenzyle.

Stade A : (1R, cis) 2,2-diméthyl 3-[pentyloxycarbonyl éthynyl] cyclopropane carboxylate de terbutyle.

On opère comme décrit au stade A de l'exemple 7 en partant de 3 g de (1R, cis) 2,2-diméthyl 3-(2-carboxyéthynyl) cyclopropane carboxylate de terbutyle et 2 cm$^3$ d'alcool n amylique. Après chromatographie du résidu sur silice en éluant avec un mélange hexane-éther isopropylique (8-2) on obtient 2,7 g de produit attendu.

Stade B : Acide (1R, cis) 2,2-diméthyl 3-(pentoxycarbonyléthynyl) cyclopropane carboxylique.

On mélange 5,24 g du produit obtenu ci-dessus, 50 cm$^3$ de toluène et 250 mg d'acide paratoluènesulfonique. On porte au reflux jusqu'à cessation du dégagement gazeux puis concentre à sec sous pression réduite. On obtient 4,8 g de produit brut utilisé tel quel pour la suite de la synthèse.

Stade C : Acide (1R, cis) 2,2-diméthyl 3-[(Z) 2-pentyloxycarbonyl éthényl] cyclopropane carboxylique.

On hydrogène 4,8 g de produit obtenu précédemment dans 50 cm$^3$ d'acétate d'éthyle et 1 cm$^3$ de quinoléine en présence d'1 g de sulfate de baryum contenant 10 % d'hydroxyde de palladium en suspension dans 50 cm$^3$ d'acétate d'éthyle. On élimine le catalyseur, lave le filtrat à l'acide chlorhydrique N puis à l'eau, sèche, concentre à sec sous pression réduite et chromatographie le résidu sur silice en

éluant avec un mélange de cyclohexane-acétate d'éthyle-acide acétique (70-30-1). On obtient 3 g de produit attendu.

RMN ($CDCl_3$) ppm
1,3 et 1,32 : protons des méthyles en 2 du cyclopropane
1,85 et 1,99 : proton en 1 du cyclopropane
3,15 à 3,45 : proton en 3 du cyclopropane
6,4 à 6,75 : proton en 1 de la chaîne allylique
5,78-5,96 : proton en 2 de la chaîne allylique

Stade D : (1R, cis) 2,2-diméthyl 3-[(Z) 2-pentyloxycarbonyl éthényl] cyclopropane carboxylate de (S) α-cyano 3-phénoxybenzyle.

On opère comme à l'exemple 7 à partir de 3,05 g d'acide (1R, cis) 2,2-diméthyl 3-[(Z) 2-(pentyxolycarbonyl) éthényl] cyclopropane carboxylique, 1,5 $cm^3$ de pyridine, 2,4 g de dicyclohexylcarbodiimide et 2,7 g d'alcool (S) α-cyano 3-phénoxybenzylique dans 20 $cm^3$ de chlorure de méthylène. On chromatographie une deuxième fois sur silice en éluant avec un mélange hexane-éther éthylique (9-1) et obtient 3,5 g de produit attendu.

RMN ($CDCl_3$) ppm
1,25-1,27 : protons des méthyles en 2 du cyclopropane
1,92-2,07 : proton en 1 du cyclopropane
3,37 (t) : proton en 3 du cyclopropane
6,3-6,5 et 6,53-6,7 : proton en 1 de la chaîne allylique
5,8-6 : proton en 2 de la chaîne allylique
6,3 : proton porté par le même carbone que CN
6,9 à 7,6 : protons aromatiques

Exemple 9 : (1R, cis) 2,2-diméthyl 3-[(Z) 2-((RS) 1-méthyl-propyloxycarbonyl) éthényl] cyclopropane carboxylate de (S) α-cyano 3-phénoxybenzyle.

Stade A : (1R, cis) 2,2-diméthyl 3-[(RS) 1-méthylpropyloxycarbonyl éthynyl] cyclopropane carboxylate de terbutyle.

On opère comme indiqué au stade A de l'exemple 7 en utilisant, cette fois, 2 $cm^3$ d'alcool 1-méthylpropanol. On chromatographie le résidu sur silice en éluant par un mélange n-hexane-éther isopropylique (8-2) et obtient 3,5 g de produit attendu.

RMN ($CDCl_3$) ppm
1,2-1,4 : protons des méthyles en 2 du cyclopropane
1,73 : protons en 1 et 3 du cyclopropane
4,92 : proton en 1 du propyle

Stade B : (1R, cis) 2,2-diméthyl 3-[(Z) 2-((RS) 1-méthylpropyloxycarbonyl) éthényl] cyclopropane carboxylate de terbutyle.

On hydrogène 3 g du produit obtenu précédemment comme indiqué dans l'exemple 7 (Stade B). On chromatographie sur silice en éluant par un mélange n-hexane-éther isopropylique (9-1) et obtient 2,5 g de produit attendu.

Stade C : Acide (1R, cis) 2,2-diméthyl 3-[(Z) 2-((RS) 1-méthyl propyloxycarbonyl) éthényl] cyclopropane carboxylique.

On opère comme au stade C de l'exemple 7 à partir de 3 g de produit obtenu précédemment. On chromatographie le résidu sur silice en éluant par un mélange cyclohexane-acétate d'éthyle-acide acétique (70-30-1) et obtient 1,85 g de produit attendu.

Spectre IR ($CHCl_3$)
—OH : acide 3 510 $cm^{-1}$
—C=O : acide 1 735 $cm^{-1}$
ester 1 710 $cm^{-1}$-1 700 $cm^{-1}$
C=C : conj 1 637 $cm^{-1}$
gem. di Me 1 381 $cm^{-1}$

Stade D : (1R, cis) 2,2-diméthyl 3-[(Z) 2-((RS) 1-méthylpropyloxycarbonyl) éthényl] cyclopropane carboxylate de (S) α-cyano 3-phénoxybenzyle.

On opère comme à l'exemple 7 à partir de 1,83 g d'acide (1R, cis) 2,2-diméthyl 3-[(Z) 2-((RS) 1-méthylpropyloxycarbonyl) éthényl] cyclopropane carboxylique. On obtient 2,6 g de produit attendu.

$(\alpha)_D = + 48° \pm 2,5$ (c = 0,5 benzène)
RMN ($CDCl_3$) ppm
1,22-1,23 : protons des méthyles en 2 du cyclopropane
1,88-2,02 : proton en 1 du cyclopropane
3,21 à 3,5 : proton en 3 du cyclopropane
6,35 à 6,68 : proton en 1 de la chaîne allylique
5,8 à 6 : proton en 2 de la chaîne allylique
4,92 (m) : proton en 1 du propyle
6,35 : proton porté par le même carbone que CN

Exemple 10 : (1R, cis) 2,2-diméthyl 3-[(Z) 2-(isobutoxycarbonyl) éthényl] cyclopropane carboxylate de (S) α-cyano 3-phénoxybenzyle.

Stade A : (1R, cis) 2,2-diméthyl 3-[isobutoxycarbonyléthynyl] cyclopropane carboxylate de terbutyle.

On opère comme dans l'exemple 7 en utilisant 2 $cm^3$ d'alcool isobutylique et obtient 4,6 g de produit attendu.
RMN ($CDCl_3$) ppm
1,22-1,4 : protons des méthyles en 2 du cyclopropane
3,88-4 : proton en 1 de l'isobutyle

Stade B : Acide (1R, cis) 2,2-diméthyl 3-[isobutoxycarbonyléthynyl] cyclopropane carboxylique

On chauffe au reflux 4,6 g du produit précédent et 450 mg d'acide paratoluènesulfonique dans 50 $cm^3$ de toluène pendant 45 minutes. On concentre à sec sous pression réduite, chromatographie le résidu sur silice en éluant par un mélange cyclohexane-acétate d'éthyle-acide acétique (70-30-1) et obtient 2,9 g de produit attendu.
RMN ($CDCl_3$) ppm
1,25-1,27 : protons des méthyles en 2 du cyclopropane
~ 1,9 : proton en 1 et 3 du cyclopropane
7,9 : proton du carboxyle en 1
3,88-4 : proton en 1 de l'isobutyle

Stade C : (1R, cis) 2,2-diméthyl 3-[isobutoxycarbonyléthynyl] cyclopropane carboxylate de (S) α-cyano 3-phénoxybenzyle.

On opère comme dans l'exemple 7 en partant de 2,4 g du produit obtenu au stade B. On chromatographie sur silice en éluant par un mélange cyclohexane-acétate d'éthyle (8-2) et obtient 3,3 g de produit attendu.
RMN ($CDCl_3$) ppm
1,33-1,23 : protons des méthyles en 2 du cyclopropane
1,95 : protons en 1 et 3 du cyclopropane
6,45 : proton porté par le même carbone que CN

Stade D : (1R, cis) 2,2-diméthyl 3-[(Z) 2-(isobutoxycarbonyl) éthényl] cyclopropane carboxylate de (S) α-cyano 3-phénoxybenzyle.

On hydrogène 1,4 g de (1R, cis, 2,2-diméthyl 3-[2-isobutoxy carbonyl) éthynyl] cyclopropane carboxylate de (S) α-cyano 3-phénoxybenzyle d'une manière analogue à celle décrite dans l'exemple 7 au stade B. On purifie par chromatographie sur silice en éluant par un mélange n-hexane-éther isopropylique (8-2) et obtient 1,23 g de produit attendu.
$(\alpha)_D = + 57,5 \pm 4°$ (c = 0,4 % dans benzène)
RMN ($CDCl_3$) ppm
1,25 : protons des méthyles en 2 du cyclopropane
1,9-2,0 : proton en 1 du cyclopropane
3,2 à 3,5 : proton en 3 du cyclopropane
6,3 à 6,6 : proton en 1 de la chaîne allylique
5,8-6 : proton en 2 de la chaîne allylique
6,3 : proton porté par le même carbone que CN

Exemple 11 : (1R, cis) 2,2-diméthyl 3-[(Z) 2-(cyclohexyloxycarbonyl) éthényl] cyclopropane carboxylate de (S) α-cyano 3-phénoxybenzyle.

Stade A : (1R, cis) 2,2-diméthyl 3-[cyclohexyloxycarbonyléthynyl] cyclopropane carboxylate de terbutyle.

On opère comme indiqué dans l'exemple 7 en utilisant 2 $cm^3$ de cyclohexanol. On obtient 3,67 g de produit attendu.

Spectre IR ($CHCl_3$)
C≡C : conj 2 225 $cm^{-1}$
C=O : ester + conj 1 729 $cm^{-1}$-1 700 $cm^{-1}$
gem di ME 1 392 $cm^{-1}$-1 380 $cm^{-1}$
terbut 1 370 $cm^{-1}$

Stade B : (1R, cis) 2,2-diméthyl 3-[(Z) 2-(cyclohexyloxycarbonyl) éthényl] cyclopropane carboxylate de terbutyle.

On opère comme indiqué dans l'exemple 7 à partir de 3,67 g de produit obtenu ci-dessus et obtient 3,4 g de produit brut utilisé tel quel pour le stade suivant.

Spectre IR ($CHCl_3$)
C = O : 1 715 $cm^{-1}$
C = C : 1 634 $cm^{-1}$

Stade C : Acide (1R, cis) 2,2-diméthyl 3-[(Z) 2-(cyclohexyloxycarbonyl) éthényl] cyclopropane carboxylique.

On opère comme indiqué dans l'exemple 7 en partant de 3,404 g de produit obtenu précédemment et en utilisant le système cyclo-hexane-acétate d'éthyle-acide acétique (70-30-1) pour la chromatographie. On obtient 2,5 g de produit attendu.

Spectre IR ($CHCl_3$)
—OH acide 3 510 $cm^{-1}$
—C = O acide 1 735 $cm^{-1}$
ester 1 707 $cm^{-1}$
— gem di Me 1 380 $cm^{-1}$
—C=C— : 1 638 $cm^{-1}$

Stade D : (1R, cis) 2,2-diméthyl 3-[(Z) 2-(cyclohexyloxycarbonyl) éthényl] cyclopropane carboxylate de (S) α-cyano 3-phénoxybenzyle.

On opère comme décrit à l'exemple 7 à partir de 2,5 g d'acide (1R, cis) 2,2-diméthyl 3-[(Z) 2-(cyclohexyloxycarbonyl) éthényl] cyclopropane carboxylique et de 2,2 g d'alcool α-cyano 3-phénoxybenzylique et en éluant par un mélange hexane-éther éthylique (9-1). On obtient 1,883 g de produit attendu.

$(\alpha)_D = +41° \pm 2,5$ (c = 0,5 % $CHCl_3$)
RMN ($CDCl_3$) ppm
1,26 : protons des méthyles en 2 du cyclopropane
1,92-2,05 : proton en 1 du cyclopropane
3,23-3,55 : proton en 3 du cyclopropane
6,3 à 6,6 : proton en 1 de la chaîne allylique
5,8-5,99 : proton en 2 de la chaîne allylique
6,3 : proton porté par le même carbone que CN
4,8 : proton en 1 du cyclohexyle

Exemple 12 : (1R, cis) 2,2-diméthyl 3-[(Z) 2-cyclopropylméthoxy carbonyl) éthényl] cyclopropane carboxylate de (S) α-cyano 3-phénoxybenzyle.

Stade A : (1R, cis) 2,2-diméthyl 3-(cyclopropylméthoxycarbonyl éthynyl) cyclopropane carboxylate de (S) α-cyano 3-phénoxybenzyle.

On opère comme à l'exemple 7 au stade D en partant de 1,4 g de (1R, cis) 2,2-diméthyl 3-(2-carboxyéthynyl) cyclopropane carboxylate de (S) α-cyano 3-phénoxybenzyle obtenu à l'exemple 6 au stade D et 0,3 $cm^3$ de cyclopropylcarbinol. Après chromatographie sur silice en éluant par un mélange cyclohexane-acétate d'éthyle (8-2), on obtient 1,5 g de produit attendu. F = 72 °C.

Spectre IR ($CHCl_3$)
—C≡C— conj 2 235 $cm^{-1}$
—C=O ester { 1 754 $cm^{-1}$
ester conj { 1 704 $cm^{-1}$
Gem di Me { 1 390 $cm^{-1}$ / 1 380 $cm^{-1}$

Stade B : (1R, cis) 2,2-diméthyl 3-[(Z) 2-(cyclopropylméthoxycarbonyl) éthényl] cyclopropane carboxylate de (S) α-cyano 3-phénoxybenzyle.

On opère de la même manière qu'au stade B de l'exemple 7 en partant de 1,5 g du produit obtenu ci-dessus, chromatographie sur silice en éluant par un mélange n-hexane-éther isopropylique (8-2) et obtient 1,2 g de produit attendu.

$(\alpha)_D = +48,5° \pm 2,5$ (c = 0,5 % benzène)

RMN ($CDCl_3$) ppm

1,27 : protons des méthyles en 2 du cyclopropane

1,92-2,06 : proton en 1 du cyclopropane

3,2 à 3,5 : proton en 3 du cyclopropane

6,3 à 6,75 : proton en 1 de la chaîne allylique

5,9-6 : proton en 2 de la chaîne allylique

6,37 : proton porté par le même carbone que CN

Exemple 13 : (1R, cis) 2,2-diméthyl 3-[(Z) 2-(cyclobutyloxycarbonyl) éthényl] cyclopropane carboxylate de (S) α-cyano 3-phénoxybenzyle.

Stade A : (1R, cis) 2,2-diméthyl 3-[(Z) 2-(cyclobutyloxycarbonyl) éthényl] cyclopropane carboxylate de terbutyle.

On dissout 4 g de (1R, cis) 2,2-diméthyl 3-[(Z) 2-carboxy-éthényl] cyclopropane carboxylate de terbutyle dans 20 $cm^3$ de chlorure de méthylène puis on ajoute 1,7 $cm^3$ de cyclobutanol. On laisse la température à 0/+ 5 °C, ajoute 3,45 g de dicyclohexylcarbodiimide, 28 mg de diméthylaminopyridine dans 20 $cm^3$ de chlorure de méthylène et maintient l'agitation pendant 2 heures à 5 °C environ et 2 heures à température ambiante. On élimine la dicyclohexylurée formée, concentre à sec le filtrat et chromatographie sur silice en éluant par un mélange n-hexane-éther isopropylique (9-1). On obtient 2,3 g de produit attendu.

RMN ($CDCl_3$) ppm

1,23-1,26 : protons des méthyles en 2 du cyclopropane

1,77-1,9 : proton en 1 du cyclopropane

2,95 à 3,28 : proton en 3 du cyclopropane

6,4 à 6,8 : proton en 1 de la chaîne allylique

5,7-5,9 : proton en 2 de la chaîne allylique

5 : proton en 1 du cyclobutyle

Stade B : Acide (1R, cis) 2,2-diméthyl 3-[(Z) 2-(cyclobutyloxycarbonyl) éthényl] cyclopropane carboxylique.

On chauffe au reflux pendant 15 minutes 2,3 g du produit obtenu précédemment, 25 $cm^3$ de toluène et 250 mg d'acide paratoluènesulfonique, refroidit et agite pendant 2 heures à 0/+ 5 °C. On filtre l'insoluble et concentre à sec le filtrat pour obtenir 1,8 g de produit attendu.

Spectre IR

—OH : acide 3 500 $cm^{-1}$

—C=O : acide 1 733 $cm^{-1}$

+ ester conj 1 702 $cm^{-1}$

gem di Me $\begin{cases} 1\,390\ cm^{-1} \\ 1\,380\ cm^{-1} \end{cases}$

Stade C : (1R, cis) 2,2-diméthyl 3-[(Z) 2-(cyclobutyloxycarbonyl) éthényl] cyclopropane carboxylate de (S) α-cyano 3-phénoxybenzyle.

On opère comme au stade A ci-dessus à partir de 1,8 g du produit précédent et 2 g d'alcool (S) α-cyano 3-phénoxybenzylique. On effectue deux chromatographies successives sur silice en éluant par un mélange cyclohexane-acétate d'éthyle (9-1) puis un second mélange n-hexane-éther isopropylique (8-2). On obtient 3 g de produit attendu.

$(\alpha)_D = +45,5° \pm 2°$ (c = 0,6 % $CHCl_3$)

RMN ($CDCl_3$) ppm

1,25-1,26 : protons des méthyles en 2 du cyclopropane

3,4 : proton en 3 du cyclopropane

6,5 à 6,7 : proton en 1 de la chaîne allylique

5,8-6,0 : proton en 2 de la chaîne allylique - 5,1 : proton en 1 du cyclobutyle - 6,4 : proton porté par le même carbone que CN

Exemple 14 : (1R, cis, 2,2-diméthyl 3-[(Z) 2-(cyclopentyloxycarbonyl) éthényl] cyclopropane carboxylate de (S) α-cyano 3-phénoxybenzyle.

Stade A : (1R, cis, 2,2-diméthyl 3-[2-(cyclopentyloxycarbonyl) éthynyl] cyclopropane carboxylate de terbutyle.

On opère comme indiqué au stade A de l'exemple 7 à partir de 5,8 g de (1R cis) 2,2-diméthyl 3-[2-carboxyéthynyl] cyclopropane carboxylate de terbutyle et 2,2 $cm^3$ de cyclopentanol. On chromatographie sur silice en éluant par un mélange hexane-éther isopropylique (7-3) et obtient 4,8 g de produit attendu.

Spectre IR ($CHCl_3$)
Absence d'OH
C≡conj. 2 230 $cm^{-1}$
C=O 1 727 $cm^{-1}$-1 695 $cm^{-1}$
gem. diméthyle { 1 395 $cm^{-1}$ ; 1 380 $cm^{-1}$ }
t bu 1 372 $cm^{-1}$

Stade B : Acide (1R, cis) 2,2-diméthyl 3-[2-cyclopentyloxycarbonyléthynyl] cyclopropane carboxylique.

On chauffe au reflux pendant 10 minutes 4,8 g de produit obtenu au stade A dans 50 $cm^3$ de toluène et 500 mg d'acide paratoluènesulfonique. On refroidit et lave à l'eau le mélange réactionnel, sèche et concentre à sec sous pression réduite. On obtient 3,6 g de produit utilisé tel quel dans le stade suivant.

Stade C : (1R, cis) 2,2-diméthyl 3-[2-cyclopentyloxycarbonyl-éthynyl] cyclopropane carboxylate de (S) α-cyano 3-phénoxybenzyle.

On agite 3,6 g du produit obtenu ci-dessus, 30 $cm^3$ de chlorure de méthylène et 30 mg de diméthylaminopyridine. On refroidit à 0 °C et ajoute 2,96 g de dicyclohexylcarbodiimide puis 3,3 g d'alcool (S) α-cyano 3-phénoxybenzylique dans 10 $cm^3$ de chlorure de méthylène. On agite 5 minutes à 0 °C puis 3 heures à 20-25 °C. On filtre la dicyclohexylurée formée et concentre à sec le filtrat sous pression réduite. On chromatographie le résidu sur silice en éluant par un mélange cyclohexane-acétate d'éthyle et obtient 4,5 g de produit attendu. F ≃ 72 °C.

RMN ($CDCl_3$) ppm
1,25 et 1,37 : protons des méthyles en 2 du cyclopropane
1,95 : protons en 1 et 3 du cyclopropane
6,65 : proton porté par le même carbone que CN
5,25 : proton en 1 du cyclopentyle

Stade D : (1R, cis) 2,2-diméthyl 3-[(Z) 2-(cyclopentyloxycarbonyl) éthényl] cyclopropane carboxylate de (S) α-cyano 3-phénoxybenzyle.

On opère comme indiqué à l'exemple 10 stade D à partir de 3 g du composé ci-dessus, chromatographie sur silice en éluant par un mélange cyclohexane-acétate d'éthyle (85-15) et isole 2,2 g de produit attendu.

$(\alpha)_D$ = + 43,5 ° ± 2,5 (c = 0,5 % benzène)
RMN ($CDCl_3$) ppm
1,27-1,28 : protons des méthyles en 2 du cyclopropane
1,92-2,06 : proton en 1 du cyclopropane
3,25 à 3,57 : proton en 3 du cyclopropane
6,38 à 6,7 : proton en 1 de la chaîne allylique
5,83-6,0 : proton en 2 de la chaîne allylique
5,25 : proton en 1 du cyclopentyle
6,38 : proton porté par le même carbone que CN

Exemple 15 : (1R, cis) 2,2-diméthyl 3-[(Z) 2-(téramyloxycarbonyl) éthényl] cyclopropane carboxylate de (S) α-cyano 3-phénoxybenzyle.

Stade A : (1R, cis) 2,2-diméthyl 3-[2-téramyloxycarbonyléthynyl] cyclopropane carboxylate de (S) α-cyano 3-phénoxybenzyle.

On agite 2,5 g de (1R, cis) 2,2-diméthyl 3-(2-carboxyéthynyl) cyclopropane carboxylate de (S) α-cyano 3-phénoxybenzyle dans 12,5 $cm^3$ de chlorure de méthylène et 2,5 $cm^3$ d'alcool téramylique, refroidit à 5 °C environ et ajoute 82 mg de N,N-diméthylaminopyridine puis 1,55 g de dicyclohexylcarbodiimide et maintient l'agitation pendant 4 heures 30 minutes à 20 °C. Après refroidissement à 0 °C, on filtre l'insoluble et concentre le filtrat sous pression réduite. On chromatographie le résidu sur silice en éluant par un mélange cyclchexane-acétate d'éthyle (8-2). On obtient 1,35 g de produit attendu.

Spectre IR ($CHCl_3$)

—C≡C : 2 235 $cm^{-1}$
—C=O : ester 1 756 $cm^{-1}$
ester conj 1 699 $cm^{-1}$
aromatiques { 1 589 $cm^{-1}$ ; 1 489 $cm^{-1}$ }
gem diméthyle { 1 392 $cm^{-1}$ ; 1 382 $cm^{-1}$ }

Stade B : (1R, cis) 2,2-diméthyl 3-[(Z) 2-(téramyloxycarbonyl) éthényl] cyclopropane carboxylate de (S) α-cyano 3-phénoxybenzyle.

On hydrogène pendant 30 minutes 1,34 g du produit obtenu ci-dessus, dissous dans 20 $cm^3$ d'acétate d'éthyle, avec 220 mg d'hydroxyde de palladium à 10 % dans du sulfate de baryum et 0,25 $cm^3$ de quinoléine. On élimine le catalyseur, concentre à sec le filtrat et chromatographie le résidu sur silice en éluant par un mélange cyclohexane-acétate d'éthyle (9-1) et obtient 1,05 g de produit attendu.

$(\alpha)_D = +63{,}5° \pm 2{,}5°$ (c = 0,7 % $CHCl_3$)
RMN ($CDCl_3$) ppm
1,26-1,27 : protons des méthyles en 2 du cyclopropane
6,28-6,45 et 6,48-6,65 : proton en 1 de la chaîne allylique
5,79-5,82 : proton en 2 de la chaîne allylique
6,38 : proton porté par le même carbone que CN
6,98-7,67 : protons aromatiques
1,48 : protons des méthyles en 1 du téramyle
0,9 (t) : proton du méthyle en 3 du téramyle

Exemple 16 : (1R, cis) 2,2-diméthyl 3-[(Z) 2-(1-méthylcyclobutyloxycarbonyl) éthényl] cyclopropane carboxylate de (S) α-cyano 3-phénoxybenzyle.

Stade A : (1R, cis) 2,2-diméthyl 3-[2-(1-méthylcyclobutyloxycarbonyl) éthynyl] cyclopropane carboxylate de (S) α-cyano 3-phénoxybenzyle.

On opère comme indiqué à l'exemple 15 à partir de 3,9 g de (1R, cis) 2,2-diméthyl 3-(2-carboxyéthynyl) cyclopropane carboxylate de (S) α-cyano 3-phénoxybenzyle et 4,3 g de 1-méthylcyclobutanol. L'agitation dure 18 heures à 20 °C. On chromatographie avec un mélange cyclohexane-acétate d'éthyle (85-15). On obtient 2,7 g de produit attendu. F = 76 °C.

RMN ($CDCl_3$) ppm
1,23-1,35 : protons des méthyles en 2 du cyclopropane
1,93 : proton en 1 et 3 du cyclopropane
1,58 : proton du méthyle en 1 du cyclobutyle
6,55 : proton porté par le même carbone que CN

Stade B : (1R, cis) 2,2-diméthyl 3-[(Z) 2-(1-méthylcyclobutyloxycarbonyl) éthényl] cyclopropane carboxylate de (S) α-cyano 3-phénoxybenzyle.

On opère comme indiqué à l'exemple 15, en 15 minutes, à partir de 2,7 g de produit obtenu ci-dessus. Le système éluant est un mélange de cyclohexane-acétate d'éthyle (95-5). On obtient 2,38 g de produit attendu. F = 78 °C.

RMN ($CDCl_3$) ppm
1,27 : protons des méthyles en 2 du cyclopropane
3,23 à 3,55 : proton en 3 du cyclopropane
6,23 à 6,68 : proton en 1 de la chaîne allylique
5,79-5,97 : proton en 2 de la chaîne allylique
1,6 : proton du méthyle en 1 du cyclobutyle
6,38 : proton porté par le même carbone que CN
6,97 à 7,6 : protons aromatiques

Exemple 17 : (1R, cis) 2,2-diméthyl 3-[(Z) 2-(néopentyloxycarbonyl) éthényl] cyclopropane carboxylate de (S) α-cyano 3-phénoxybenzyle.

Stade A : (1R, cis) 2,2-diméthyl 3-[2-néopentyloxycarbonyléthynyl] cyclopropane carboxylate de (S) α-cyano 3-phénoxybenzyle.

On opère comme à l'exemple 15 à partir de 3,89 g de (1R, cis) 2,2-diméthyl 3-(2-carboxyéthynyl) cyclopropane carboxylate de (S) α-cyano 3-phénoxybenzyle et 1 g d'alcool néopentylique. On agite 4 heures à température ambiante, filtre, lave le filtrat à l'acide chlorhydrique N puis à l'eau, sèche et

concentre à sec sous pression réduite. Le résidu est chromatographié sur silice. On élue par un mélange cyclohexane-acétate d'éthyle (8-2) et on obtient 3,8 g de produit attendu. F = 67 °C.

RMN ($CDCl_3$) ppm
1,25 et 1,36 : protons des méthyles en 2 du cyclopropane
1,95 : protons en 1 et 3 du cyclopropane
3,9 : protons en 1 du néopentyle
0,97 : protons des méthyles du néopentyle
6,53 : proton porté par le même carbone que CN

Stade B : (1R, cis) 2,2-diméthyl 3-[(Z) 2-(néopentyloxycarbonyl) éthényl] cyclopropane carboxylate de (S) α-cyano 3-phénoxybenzyle

On utilise la même technique qu'à l'exemple 15 pour hydrogéner 2,6 g du produit obtenu ci-dessus. Après élimination du catalyseur, le filtrat est lavé avec de l'acide chlorhydrique N puis à l'eau, séché puis concentré à sec sous pression réduite. On chromatographie le résidu sur silice 2 fois — 1er éluant : cyclohexane-acétate d'éthyle (8-2) — 2ème éluant : cyclohexane-acétate d'éthyle (95-5). On obtient 2 g de produit attendu.

$(\alpha)_D = +52{,}5° \pm 2{,}5°$ (c = 0,5 % benzène)
RMN ($CDCl_3$) ppm
1,27-1,275 : protons des méthyles en 2 du cyclopropane
1,92-2,07 : proton en 1 du cyclopropane
3,25 à 3,58 : proton en 3 du cyclopropane
6,4 à 6,75 : proton en 1 de la chaîne allylique
5,9-6,1 : proton en 2 de la chaîne allylique
6,4 : proton porté par le même carbone que CN
3,87 : protons en 1 du néopentyle
0,97 : protons des méthyles du néopentyle.

Exemple 18 : (1R, cis) 2,2-diméthyl 3-[(Z) 2-(1-méthylcyclopentyloxycarbonyl) éthényl] cyclopropane carboxylate de (S) α-cyano 3-phénoxybenzyle

Stade A : (1R, cis) 2,2-diméthyl 3-[2-(1-méthylcyclopentyloxycarbonyl) éthynyl] cyclopropane carboxylate de (S) α-cyano 3-phénoxybenzyle

On opère comme à l'exemple 15 à partir de 3 g de (1R, cis) 2,2-diméthyl 3-(2-carboxyéthynyl) cyclopropane carboxylate de (S) α-cyano 3-phénoxybenzyle et 3 g de 1-méthyl cyclopentanol. Après chromatographie sur silice en éluant par un mélange n-hexane-acétate d'éthyle (8-2), on obtient 2 g de produit attendu.

Spectre IR ($CHCl_3$)

| | |
|---|---|
| —C≡C- conj | 2 230 $cm^{-1}$ |
| —C=O ester | 1 765 $cm^{-1}$ |
| ester conj | 1 695 $cm^{-1}$ |
| Aromatiques | 1 585 $cm^{-1}$ |
| | 1 485 $cm^{-1}$ |
| gem diméthyle | 1 390 $cm^{-1}$ |
| | 1 380 $cm^{-1}$ |

Stade B : (1R, cis) 2,2-diméthyl 3-[(Z) 2-(1-méthylcyclopentyloxycarbonyl) éthényl] cyclopropane carboxylate de (S) α-cyano 3-phénoxybenzyle

On opère comme à l'exemple 15 à partir de 2 g du produit ci-dessus. L'éluant utilisé est un mélange hexane-acétate d'éthyle (9-1). On obtient 1,5 g de produit attendu.

$(\alpha)_D = +61{,}5° \pm 2{,}5$ (c = 0,5 % $CHCl_3$)
RMN ($CDCl_3$) ppm
1,25-1,26 : protons des méthyles en 2 du cyclopropane
1,91-2,05 : proton en 1 du cyclopropane
3,4 (t) : proton en 3 du cyclopropane
6,3 à 6,7 : proton en 1 de la chaîne allylique
5,8-6 : proton en 2 de la chaîne allylique
1,61 : protons du méthyle en 1 du cyclopentyle
6,38 : proton porté par le même carbone que CN
1,67 : protons du cyclopentyle
6,9 à 7,6 : protons aromatiques

Exemple 19 : (1R, cis) 2,2-diméthyl 3-[(Z) 2-(1-RS-cyclopropyléthoxycarbonyl) éthényl] cyclopropane carboxylate de RS α-cyano 3-phénoxybenzyle

On opère comme indiqué à l'exemple 13 à partir de 2 g de (1R, cis) 2,2-diméthyl 3-[(Z) 2-carboxyéthényl] cyclopropane carboxylate de (S) α-cyano 3-phénoxybenzyle et 0,8 $cm^3$ de 1-RS-cyclopropyléthanol. On agite 3 heures à 12 °C. Après chromatographie, on élue avec un mélange hexane-éther isopropylique (8-2) et obtient 0,960 g de produit attendu.

$(\alpha)_D = +42° \pm 2,5$ (c = 0,6 % $CHCl_3$)
RMN ($CDCl_3$) ppm
1,25-1,28 : protons des méthyles en 2 du cyclopropane
3,25 à 3,58 : proton en 3 du cyclopropane
1,92-2,06 : proton en 1 du cyclopropane
6,4 à 6,75 : proton en 1 de la chaîne allylique
4,43 : proton en 1 de l'éthoxyle
6,4 : proton porté par le même carbone que CN
1,28-1,38 : protons en 2 de l'éthoxyle

Exemple 20 : (1R, cis) 2,2-diméthyl 3-[(Z) 2-(2-isopropylpropanoxycarbonyl) éthényl] cyclopropane carboxylate de (S) α-cyano 3-phénoxybenzyle

On mélange 3 $cm^3$ de 1-diméthylamino-1-chloro-2-méthyl propène 1-yle (décrit dans J. Org. Chem. 1970, *35,* 3 970), 3 $cm^3$ de chlorure de méthylène puis ajoute 3 g de (1R, cis) 2,2-diméthyl 3-[(Z) 2-carboxyéthényl] cyclopropane carboxylate de (S) α-cyano 3-phénoxybenzyle dans 30 $cm^3$ de chlorure de méthylène. On agite 15 minutes à température ambiante, refroidit à + 5 °C et ajoute 5 $cm^3$ de 2-isopropyl propanol. On maintient l'agitation pendant 16 heures à température ambiante. On lave à l'eau, puis à l'eau salée, sèche et concentre à sec sous pression réduite. On chromatographie le résidu sur silice en éluant par un mélange cyclohexane-acétate d'éthyle (9-1) et obtient 2,25 g de produit attendu.

$(\alpha)_D = +61,5° \pm 2,5$ (c = 0,5 % benzène)
RMN ($CDCl_3$) ppm
1,27 : protons des méthyles en 2 du cyclopropane
3,25 à 3,57 : proton en 3 du cyclopropane
1,90-2,05 : proton en 1 du cyclopropane
6,3 à 6,6 : proton en 1 de la chaîne allylique
5,8-5,9 : proton en 2 de la chaîne allylique
2,28 : proton en 2 de l'isopropyle propanyle
6,4 : proton porté par le même carbone que CN

Exemple 21 : (1R, cis) 2,2-diméthyl 3-[(Z) 2-(1,1-diméthylallyloxycarbonyl) éthényl] cyclopropane carboxylate de (S) α-cyano 3-phénoxybenzyle

Stade A : (1R, cis) 2,2-diméthyl 3-[2-(1,1-diméthylallyloxycarbonyl) éthynyl] cyclopropane carboxylate de (S) α-cyano 3-phénoxybenzyle

On opère comme indiqué à l'exemple 15 à partir de 2 g de (1R, cis) 2,2-diméthyl 3-[carboxyéthynyl] cyclopropane carboxylate de (S) α-cyano 3-phénoxybenzyle et de 3 $cm^3$ de 2-méthyl 3-buten 2-ol avec 150 mg de diméthylaminopyridine. On refroidit à 0 °C pour ajouter 1,06 g de dicyclohexylcarbodiimide dans 3 $cm^3$ de chlorure de méthylène et agite 16 heures à température ambiante. On isole 700 mg de produit attendu après élution par le mélange cyclohexane-acétate d'éthyle (9-1).

Stade B : (1R, cis) 2,2-diméthyl 3-[(Z) 2-(1,1-diméthylallyloxy carbonyl) éthényl] cyclopropane carboxylate de (S) α-cyano 3-phénoxybenzyle

On opère comme indiqué à l'exemple 15 à partir de 450 mg de produit obtenu précédemment et obtient 370 mg de produit attendu.

$(\alpha)_D = 40°$ (c = 0,5 % benzène)
RMN ($CDCl_3$) ppm
1,27 : protons des méthyles en 2 du cyclopropane
3,25 à 3,6 : proton en 3 du cyclopropane
1,9-2,04 : proton en 1 du cyclopropane
6,32-6,68 : proton en 1 de la chaîne allylique en 3 du cyclopropane
5,82-6,02 : proton en 2 de la chaîne allylique en 3 du cyclopropane
1,57 : proton des méthyles en 1 de l'ester allylique
6 à 6,5 : proton en 2 de l'ester allylique
5,03 à 5,37 : proton en 3 de l'ester allylique
6,4 : proton porté par le même carbone que CN

Exemple 22 : (1R, cis) 2,2-diméthyl 3-[(Z) 2-(cyclopropyloxycarbonyl) éthényl] cyclopropane carboxylate de (S) α-cyano 3-phénoxybenzyle

On mélange 5,5 g de (1R, cis) 2,2-diméthyl 3-[(Z) 2-carboxyéthényl] cyclopropane carboxylate de (S) α-cyano 3-phénoxybenzyle, 26 $cm^3$ de chlorure de méthylène puis 0,9 g de cyclopropanol. On refroidit à 0 °C, ajoute 1 g de diméthylaminopyridine et 3 g de dicyclohexylcarbodiimide et agite pendant 1 heure 30 minutes à 20 °C. Après filtration, on concentre à sec le filtrat sous pression réduite, chromatographie le résidu sur silice en éluant avec un mélange cyclohexane-acétate d'éthyle (85-15). On obtient 3,6 g de produit attendu. F = 62 °C.

RMN ($CDCl_3$) ppm
1,27-1,3 : protons des méthyles en 2 du cyclopropane
3,25 à 3,4 : proton en 3 du cyclopropane
1,94-2,08 : proton en 1 du cyclopropane
6,4 à 6,5 : proton en 1 de la chaîne allylique
5,8-6,0 : proton en 2 de la chaîne allylique
6,4 : proton porté par le même carbone que CN
7 à 7,7 : protons aromatiques
0,7-0,77 : protons du cyclopropane ester

Exemple 23 : (1R, cis) 2,2-diméthyl 3-[(Z) 2-allyloxycarbonyl) éthényl] cyclopropane carboxylate de (S) α-cyano 3-phénoxybenzyle

On opère comme indiqué à l'exemple 7 à partir de 2,75 g de (1R, cis) 2,2-diméthyl 3-[(Z) 2-carboxyéthényl] cyclopropane carboxylate de (S) α-cyano 3-phénoxybenzyle et 0,8 $cm^3$ d'alcool allylique. Pour la chromatographie, l'éluant est un mélange n-hexane-éther isopropylique (8-2). On obtient 1,25 g de produit attendu.

$(\alpha)_D = +45° \pm 3$ (c = 0,35 % benzène)
RMN ($CDCl_3$) ppm
1,25-1,27 : protons des méthyles en 2 du cyclopropane
3,22 à 3,52 : proton en 3 du cyclopropane
1,93-2,07 : proton en 1 du cyclopropane
6,4 à 6,75 : proton en 1 de la chaîne allylique
5,87-6,06 : proton en 2 de la chaîne allylique
4,6-4,7 : protons en 1 de l'allyle ester
5,7 à 6,3 : proton en 2 de l'allyle ester
5,1 à 5,45 : proton en 3 de l'allyle ester
6,3 : proton porté par le même carbone que CN
6,9 à 7,6 : protons aromatiques

Exemple 24 : (1R, cis) 2,2-diméthyl 3-[(Z) 2-propargyloxycarbonyl) éthényl] cyclopropane carboxylate de (S) α-cyano 3-phénoxybenzyle.

Stade A : (1R, cis) 2,2-diméthyl 3-[(Z) 2-propargyloxycarbonyl) éthényl] cyclopropane carboxylate de terbutyle

On opère comme à l'exemple 13 à partir de 3,6 g de (1R, cis) 2,2-diméthyl 3-[(Z) 2-carboxyéthényl] cyclopropane carboxylate de terbutyle et 1 $cm^3$ d'alcool propargylique. On agite pendant 16 heures à 20 °C. On filtre l'insoluble, dilue le filtrat par du chlorure de méthylène, lave à l'acide chlorhydrique 0,1 N puis à l'eau jusqu'à neutralité, sèche et concentre à sec sous pression réduite. On reprend le résidu par un mélange cyclohexaneacétate d'éthyle (9-1) et agite 1 heure 30 minutes à 20 °C. On élimine l'insoluble, concentre à sec le filtrat et chromatographie le résidu sur silice en éluant par un mélange cyclohexane-acétate d'éthyle (9-1). On obtient 2,1 g de produit attendu.

Spectre IR ($CHCl_3$)
$—C{\equiv}C— \left\{ \begin{array}{l} 3\,300\ cm^{-1} \\ 2\,225\ cm^{-1} \end{array} \right.$
—C=O ester et conj 1 712 $cm^{-1}$
—C=C conj 1 629 $cm^{-1}$

Stade B : Acide (1R, cis) 2,2-diméthyl 3-[(Z) 2-propargyloxycarbonyl) éthényl] cyclopropane carboxylique

Pendant 10 minutes, on chauffe au reflux 2,0 g du produit obtenu ci-dessus en solution dans 20 $cm^3$ de toluène anhydre avec 0,2 g d'acide paratoluènesulfonique, refroidit et agite pendant 30 minutes à 0 °C. On filtre l'insoluble et concentre à sec le filtrat pour obtenir 1,59 g de produit attendu utilisé tel quel pour le stade suivant.

Stade C : (1R, cis) 2,2-diméthyl 3-[(Z) 2-propargyloxycarbonyl) éthényl] cyclopropane carboxylate de (S) α-cyano 3-phénoxybenzyle

On opère comme à l'exemple 13 à partir de 1,59 g de produit obtenu précédemment et 1,8 g d'alcool (S) α-cyano 3-phénoxybenzylique. Après 2 heures de réaction, on filtre, concentre à sec le filtrat sous pression réduite et chromatographie le résidu sur silice en éluant par un mélange cyclohexane-acétate d'éthyle (9-1). On obtient 2,86 g de produit attendu.

$(\alpha)_D = + 44 \pm 1,5°$ (c = 1 % $CHCl_3$)

RMN ($CDCl_3$) ppm

1,23-1,27 : protons des méthyles en 2 du cyclopropane
1,93-2,07 : proton en 1 du cyclopropane
3,18 à 3,5 : proton en 3 du cyclopropane
6,46-6,65 } proton en 1 de la chaîne allylique
6,62-6,82 }
5,87-6,05 : proton en 2 de la chaîne allylique
4,72-4,73 : protons en 1 du propargyle
2,47 (t) : proton en 3 du propargyle
6,3 : proton porté par le même carbone que CN
6,92 à 7,6 : protons aromatiques.

En plus des composés nommés précédemment dans la partie expérimentale, les composés suivants peuvent également être obtenus selon le procédé de l'invention :

— le (1R, cis) 2,2-diméthyl 3-[(Z) 2-(n-undécyloxycarbonyl) éthényl] cyclopropane carboxylate de (S) α-cyano 3-phénoxybenzyle,

— le (1R, cis) 2,2-diméthyl 3-[(Z) 2-(1-méthylcyclohexyl) éthényl] cyclopropane carboxylate de (S) α-cyano 3-phénoxybenzyle,

— le (1R, cis) 2,2-diméthyl 3-[(Z) 2-(n-hexyloxycarbonyl) éthényl] cyclopropane carboxylate de (S) α-cyano 3-phénoxybenzyle,

— le (1R, cis) 2,2-diméthyl 3-[(Z) 2-(cyclobutylméthyloxycarbonyl) éthényl] cyclopropane carboxylate de (S) α-cyano 3-phénoxybenzyle.

## Exemples de compositions

### Exemple A : Préparation d'un concentré soluble

On effectue un mélange homogène de :

| | |
|---|---|
| — Produit de l'exemple 1 | 0,25 g |
| — Butoxyde de pipéronyle | 1 g |
| — Tween 80 | 0,25 g |
| — Topanol A | 0,1 g |
| — Eau | 98,4 g |

### Exemple B : Préparation d'un concentré émulsifiable

On mélange intimement :

| | |
|---|---|
| — Produit de l'exemple 1 | 0,015 g |
| — Butoxyde de pipéronyle | 0,5 g |
| — Topanol A | 0,1 g |
| — Xylène | 95,885 g |
| — Tween 80 | 3,5 g |

### Exemple C : Préparation d'un concentré émulsifiable

On effectue un mélange homogène de :

| | |
|---|---|
| — Produit de l'exemple 1 | 1,5 g |
| — Tween 80 | 20 g |
| — Topanol A | 0,1 g |
| — Xylène | 78,4 g |

### Exemple D : Préparation d'un concentré soluble

On effectue un mélange homogène de :

| | |
|---|---|
| — Produit de l'exemple 6 | 0,25 g |

| | |
|---|---|
| — Butoxyde de pipéronyle | 1 g |
| — Tween 80 | 0,25 g |
| — Topanol A | 0,1 g |
| — Eau | 98,4 g |

Exemple E : Préparation d'un concentré émulsiable

On mélange intimement :

| | |
|---|---|
| — Produit de l'exemple 6 | 0,015 g |
| — Butoxyde de pipéronyle | 0,5 g |
| — Topanol A | 0,1 g |
| — Xylène | 95,885 g |
| — Tween 80 | 3,5 g |

Exemple F : Préparation d'un concentré émulsiable

On effectue un mélange homogène de :

| | |
|---|---|
| — Produit de l'exemple 6 | 1,5 g |
| — Tween 80 | 20 g |
| — Topanol A | 0,1 g |
| — Xylène | 78,4 g |

Exemple G : Préparation d'une composition fumigène

| | |
|---|---|
| — Produit de l'exemple 1 | 0,25 g |
| — Poudre de tabu | 25 g |
| — Poudre de feuille de cèdre | 40 g |
| — Poudre de bois de pin | 33,75 g |
| — Vert brillant | 0,5 g |
| — p-nitrophénol | 0,5 g |

Exemple H : Préparation d'une composition fumigène

| | |
|---|---|
| — Produit de l'exemple 6 | 0,25 g |
| — Poudre de tabu | 25 g |
| — Poudre de feuille de cèdre | 40 g |
| — Poudre de bois de pin | 33,75 g |
| — Vert brillant | 0,5 g |
| — p-nitrophénol | 0,5 g |

Exemple I : Composition antifongique

On a préparé une poudre mouillable dont la composition est la suivante :

| | |
|---|---|
| — Acide (1R, cis) 2,2-diméthyl 3-[(Z) 2-méthoxycarbonyl) éthényl] cyclopropane-1-carboxylique | 20 g |
| — Ekapersol « S » (1) | 15 g |
| — Brecolane NVA (2) | 0,5 g |
| — Zéosil 39 (3) | 32,5 g |
| — Vercoryl (S) (4) | 25 g |

(1) produit de condensation du naphtalène sulfonate de sodium.
(2) alcoyl naphtalène sulfonate de sodium.
(3) silice hydratée synthétique obtenue par précipitation.
(4) Kaolin colloïdal.

Exemple J : Composition antifongique

On a préparé une solution contenant :

| | |
|---|---|
| — Acide (1R, cis) 2,2-diméthyl 3-[(Z) 2-méthoxycarbonyl)éthényl] cyclopropane-1-carboxylique | 25 g/l |
| — Emcol H 300 B | 80 g/l |
| — Xylène | 895 g/l |

Etude de l'activité des composés de l'invention

Exemple 25 : Etude de l'effet létal des composés des exemples 1, 3 et 5 sur mouches domestiques

Les insectes tests sont des mouches domestiques femelles âgées de 4 jours. On opère par application topique de 1 µl de solution acétonique sur le thorax dorsal des insectes à l'aide du micro manipulateur d'Arnold. On utilise 50 individus par traitement. On effectue le contrôle de mortalité 24 heures après traitement.

Le résultat obtenu exprimé en DL 50 ou dose (en nanogrammes) nécessaire pour tuer 50 % des insectes, est le suivant :

| Composé de l'exemple | DL50 (en ng par individu) |
|---|---|
| 1 | 7,128 |
| 3 | 3,494 |
| 5 | 2,781 |

Conclusion : sur le test utilisé, les produits présentent une bonne activité.

Exemple 26 : Etude de l'effet létal sur larves de Spodoptera littoralis

Les essais sont effectués par application topique d'une solution acétonique à l'aide du micro manipulateur d'Arnold sur le thorax dorsal des larves. On utilise 10 à 15 larves par dose de produit à tester. Les larves utilisées sont des larves du quatrième stade larvaire, c'est-à-dire âgées d'environ 10 jours lorsqu'elles sont élevées à 24 °C et 65 % d'humidité relative. Après traitement, les individus sont placés sur un milieu nutritif artificiel (milieu de Poitout).

On effectue le contrôle des mortalités 48 heures après traitement.

Le résultat expérimental obtenu est le suivant :

| Composé de l'exemple | DL50 (en ng par individu) |
|---|---|
| 1 | 3,602 |
| 3 | 3,260 |
| 5 | 7,920 |

Conclusion : sur le test utilisé, les produits présentent une bonne activité.

Exemple 27 : Etude de l'activité des produits des exemples 1, 3, 5 et 6 sur larves d'Epilachna Varivestris

Les essais sont effectués par application topique de manière analogue à celle utilisée pour les larves de Spodoptera. On utilise des larves de l'avant dernier stade larvaire et après traitement les larves sont alimentées par des plants de haricots. On effectue le contrôle de mortalité 72 heures après traitement.

Le résultat obtenu est le suivant :

| Composé de l'exemple | DL50 (en ng par individu) |
|---|---|
| 1 | 8,290 |
| 3 | 8,850 |
| 5 | 3,705 |
| 6 | 0,446 |

Conclusion : sur le test utilisé, les produits présentent une bonne activité, notamment le produit de l'exemple 6.

Exemple 28 : Etude de l'activité de choc sur mouche domestique

Les insectes tests sont des mouches domestiques femelles âgées de 4 jours. On opère par

pulvérisation directe en chambre de Kearns et March en utilisant comme solvant un mélange d'acétone (5 %) et d'Isopar L (solvant pétrolier) (quantité de solvant utilisée 2 ml en une seconde). On utilise 50 insectes par traitement. On effectue les contrôles toutes les minutes jusqu'à 10 minutes, puis à 15 minutes et l'on détermine le KT 50 par les méthodes habituelles.

Le résultat obtenu est le suivant :

| Composé de l'exemple | KT 50 (en mn Conc. 0,25g/1) |
|---|---|
| 1 | 1,226 |
| 3 | 3,340 |
| 5 | 4,390 |

Conclusion : sur le test utilisé, les produits et notamment le produit de l'exemple 1 présentent une activité de choc remarquable.

Exemple 29 : Activité sur Tetranychus Urticae du produit de l'exemple 6. Essai aldulticide :

On utilise des plants de haricots comportant 2 feuilles infestées de 25 femelles de Tetranychus Urticae par feuille et mis sous bonette aérée sous plafond lumineux en lumière constante. Les plants sont traités au pistolet Fisher (4 ml de solution toxique par plant d'un mélange à volume égal d'eau et d'acétone renfermant le produit à étudier). On laisse sécher pendant 12 heures puis on procède à l'infestation. Les contrôles de mortalité sont effectués 80 heures après.

La DL 50 du produit a été déterminée par un essai préliminaire à 3 doses suivi d'un essai complémentaire à 5 doses.

Avec ce produit une mortalité de 50 % des acariens a été obtenue à la dose de 0,586 g par hl.

Conclusion : Le produit présente une activité acaricide remarquable.

Exemple 30 : Etude de l'activité du composé de l'exemple 6 sur des acariens parasites des animaux

a) Etude de l'activité sur larve de Boophilus Microplus :

La substance à tester est dissoute dans un mélange constitué de diméthylformamide, d'émulsifiants et d'Arcopal de façon à obtenir un concentré émulsifiant à 10 %. On dilue ce concentré avec de l'eau pour obtenir des solutions de concentration souhaitée de 10, 5 et 1 ppm.

Au moyen d'une tour de pulvérisation, on pulvérise les différentes solutions ci-dessus sur des larves de tiques de bœufs des tropiques, de type Boophilus Microplus (souches Mexico sensible et DDT résistante et on détermine après 24 heures, par comptage des larves mortes et vivantes, le pourcentage de mortalité.

Les résultats sont les suivants :

| Dose de produit en ppm | % de mortalité (Mexico sensible | % de mortalité (DDT résistante |
|---|---|---|
| 10 | 100 | 100 |
| 5 | 100 | 100 |
| 1 | 100 | 100 |

Conclusion : Le produit de l'exemple 6 présente une activité remarquable.

b) Etude de l'activité sur tiques adultes (Rhipicephalus appendiculatus et Amblyomma hebraeum).

On utilise des concentrations de 100, 10 et 1 ppm. Les solutions sont pulvérisées comme ci-dessus. On utilise 10 tiques par concentration.

Les résultats sont les suivants :

| Dose de produit en ppm | % de mortalité (Rhip. app.) | % de mortalité (Ambl. heb.) |
|---|---|---|
| 100 | 100 | 100 |
| 10 | 100 | 100 |
| 1 | 100 | 70 |

Conclusion : Le produit de l'exemple 6 présente une activité remarquable.

c) Etude de l'activité du produit sur l'inhibition de la reproduction de tiques Boophilus Microplus (souche Mexico sensible)

On plonge des femelles de Boophilus Microplus prêtes à pondre pendant 5 minutes dans les solutions préparées ci-dessus, puis on les porte dans une enceinte chauffée pour la ponte.

On détermine a) le pourcentage de tiques n'ayant pas pondu, b) la quantité d'œufs pondus en fonction d'un témoin, c) le pourcentage de larves ayant éclos, après 2 semaines.

On calcule, en fonction des chiffres obtenus, le pourcentage d'inhibition de la reproduction, 100 % signifie que l'inhibition est totale et 0 % que la reproduction est identique à celle obtenue avec les témoins. On obtient :

| Doses en ppm | % inhibition |
|---|---|
| 100 | 100 |
| 50 | 100 |
| 25 | 100 |
| 12,50 | 100 |
| 6,25 | 100 |

Conclusion : Le produit présente une activité remarquable.

Exemple 31 : Etude de l'activité antifongique de l'acide (1R, cis) 2,2-diméthyl 3-[(Z) 2-méthoxycarbonyl) éthényl] cyclopropane carboxylique

Essai sur colles

On utilise une solution à 3 % carboxyméthylamidon dans l'eau. On incorpore le composé à tester dans 1 $cm^3$ de solution acétonique, ajoute 5 $cm^3$ d'un inoculum constitué par un mélange de spores de Fusarium Roseum, Penicillium Roqueforti et Aspergilus Niger. On contrôle le développement du champignon par rapport à un témoin non traité (Note de 3 à 0) 48 heures et 8 jours après traitement et contamination.

Les résultats sont les suivants :

| % matière active | après 48 heures | après 8 jours |
|---|---|---|
| 0,05 | 0 | 0 |
| 0,01 | 3 | 3 |
| Témoin | 3 | 3 |

Conclusion : Le produit testé présente une bonne activité antifongique.

Essai sur huiles

On utilise une solution à 3 % d'un fluide synthétique. La contamination est effectuée par 5 $cm^3$ d'un inoculum constitué par un mélange de Geotrichum, Trichosporon et Cephalosporium. Le contrôle est effectué quantitativement.

Les résultats sont les suivants :

| % matière active | après 48 heures | après 8 jours |
|---|---|---|
| 1 | 0 | 0 |
| 0,5 | 0 | 0 |
| Témoin | 49 x $10^3$ Geo.<br>+ 80 x $10^3$ Tri.<br>+ 11 x $10^3$ Cep. | 33 x $10^3$ Geo.<br>+ 15 x $10^3$ Tri. |

Conclusion : Le produit testé présente une bonne activité antifongique.

Exemple 32 : Etude de l'activité bactéricide de l'acide (1R, cis) 2,2-diméthyl 3-[(Z) 2-(méthoxycarbonyl) éthényl] cyclopropane carboxylique

Essais sur colle

On utilise une solution à 3 % de carboxyméthylamidon dans l'eau. On incorpore le composé à tester dans 1 $cm^3$ de solution acétonique, ajoute 5 $cm^3$ d'un inoculum constitué par un mélange de spores d'Aerobacter-Aerogenes, de Pseudomonas Aeruginosa, d'Escherichia Coli, de Serratia Marcescens et de Bacillus Subtilis. On porte ce mélange à l'étuve à 37 °C pendant 48 heures puis à 20 °C pendant 6 jours.

On effectue le contrôle de la population bactérienne 48 heures et 8 jours après traitement et contamination par la méthode des dilutions dans le sérum et incorporation dans le bouillon gélosé.

En utilisant ce protocole opératoire, on a obtenu les résultats suivants :

| POPULATION (Colonies/ml après traitement et infestation 48 heures après | | | 8 jours après | | |
|---|---|---|---|---|---|
| 0,05 % | 0,025 % | non traité | 0,05 % | 0,025 % | non traité |
| $52 \times 10^2$ | $10 \times 10^4$ | $112 \times 10^7$ | $68 \times 10^4$ | $16 \times 10^6$ | $16 \times 10^7$ |

Conclusions : Le produit testé présente une bonne activité bactéricide.

**Revendications** (pour les Etats contractants : BE, CH, DE, FR, GB, IT, LI, LU, NL, SE)

1. Les composés répondant à la formule (1) :

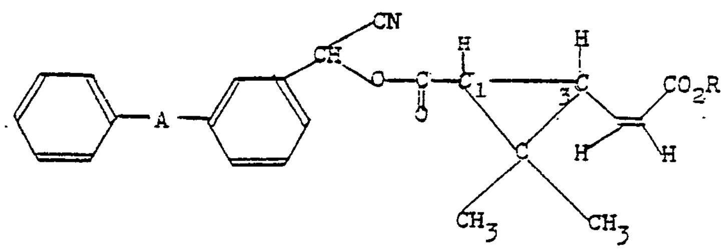

dans laquelle A représente un atome d'oxygène, un groupement méthylène ou un groupement carbonyle, et R représente un radical alcoyle linéaire, ramifié ou cyclique, saturé ou insaturé, renfermant de 1 à 8 atomes de carbone, dans laquelle la double liaison a la géométrie Z et la copule cyclopropanique est de structure 1R, cis sous toutes leurs formes stéréo isomères possibles ainsi que les mélanges de ces stéréo isomères.

2. Les composés de formule I, tels que définis à la revendication 1, pour lesquels A représente un atome d'oxygène.

3. Les composés de formule I, tels que définis à l'une quelconque des revendications 1 et 2, pour lesquels la copule alcoolique de l'ester en position 1 du cyclopropane est le reste de l'alcool (S) α-cyano 3-phénoxy benzylique.

4. Les composés de formule I, tels que définis à l'une quelconque des revendications 1 à 3, pour lesquels R représente un radical méthyle, éthyle, propyle, isopropyle, butyle ou terbutyle.

5. Les composés de formule (I), tels que définis à l'une quelconque des revendications 1 à 3, pour lesquels R représente un radical cyclopropyle ou cyclopropylméthyle.

6. Le composé de formule I, tel que défini à la revendication 1, dont le nom suit :

— le (1R, cis) 2,2-diméthyl 3-[(Z) 2(méthoxy carbonyl) éthényl] cyclopropane carboxylate de (S) α-cyano 3-phénoxy benzyle.

7. L'un quelconque des composés de formule I, tels que définis à la revendication 1, dont les noms suivent :

— le (1R, cis) 2,2-diméthyl 3-[(Z) 2-(éthoxy carbonyl) éthényl] cyclopropane carboxylate de (S) α-cyano 3-phénoxy benzyle,

— le (1R, cis) 2,2-diméthyl 3-[(Z) 2-isopropyloxy carbonyl) éthényl] cyclopropane carboxylate de (S) α-cyano 3-phénoxy benzyle,

— le (1R, cis) 2,2-diméthyl 3-[(Z) 2-(cyclopropylméthoxycarbonyl) éthényl] cyclopropane carboxylate de (S) α-cyano 3-phénoxybenzyle,

— le (1R, cis) 2,2-diméthyl 3-[(Z) 2-(cyclopropyloxycarbonyl) éthényl] cyclopropane carboxylate de (S) α-cyano 3-phénoxybenzyle,

— le (1R, cis) 2,2-diméthyl 3-[(Z) 2-terbutoxycarbonyl) éthényl] cyclopropane carboxylate de (S) α-cyano 3-phénoxybenzyle.

8. Application des composés de formule I, tels que définis à l'une quelconque des revendications 1 à 7, à la lutte contre les parasites des végétaux et les parasites domestiques.

9. Les compositions destinées à la lutte contre les parasites des végétaux, les parasites domestiques et les parasites des animaux à sang chaud, caractérisées en ce qu'elles renferment au moins l'un des produits définis à l'une quelconque des revendications 1 à 7.

10. Les compositions insecticides renfermant comme principe actif l'un quelconque des produits défini à une quelconque des revendications 1 à 5.

11. Les compositions insecticides renfermant comme principe actif le produit défini à la revendication 6.

12. Les compositions insecticides renfermant comme principe actif au moins l'un des produits définis à la revendication 7.

13. Les compositions destinées à la lutte contre les acariens parasites des végétaux, caractérisées en ce qu'elles renferment au moins l'un des produits définis à l'une quelconque des revendications 1 à 7.

14. Les compositions destinées à la lutte contre les acariens parasites des végétaux, caractérisées en ce qu'elles renferment comme principe actif, le (1R, cis) 2,2-diméthyl 3-[(Z) 2-(terbutoxycarbonyl) éthényl] cyclopropane carboxylate de (S) α-cyano 3-phénoxybenzyle.

15. Les compositions destinées à l'alimentation animale, renfermant comme principe actif au moins l'un des produits définis à l'une quelconque des revendications 1 à 7, associé à un aliment destiné à l'alimentation animale.

16. A titre de médicament, les composés définis à l'une quelconque des revendications 1 à 7.

17. Associations douées d'activité insecticide, acaricide ou nématicide, caractérisées en ce qu'elles contiennent comme matière active, d'une part un au moins des composés de formule générale (I), et d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxybenzylique et d'alcools α-cyano 3-phénoxybenzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxybenzylique et d'alcools α-cyano 3-phénoxybenzyliques des acides 2,2-diméthyl 3-(2,2-dichlorovinyl) cyclopropane-1-carboxyliques, par les esters d'alcools α-cyano 3-phénoxybenzyliques d'acides 2,2-diméthyl 3-(2,2-dibromovinyl) cyclopropane-1-carboxyliques, par les esters d'alcool 3-phénoxybenzylique des acides 2-parachlorophényl 2-isopropyl acétiques, par les esters d'alléthrolones, d'alcool 3,4,5,6-tétrahydrophalimidométhylique, d'alcool 5-benzyl 3-furyl méthylique, d'alcool 3-phénoxybenzylique et d'alcools α-cyano 3-phénoxybenzyliques des acides 2,2-diméthyl 3-(1,2,2,2-tétrahalo) cyclopropane-1-carboxyliques, dans lesquels « halo » représente un atome de fluor, de chlore ou de brome, étant entendu que les composés I peuvent exister sous toutes leurs formes stéréoisomères possibles de même que les copules acides et alcools des esters pyréthrinoïdes ci-dessus.

18. Procédé de préparation des composés de formule I, tels que définis à l'une quelconque des revendications 1 à 7, caractérisé en ce que l'on soumet un acide de formule II :

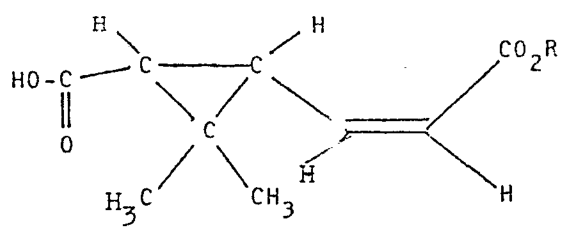

(II)

dans laquelle la double liaison a la géométrie Z et la copule cyclopropanique est de structure 1R, cis et R conserve la même signification que dans la revendication 1, ou un dérivé fonctionnel de cet acide, avec un alcool de formule :

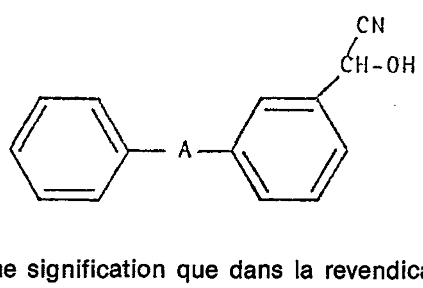

(III)

dans laquelle A conserve la même signification que dans la revendication 1.

19. Procédé de préparation selon la revendication 18, caractérisé en ce que le composé de formule II utilisé est préparé en faisant réagir un composé de formule IV :

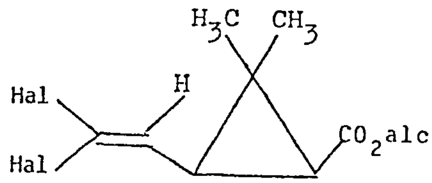

(IV)

dans laquelle la copule cyclopropanique est de structure 1R, cis et Hal représente un atome d'halogène et alc représente un radical alcoyle renfermant de 1 à 8 atomes de carbone, dans un premier temps avec un agent alcalin capable d'arracher les atomes d'halogène et dans un deuxième temps :

— soit avec un agent capable d'introduire le groupement carboxylique pour obtenir le composé de formule V :

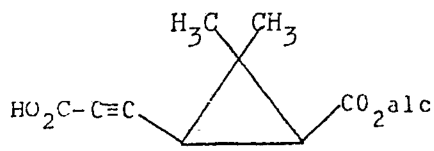

(V)

que l'on soumet à l'action d'un agent d'estérification pour obtenir le composé de formule VI :

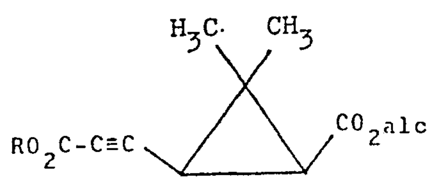

(VI)

dans lequel R conserve la même signification que précédemment

— soit avec un chloroformiate d'alcoyle de formule :

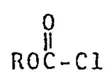

(V')

dans laquelle R conserve la signification précédente, pour obtenir directement le composé de formule VI :

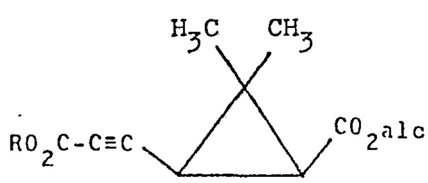

(VI)

puis en soumettant le composé de formule VI obtenu, à l'action d'un agent d'hydrogénation ménagée pour obtenir le composé de formule :

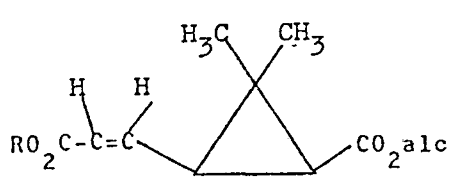

(VII)

dans laquelle la double liaison a la géométrie Z, que l'on soumet à l'action d'un agent d'hydrolyse acide capable de cliver sélectivement la fonction ester en 1 du cyclopropane pour obtenir le composé de formule II correspondant :

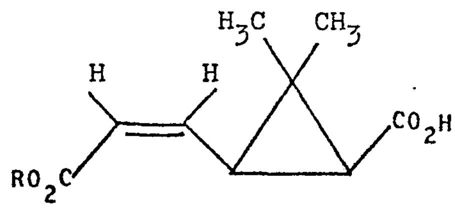

(II)

20. Procédé selon la revendication 19, caractérisé en ce que l'on soumet un composé de formule V :

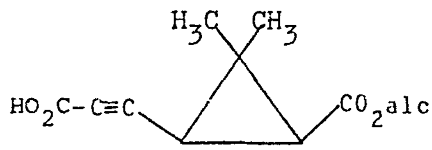

(V)

dans laquelle la copule cyclopropanique est de structure 1R, cis et alc conserve sa signification précédente, d'abord à l'action d'un agent d'hydrogénation ménagée, pour obtenir un composé de formule :

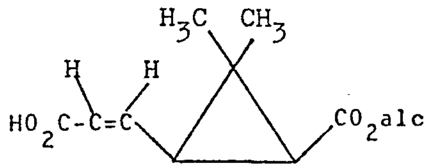

(VIII)

dans laquelle la double liaison a la géométrie Z, que l'on soumet à l'action d'un agent d'estérification, pour obtenir le composé de formule VII correspondant :

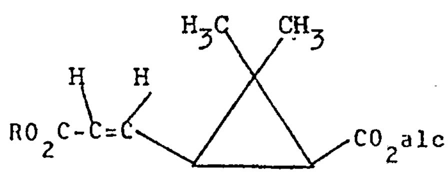

(VII)

puis poursuit la synthèse comme décrit à la revendication 20.

21. Procédé selon les revendications 18 et 19, caractérisé en ce que l'on soumet un composé de formule VI :

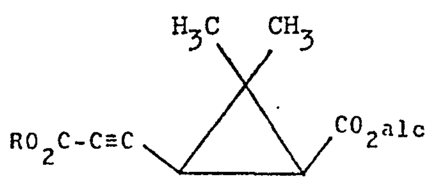

(VI)

dans laquelle la copule cyclopropanique est de structure 1R, cis et R et alc sont définis comme précédemment, à l'action d'un agent d'hydrolyse acide capable de cliver sélectivement la fonction ester en 1 du cyclopropane, pour obtenir le composé de formule (IX) :

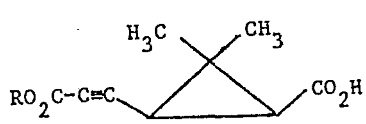

(IX)

dans laquelle R est défini comme précédemment, que

— soit l'on soumet, le cas échéant, sous forme d'un dérivé fonctionnel, à l'action d'un alcool de formule (III) :

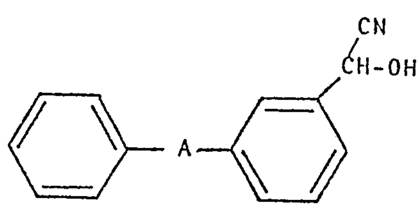

(III)

dans laquelle A conserve la même signification que précédemment, pour obtenir un composé de formule (X) :

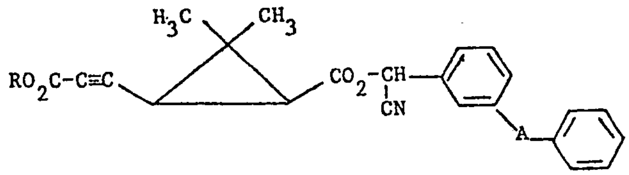

(X)

dans laquelle R et A conservent la même signification que précédemment, que l'on soumet à l'action d'un agent d'hydrogénation ménagée, pour obtenir le composé de formule (I),

— soit l'on soumet d'abord à l'action d'un agent d'hydrogénation ménagée, pour obtenir le composé de formule (II) :

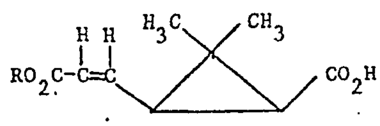

(II)

dans laquelle R est défini comme précédemment et la double liaison a la géométrie Z, puis le cas échéant sous forme d'un dérivé fonctionnel, à l'action d'un alcool (III), pour obtenir le composé de formule I.

22. Procédé de préparation des composés de formule (I) tels que définis à l'une quelconque des revendications 1 à 7, caractérisé en ce que l'on soumet un composé de formule (XI) :

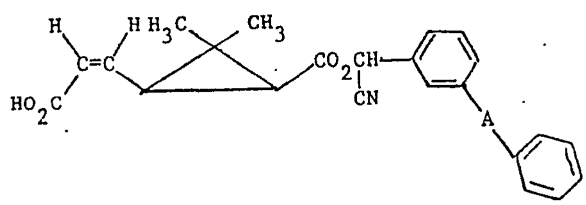

(XI)

dans laquelle la double liaison a la géométrie Z, et la copule cyclopropanique est de structure 1R, cis et A est défini comme à la revendication 1, à l'action d'un agent d'estérification, pour obtenir le composé de formule (I) correspondant :

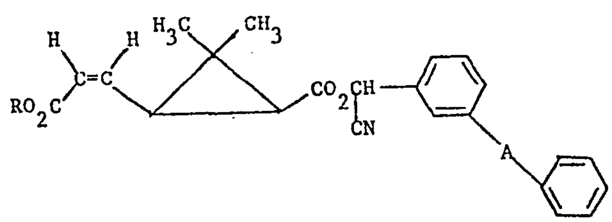

(I)

dans laquelle R est défini comme à la revendication 1.

23. Procédé selon la revendication 22, caractérisé en ce que le composé de formule XI est préparé en soumettant un acide de formule (V) :

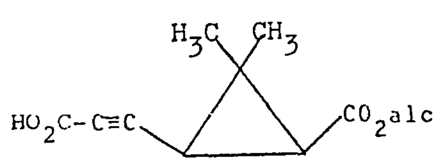

(V)

dans laquelle la copule cyclopropanique est de structure 1R, cis et alc représente un radical alcoyle renfermant de 1 à 8 atomes de carbone à l'action du 2,2,2-trichloroéthanol pour obtenir le composé de formule (XII) :

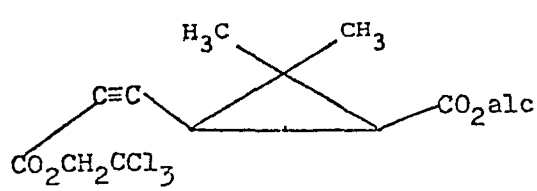

(XII)

que l'on soumet à l'action d'un agent d'hydrolyse acide pour obtenir le composé de formule (XIII) :

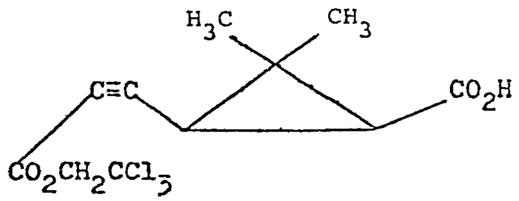

(XIII)

que l'on soumet à l'action d'un alcool de formule (III) :

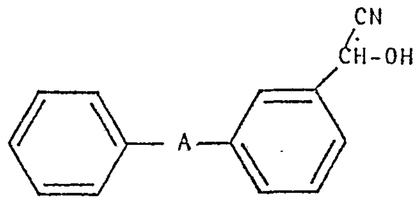

(III)

dans laquelle A conserve la même signification qu'à la revendication 1, pour obtenir le composé de formule (XIV) :

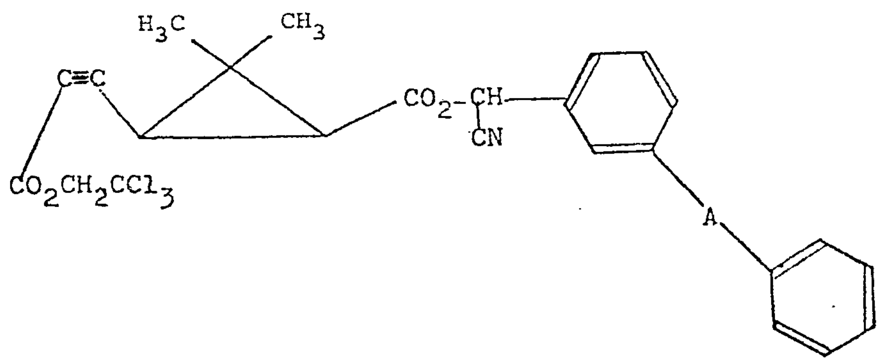

(XIV)

que l'on soumet à l'action d'un agent de clivage de la fonction ester porté par le carbone acétylénique pour obtenir le composé de formule (XV) :

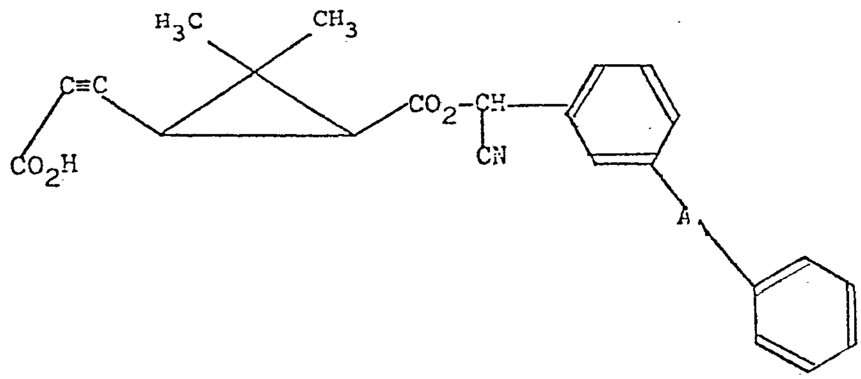

(XV)

que l'on soumet à l'action d'un agent d'hydrogénation ménagée, pour obtenir le composé de formule (XI).

24. Procédé selon la revendication 23, caractérisé en ce que l'on soumet un composé de formule (XV) :

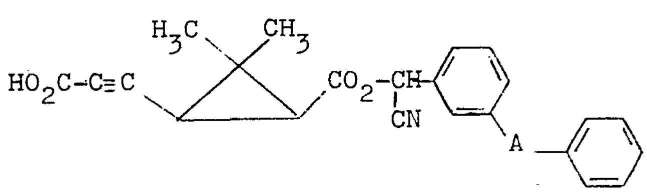

(XV)

dans laquelle la copule cyclopropanique est de structure 1R, cis et A est défini comme à la revendication 1, à l'action d'un agent d'estérification, pour obtenir le composé de formule (X)

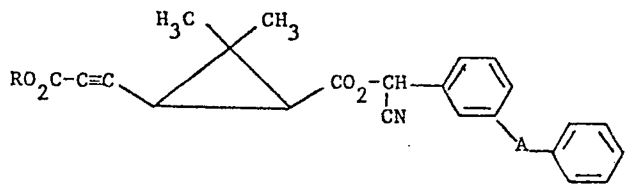

(X)

dans laquelle R et A sont définis comme à la revendication 1, que l'on soumet à l'action d'un agent d'hydrogénation ménagée, pour obtenir le composé de formule I.

25. A titre de produits industriels nouveaux et notamment à titre de produits intermédiaires nécessaires à la mise en œuvre du procédé de l'invention tel que défini à l'une quelconque des revendications 18 à 21, les composés de formules II, V, VI, VII, VIII, IX et X, tels que définis dans les revendications 18 à 21.

26. A titre de produits industriels nouveaux et notamment à titre de produits intermédiaires nécessaires à la mise en œuvre du procédé de l'invention tels que défini à l'une quelconque des revendications 22 à 24, les composés de formules XI, XII, XIII, XIV et XV, tels que définis dans les revendications 22 à 24.

**Revendications** (pour l'Etat contractant AT)

1. Procédé pour préparer les composés répondant à la formule (I) :

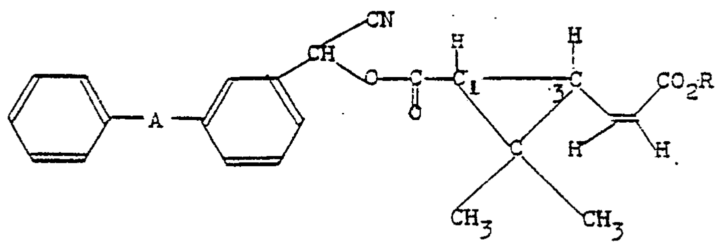

dans laquelle A représente un atome d'oxygène, un groupement méthylène ou un groupement carbonyle, et R représente un radical alcoyle linéaire, ramifié ou cyclique, saturé ou insaturé, renfermant de 1 à 8 atomes de carbone, dans laquelle la double liaison a la géométrie Z et la copule cyclopropanique est de structure 1R, cis sous toutes leurs formes stéréo isomères possibles, ainsi que les mélanges de ces stéréo isomères, caractérisé en ce que l'on soumet un acide de formule (II) :

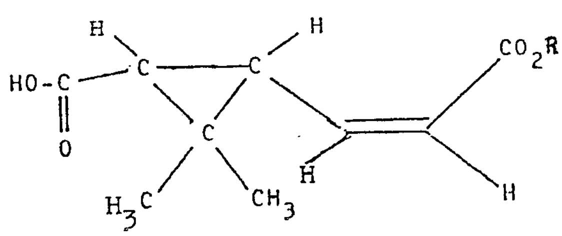

(II)

dans laquelle la double liaison a la géométrie Z et la copule cyclopropanique est de structure 1R, cis et R conserve la même signification que précédemment, ou un dérivé fonctionnel de cet acide, avec un alcool de formule :

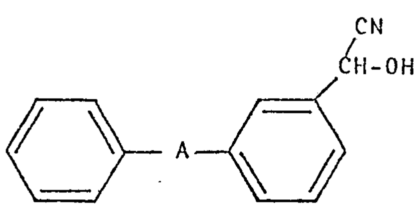

(III)

dans laquelle A conserve la même signification que précédemment.

2. Procédé de préparation selon la revendication 1, caractérisé en ce que le composé de formule II utilisé est préparé en faisant réagir un composé de formule IV :

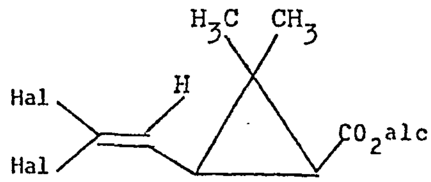

(IV)

dans laquelle la copule cyclopropanique est de structure 1R, cis, Hal représente un atome d'halogène et alc représente un radical alcoyle renfermant de 1 à 8 atomes de carbone, dans un premier temps avec un agent alcalin capable d'arracher les atomes d'halogène et dans un deuxième temps ;

— soit avec un agent capable d'introduire le groupement carboxylique pour obtenir le composé de formule V :

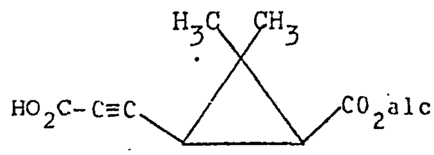

(V)

que l'on soumet à l'action d'un agent d'estérification pour obtenir le composé de formule VI :

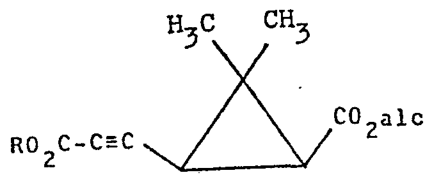

(VI)

dans lequel R conserve la même signification que précédemment

— soit avec un chloroformiate d'alcoyle de formule :

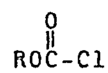

(V')

dans laquelle R conserve la signification précédente, pour obtenir directement le composé de formule VI :

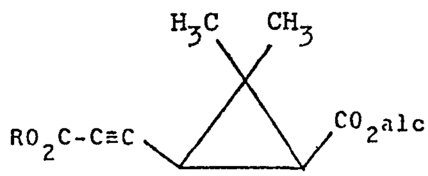

(VI)

puis en soumettant le composé de formule VI obtenu, à l'action d'un agent d'hydrogénation ménagée pour obtenir le composé de formule :

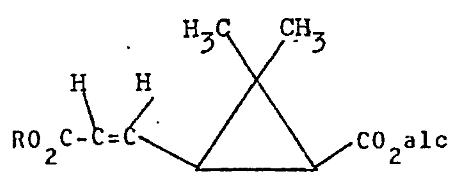

(VII)

dans laquelle la double liaison a la géométrie Z, que l'on soumet à l'action d'un agent d'hydrolyse acide capable de cliver sélectivement la fonction ester en 1 du cyclopropane pour obtenir le composé de formule II correspondant :

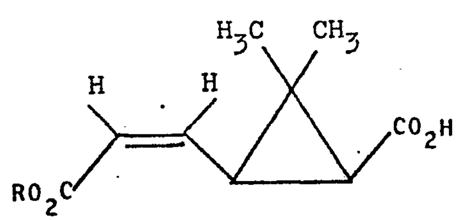

(II)

3. Procédé selon la revendication 2, caractérisé en ce que l'on soumet un composé de formule V :

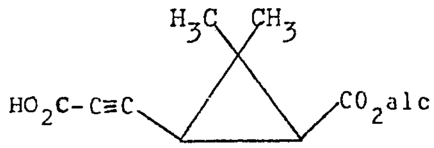

(V)

dans laquelle la copule cyclopropanique est de structure 1R, cis et alc conserve sa signification précédente, d'abord à l'action d'un agent d'hydrogénation ménagée, pour obtenir un composé de formule :

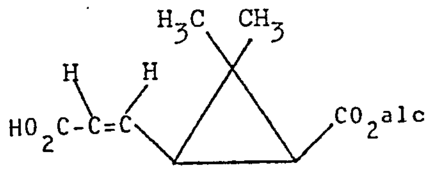

(VIII)

dans laquelle la double liaison a la géométrie Z, que l'on soumet à l'action d'un agent d'estérification, pour obtenir le composé de formule VII correspondant :

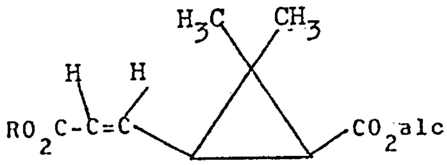

(VII)

puis poursuit la synthèse comme décrit à la revendication 2.

4. Procédé selon les revendications 1 et 2, caractérisé en ce que l'on soumet un composé de formule VI :

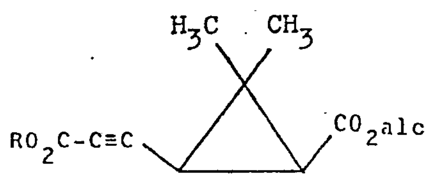

(VI)

dans laquelle la copule cyclopropanique est de structure 1R, cis et R et alc sont définis comme précédemment, à l'action d'un agent d'hydrolyse acide capable de cliver sélectivement la fonction ester en 1 du cyclopropane, pour obtenir le composé de formule (IX) :

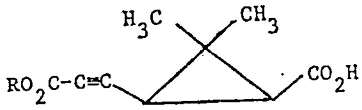

(IX)

dans laquelle R est défini comme précédemment, que

— soit l'on soumet, le cas échéant, sous forme d'un dérivé fonctionnel, à l'action d'un alcool de formule (III) :

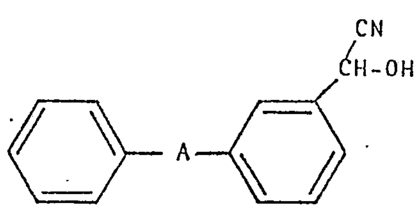

(III)

dans laquelle A conserve la même signification que précédemment, pour obtenir un composé de formule (X) :

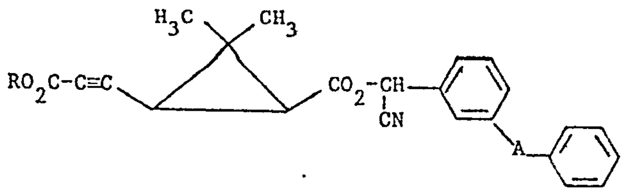

(X)

dans laquelle R et A conservent la même signification que précédemment, que l'on soumet à l'action d'un agent d'hydrogénation ménagée, pour obtenir le composé de formule (I),

— soit l'on soumet d'abord à l'action d'un agent d'hydrogénation ménagée, pour obtenir le composé de formule (II) :

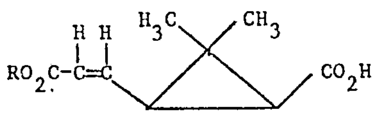

(II)

dans laquelle R est défini comme précédemment et la double liaison a la géométrie Z, puis, le cas échéant sous forme d'un dérivé fonctionnel, à l'action d'un alcool (III), pour obtenir le composé de formule I.

5. Procédé de préparation des composés de formule (I) tels que définis à la revendication 1, caractérisé en ce que l'on soumet un composé de formule (XI) :

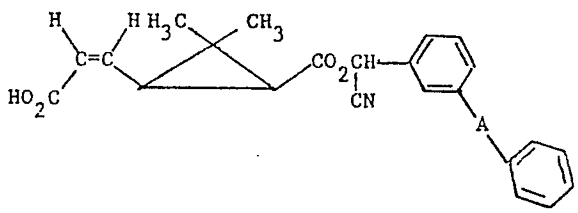

(XI)

dans laquelle la double liaison a la géométrie Z, et la copule cyclopropanique est de structure 1R, cis et A est défini comme à la revendication 1, à l'action d'un agent d'estérification, pour obtenir le composé de formule (I) correspondant :

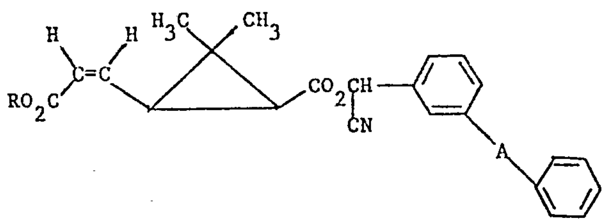

(I)

dans laquelle R est défini comme à la revendication 1.

6. Procédé selon la revendication 5, caractérisé en ce que le composé de formule XI est préparé en soumettant un acide de formule (V) :

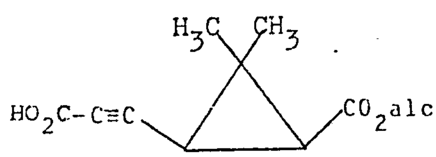

(V)

dans laquelle la copule cyclopropanique est de structure 1R, cis et alc représente un radical alcoyle renfermant de 1 à 8 atomes de carbone à l'action du 2,2,2-trichloroéthanol pour obtenir le composé de formule (XII) :

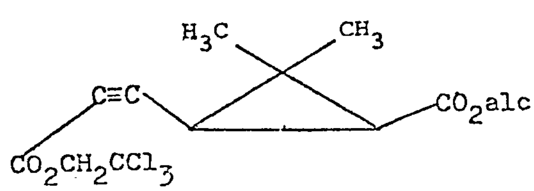

(XII)

que l'on soumet à l'action d'un agent d'hydrolyse acide pour obtenir le composé de formule (XIII) :

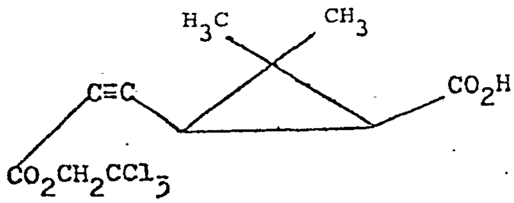

(XIII)

que l'on soumet à l'action d'un alcool de formule (III) :

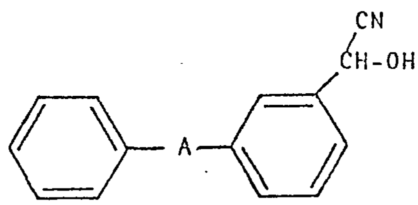

(III)

dans laquelle A conserve la même signification qu'à la revendication 1, pour obtenir le composé de formule (XIV) :

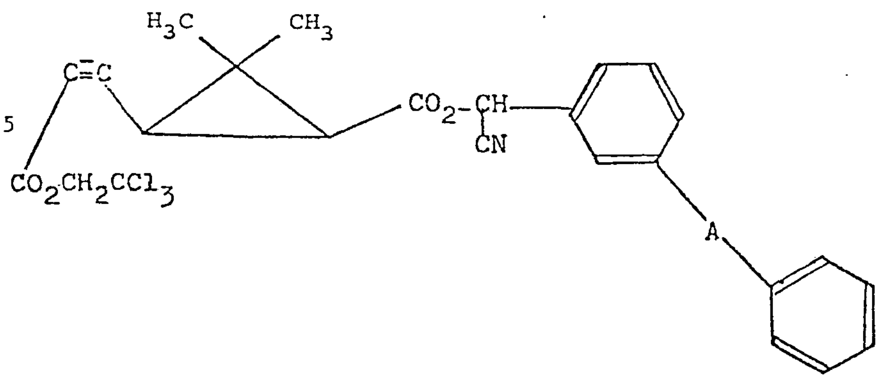

(XIV)

que l'on soumet à l'action d'un agent de clivage de la fonction ester porté par le carbone acétylénique pour obtenir le composé de formule (XV) :

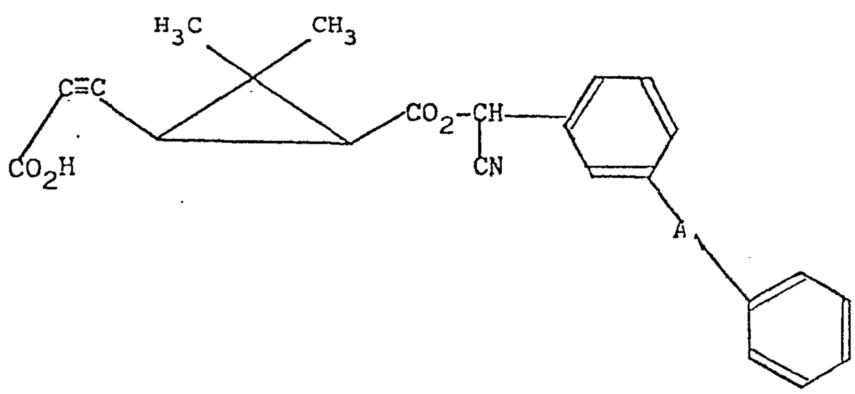

(XV)

que l'on soumet à l'action d'un agent d'hydrogénation ménagée, pour obtenir le composé de formule (XI).

7. Procédé selon la revendication 6, caractérisé en ce que l'on soumet un composé de formule (XV) :

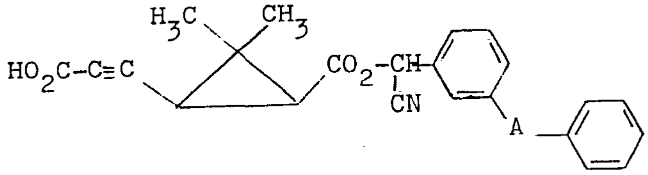

(XV)

dans laquelle la copule cyclopropanique est de structure 1R, cis et A est défini comme à la revendication 1, à l'action d'un agent d'estérification, pour obtenir le composé de formule (X) :

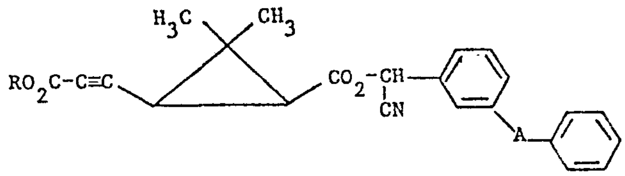

(X)

dans laquelle R et A sont définis comme à la revendication 1, que l'on soumet à l'action d'un agent d'hydrogénation ménagée, pour obtenir le composé de formule (I).

8. Procédé selon l'une quelconque des revendications 1 à 7, caractérisé en ce que A représente un atome d'oxygène et en ce que le carbone porteur du radical cyano dans la copule alcool est de configuration (S).

9. Procédé selon l'une quelconque des revendications 1 à 7, caractérisé en ce que R représente un radical méthyle, éthyle, propyle, isopropyle, butyle ou tert-butyle.

10. Procédé selon l'une quelconque des revendications 1 à 7, caractérisé en ce que R représente un radical cyclopropyle ou cyclopropyl méthyle.

11. Procédé selon l'une quelconque des revendications 1 à 7, caractérisé en ce que l'on prépare l'un quelconque des composés de formule (I), dont les noms suivent :

— le (1R, cis) 2,2-diméthyl 3-[(Z) 2-(méthoxy carbonyl) éthényl] cyclopropane carboxylate de (S) α-cyano 3-phénoxy benzyle,

— le (1R, cis) 2,2-diméthyl 3-[(Z) 2-(méthoxy carbonyl) éthényl] cyclopropane carboxylate de (S) α-cyano 3-phénoxy benzyle,

— le (1R, cis) 2,2-diméthyl 3-[(Z) 2-(isopropyloxy carbonyl) éthényl] cyclopropane carboxylate de (S) α-cyano 3-phénoxy benzyle,

— le (1R, cis) 2,2-diméthyl 3-[(Z) 2-(cyclopropylméthoxycarbonyl) éthényl] cyclopropane carboxylate de (S) α-cyano 3-phénoxybenzyle,

— le (1R, cis) 2,2-diméthyl 3-[(Z) 2-(cyclopropyloxycarbonyl) éthényl] cyclopropane carboxylate de (S) α-cyano 3-phénoxybenzyle,

— le (1R, cis) 2,2-diméthyl 3-[(Z) 2-(terbutoxycarbonyl) éthényl] cyclopropane carboxylate de (S) α-cyano 3-phénoxybenzyle.

12. Application des composés de formule (1), tels que définis à la revendication 1, à la lutte contre les parasites des végétaux et les parasites domestiques.

13. Les compositions destinées à la lutte contre les parasites des végétaux, les parasites domestiques et les parasites des animaux à sang chaud, caractérisées en ce qu'elles renferment au moins l'un des produits définis à la revendication 1.

14. Les compositions insecticides renfermant comme principe actif l'un quelconque des produits défini à la revendication 1.

15. Les compositions insecticides renfermant comme principe actif au moins l'un des produits définis à la revendication 11.

16. Les compositions destinées à la lutte contre les acariens parasites des végétaux, caractérisées en ce qu'elles renferment au moins l'un des produits définis à la revendication 1.

17. Les compositions destinées à la lutte contre les acariens parasites des végétaux, caractérisées en ce qu'elles renferment comme principe actif le (1R, cis) 2,2-diméthyl 3-[(Z) 2-(terbutoxycarbonyl) éthényl] cyclopropane carboxylate de (S) α-cyano 3-phénoxybenzyle.

18. Les compositions destinées à l'alimentation animale, renfermant comme principe actif au moins l'un des produits définis à la revendication 1, associé à un aliment destiné à l'alimentation animale.

19. Associations douées d'activité insecticide, acaricide ou nématicide, caractérisées en ce qu'elles contiennent comme matière active, d'une part un au moins des composés de formule générale (I), et d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxybenzylique et d'alcools α-cyano 3-phénoxybenzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxybenzylique et d'alcools α-cyano 3-phénoxybenzyliques des acides 2,2-diméthyl 3-(2,2-dichlorovinyl) cyclopropane-1-carboxyliques, par les esters d'alcools α-cyano 3-phénoxybenzyliques d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolones, d'alcool 3,4,5,6-tétrahydrophalimidométhylique, d'alcool 5-benzyl 3-furyl méthylique, d'alcool 3-phénoxybenzylique et d'alcools α-cyano 3-phénoxybenzyliques des acides 2,2-diméthyl 3-(1,2,2,2-tétrahalo) cyclopropane-1-carboxyliques, dans lesquels « halo » représente un atome de fluor, de chlore ou de brome, étant entendu que les composés I peuvent exister sous toutes leurs formes stéréoisomères possibles de même que les copules acides et alcools des esters pyréthrinoïdes ci-dessus.

**Claims** (for the Contracting States : BE, CH, DE, FR, GB, IT, LI, LU, NL, SE)

1. The compounds corresponding to the formula (I) :

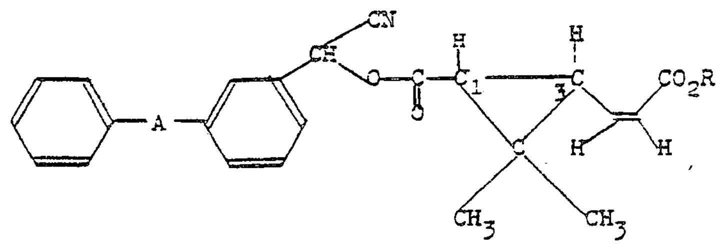

in which A represents an oxygen atom, a methylene group or a carbonyl group, and R represents an alkyl radical, linear, branched or cyclic, saturated or unsaturated, containing 1-8 carbon atoms, in which the double bond has Z geometry and the cyclopropane copula is of 1R, cis structure in all their possible stereo-isomeric forms, as well as the mixtures of these isomers.

2. The compounds with the formula (I), as defined in Claim 1, for which A represents an oxygen atom.

3. The compounds with the formula (I), as defined in either of Claims 1 or 2, for which the alcohol copula of the ester in position 1 of the cyclopropane is the residue of (S) α-cyano-3-phenoxybenzyl alcohol.

4. The compounds with the formula (I) as defined in any one of Claims 1 to 3, for which R represents a methyl, ethyl, propyl, isopropyl, butyl or tert-butyl radical.

5. The compounds with the formula (I), as defined in any one of Claims 1 to 3, for which R represents a cyclopropyl or cyclopropylmethyl radical.

6. The compound with the formula (I), as defined in Claim 1, the name of which follows :

— (1R, cis) 2,2-dimethyl-3-[(Z) 2-(methoxycarbonyl)-ethenyl] cyclopropane carboxylate of (S) α-cyano-3-phenoxybenzyl.

7. Any one of the compounds with the formula (I), as defined in Claim 1, the names of which follow :

— (1R, cis) 2,2-dimethyl-3-[(Z) 2-(ethoxycarbonyl)-ethenyl] cyclopropane carboxylate of (S) α-cyano-3-phenoxybenzyl,

— (1R, cis) 2,2-dimethyl-3-[(Z) 2-(isopropyloxycarbonyl)-ethenyl] cyclopropane carboxylate of (S) α-cyano-3-phenoxybenzyl,

— (1R, cis) 2,2-dimethyl-3-[(Z) 2-(cyclopropylmethoxycarbonyl)-ethenyl] cyclopropane carboxylate of (S) α-cyano-3-phenoxybenzyl,

— (1R, cis) 2,2-dimethyl-3-[(Z) 2-(cyclopropyloxycarbonyl)-ethenyl] cyclopropane carboxylate of (S) α-cyano-3-phenoxybenzyl,

— (1R, cis) 2,2-dimethyl-3-[(Z) 2-(tertbutoxycarbonyl)-ethenyl] cyclopropane carboxylate of (S) α-cyano-3-phenoxybenzyl.

8. Use of the compounds with the formula (I), as defined in any one of Claims 1-7, in combatting parasites of vegetation and parasites of premises.

9. The compositions intended for combatting parasites of vegetation, parasites of premises and parasites of warm-blooded animals, characterized in that they contain at least one of the products defined in any one of Claims 1-7.

10. Insecticidal compositions containing as active principle any one of the products defined in any one of Claims 1-5.

11. Insecticidal compositions containing as active principle the product defined in Claim 6.

12. Insecticidal compositions containing as active principle at least one of the products defined in Claim 7.

13. Compositions intended for combatting parasitic acaridae of vegetation, characterized in that they contain at least one of the products defined in any one of Claims 1-7.

14. Compositions intended for combatting parasitic acaridae of vegetation, characterized in that they contain as active principle (1R, cis) 2,2-dimethyl-3-[(Z) 2-(tertbutoxycarbonyl)-ethenyl] cyclopropane carboxylate of (S) α-cyano-3-phenoxybenzyl.

15. Compositions intended for animal feeding, containing as active principle at least one of the products defined in any one of Claims 1-7, combined with a food intended for animal feeding.

16. As a medicament, the compounds defined in any one of Claims 1-7.

17. Combinations endowed with insecticidal, acaricidal or nematocidal activity, characterized in that they contain as active material, for the one part at least one of the compounds with the general formula (I), and for the other part, at least one of the pyrethrinoid esters chosen from the group constituted by the esters of allethrolones, of 3,4,5,6-tetrahydrophthalimido methyl alcohol, of 5-benzyl-3-furylmethyl alcohol, of 3-phenoxybenzyl alcohol and of α-cyano-3-phenoxybenzyl alcohols with chrysanthemic acids, by the esters of 5-benzyl-3-furyl-methyl alcohol with 2,2-dimethyl-3-(2-oxo-3-tetrahydrothiophenylidenemethyl) cyclopropane-1-carboxylic acids, by the esters of 3-phenoxybenzyl alcohol and of α-cyano-3-phenoxybenzyl alcohols with 2,2-dimethyl-3-(2,2-dichlorovinyl) cyclopropane-1-carboxylic acids, by the esters of α-cyano-3-phenoxybenzyl alcohols with 2,2-dimethyl-3-(2,2-dibromovinyl) cyclopropane-1-carboxylic acids, by the esters of 3-phenoxybenzyl alcohol with 2-parachlorophenyl-2-isopropyl acetic acids, by the esters of allethrolones, of 3,4,5,6-tetrahydrophthalimidomethyl alcohol, of 5-benzyl-3-furylmethyl alcohol, of 3-phenoxybenzyl alcohol and of α-cyano-3-phenoxybenzyl alcohols with 2,2-dimethyl-3-(1,2,2,2-tetrahaloethyl) cyclopropane-1-carboxylic acids, in which « halo » represents a fluorine, chlorine or bromine atom, it being understood that the compounds (I) may exist in all their possible stereo-isomeric forms, likewise the acid and alcohol copulae of the above pyrethrinoid esters.

18. Preparation process for the compounds with the formula (I), as defined in any one of Claims 1-7, characterized in that an acid with the formula (II) :

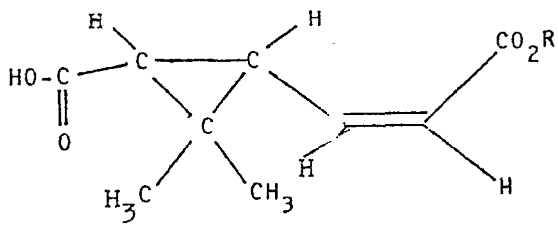

(II)

in which the double bond has Z geometry and the cyclopropane copula is of 1R, cis structure and R retains the same significance as in Claim 1, or a functional derivative of this acid, is submitted to the action of an alcohol with the formula :

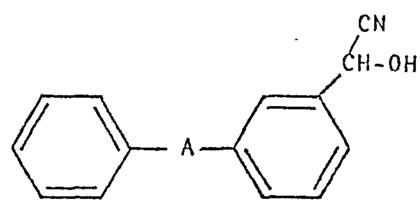

(III)

in which A retains the same significance as in Claim 1.

19. Preparation process according to Claim 18, characterized in that the compound with the formula (II) utilized is prepared by making a compound with the formula (IV) :

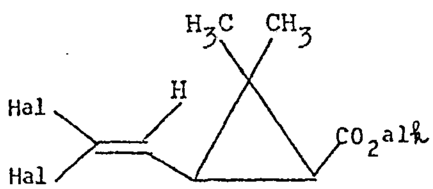

(IV)

in which the cyclopropane copula is of 1R, cis structure and Hal represents a halogen atom and alk represents an alkyl radical containing 1-8 carbon atoms, react, firstly with an alkaline agent able to extract the halogen atoms, and secondly :

— either with an agent able to introduce the carboxyl group, so as to obtain the compound with the formula (V) :

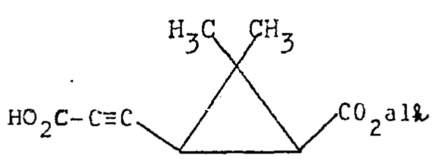

(V)

which is submitted to the action of an esterification agent so as to obtain the compound with the formula (VI) :

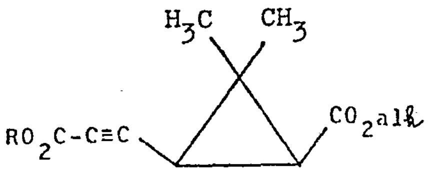

(VI)

in which R retains the same significance as previously,

— or with an alkyl chloroformate with the formula :

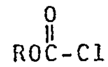

(V')

in which R retains the preceding significance, so as to obtain directly the compound with the formula (VI) :

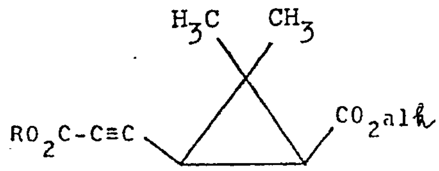

(VI)

then by submitting the compound with the formula (VI) obtained to the action of a mild hydrogenation agent so as to obtain the compound with the formula :

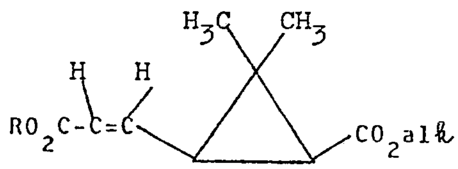

(VII)

in which the double bond has Z geometry, which is submitted to the action of an acid hydrolizing agent able to cleave selectively the ester function in position 1 of the cyclopropane, so as to obtain the corresponding compound with the formula (II) :

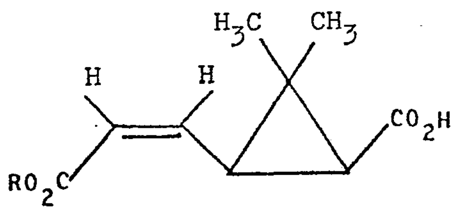

(II)

20. Process according to Claim 19, characterized in that a compound with the formula (V) :

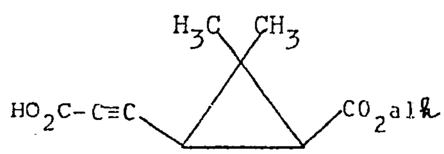

(V)

in which the cyclopropane copula is of 1R, cis structure and alk retains its preceding significance, is submitted, firstly to the action of a mild hydrogenation agent, so as to obtain a compound with the formula (VIII) :

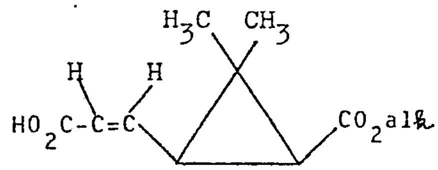

(VIII)

in which the double bond has Z geometry, which is submitted to the action of an esterification agent, so as to obtain the corresponding compound with the formula (VII) :

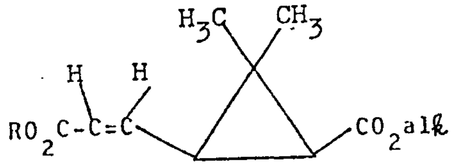

(VII)

then the synthesis is continued as described in Claim 20.

21. Process according to Claims 18 and 19, characterized in that a compound with the formula (VI) :

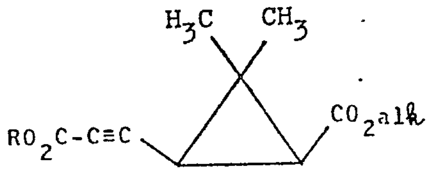

(VI)

in which the cyclopropane copula is of 1R, cis structure and R and alk are defined as previously, is submitted to the action of an acid hydrolizing agent capable of selectively cleaving the ester function in position 1 of the cyclopropane, so as to obtain the compound with the formula (IX) :

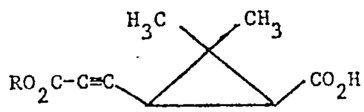

(IX)

in which R is defined as previously, which

— either, if applicable, is submitted, in the form of a functional derivative, to the action of an alcohol with the formula (III) :

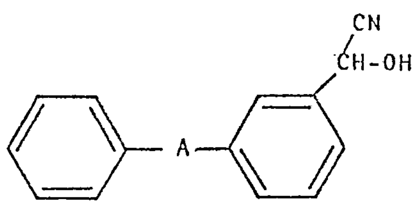

(III)

in which A retains the same significance as previously, so as to obtain a compound with the formula (X) :

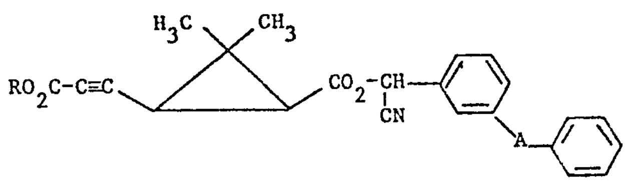

(X)

in which R and A retain the same significance as previously, which is submitted to the action of a mild hydrogenation agent so as to obtain the compound with the formula (I),

— or, firstly is submitted to the action of a mild hydrogenation agent, so as to obtain the compound with the formula (II) :

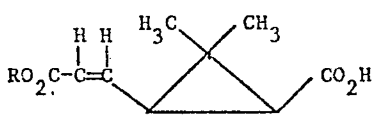

(II)

in which R is defined as previously and the double bond has Z geometry, then, if applicable in the form of a functional derivative, to the action of an alcohol (III), so as to obtain the compound with the formula (I).

22. Preparation process for the compounds with the formula (I) as defined in any one of Claims 1-7, characterized in that a compound with the formula (XI)

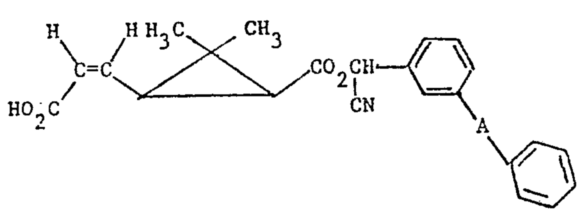

(XI)

in which the double bond has Z geometry and the cyclopropane copula is of 1R, cis structure and A is

defined as in Claim 1, is submitted to the action of an esterification agent, so as to obtain the corresponding compound with the formula (I):

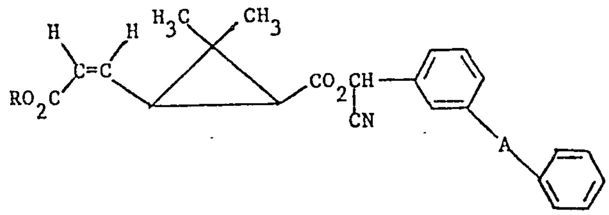

(I)

in which R is defined as in Claim 1.

23. Process according to Claim 22, characterized in that the compound with the formula (XI) is prepared by submitting an acid with the formula (V):

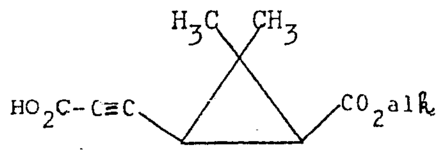

(V)

in which the cyclopropane copula is of 1R, cis structure and alk represents an alkyl radical containing 1-8 carbon atoms, to the action of 2,2,2-trichloroethanol so as to obtain the compound with the formula (XII):

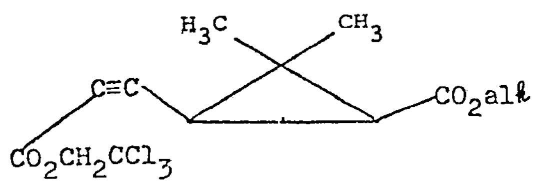

(XII)

which is submitted to the action of an acid hydrolizing agent so as to obtain the compound with the formula (XIII):

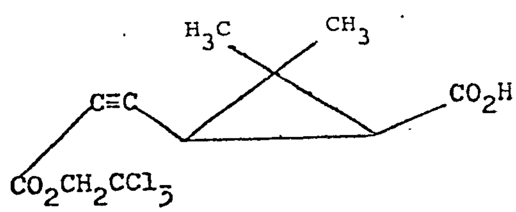

(XIII)

which is submitted to the action of an alcohol with the formula (III):

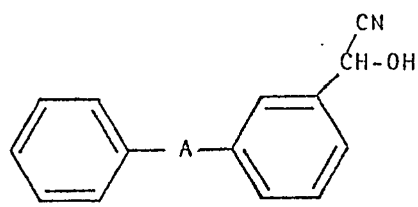

(III)

in which A retains the same significance as in Claim 1, so as to obtain the compound with the formula (XIV):

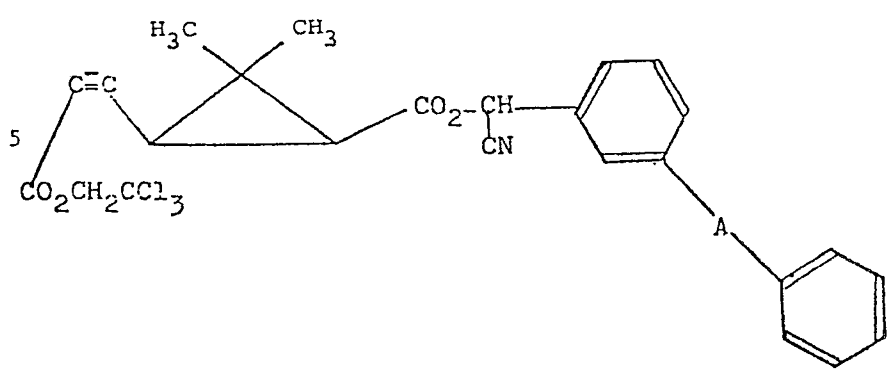

(XIV)

which is submitted to the action of a cleavaging agent of the ester carried by the acetylene carbon, so as to obtain the compound with the formula (XV) :

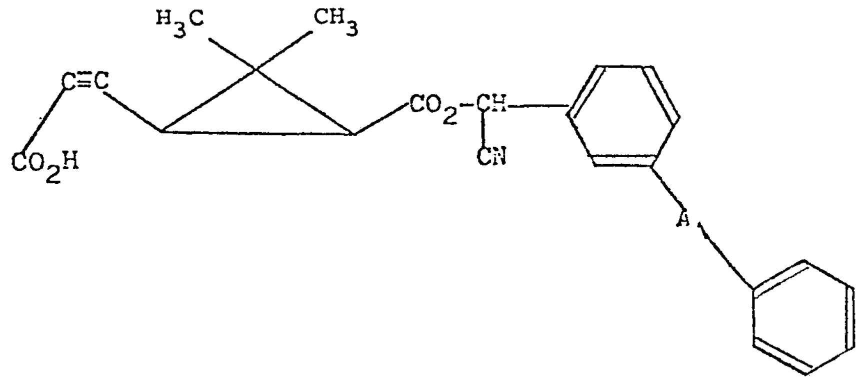

(XV)

which is submitted to the action of a mild hydrogenation agent so as to obtain the compound with the formula (XI).

24. Prucess according to Claim 23, characterized in that a compound with the formula (XV) :

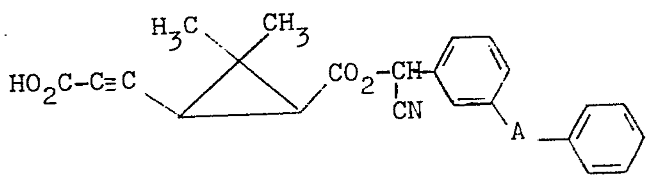

(XV)

in which the cyclopropane copula is of 1R, cis structure and A is defined as in Claim 1, is submitted to the action of an esterification agent, so as to obtain the compound with the formula (X) :

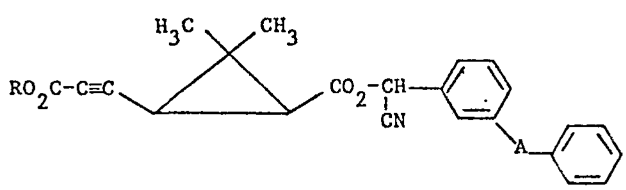

(X)

in which R and A are defined as in Claim 1, which is submitted to the action of a mild hydrogenation agent, so as to obtain the compound with the formula (I).

25. As new industrial products an notably as intermediate products necessary for putting the process of the invention into operation as defined in any one of Claims 18 to 21, the compound with the formulae : (II), (V), (VI), (VII), (VIII), (IX) and (X), as defined in Claims 18 to 21.

26. As new industrial products and notably as intermediate products necessary for putting the process of the invention into operation as defined in any one of Claims 22 to 24, the compounds with the formulae : (XI), (XII), (XIII), (XIV) and (XV), as defined in Claims 22 to 24.

**Claims** (for the Contracting State AT)

1. Process for preparing the compounds corresponding to the formula (I) :

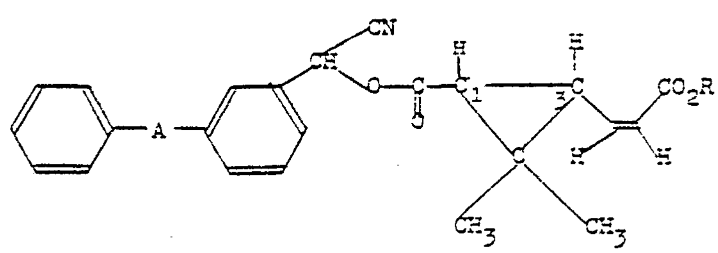

(I)

in which A represents an oxygen atom, a methylene group or a carbonyl group, and R represents an alkyl radical, linear, branched, or cyclic, saturated or unsaturated, containing 1-8 carbon atoms, in which the

double bond has Z geometry, and the cyclopropane moiety is of 1R, cis structure, in all their possible stereo-isomeric forms, as well as the mixtures of these stereo-isomers, characterized in that an acid with the formula (II) :

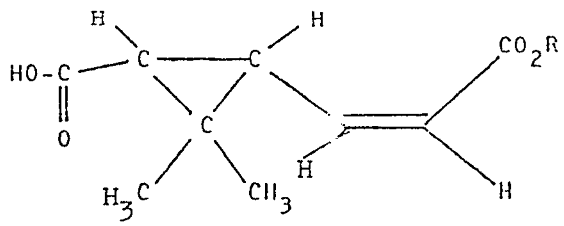

(II)

in which the double bond has Z geometry and the cyclopropane moiety is of 1R, cis structure and R retains the same significance as above, or a functional derivative of this acid, is submitted to the action of an alcohol with the formula (III) :

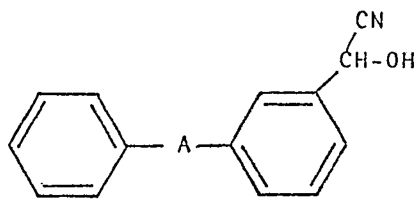

(III)

in which A retains the same significance as above.

2. Preparation process according to Claim 1, characterized in that the compound with the formula (II) used is prepared by making a compound with the formula (IV) :

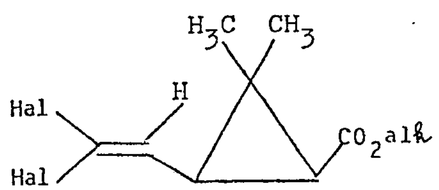

(IV)

in which the cyclopropane moiety is of 1R, cis structure, Hal represents a halogen atom and alk represents an alkyl radical containing 1-8 carbon atoms, react, firstly, with an alkaline agent able to extract the halogen atoms and secondly :

— either with an agent capable of introducing the carboxyl group, so as to obtain the compound with the formula (V) :

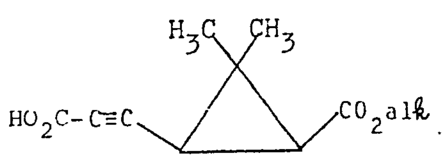

(V)

which is submitted to the action of an esterification agent so as to obtain the compound with the formula (VI) :

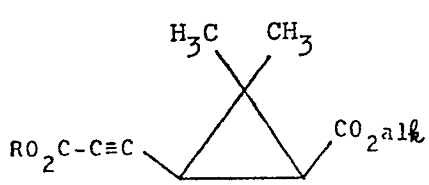

(VI)

in which R retains the same significance as previously,

— or with an alkyl chloroformate with the formula (V') :

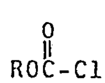

(V')

in which R retains the preceding significance, so as to obtain directly the compound with the formula (VI) :

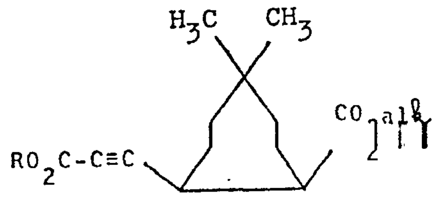

(VI)

then by submitting the compound with the formula (VI) obtained to the action of a mild hydrogenation agent so as to obtain the compound with the formula (VII) :

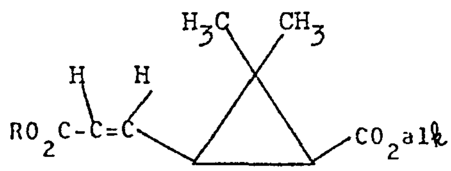

(VII)

in which the double bond has Z geometry, which is submitted to the action of an acid hydrolizing agent able to cleave selectively the ester function in position 1 of the cyclopropane so as to obtain the corresponding compound with the formula (II) :

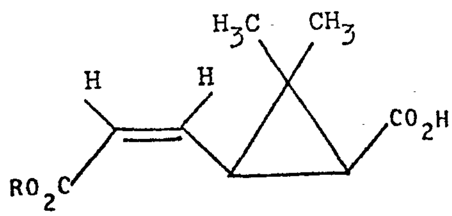

(II)

3. Process according to Claim 2, characterized in that a compound with the formula (V) :

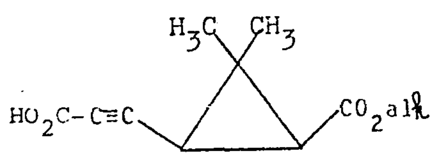

(V)

in which the cyclopropane moiety is of 1R, cis structure and alk retains its preceding significance, is submitted firstly to the action of a mild hydrogenation agent, so as to obtain a compound with the formula (VIII) :

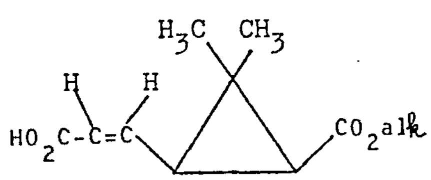

(VIII)

in which the double bond has Z geometry, which is submitted to the action of an esterification agent, so as to obtain the corresponding compound with the formula (VII) :

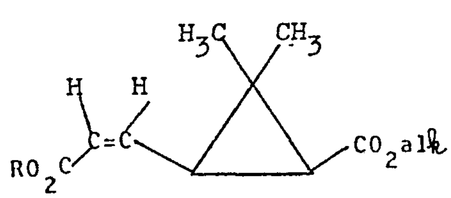

(VII)

then the synthesis is continued as described in Claim 2.

4. Process according to Claims 1 and 2, characterized in that a compound with the formula (VI) :

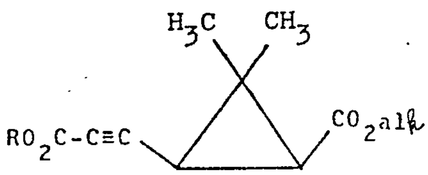

(VI)

in which the cyclopropane moiety is of 1R, cis structure and R and alk are defined as previously, is submitted to the action of an acid hydrolizing agent able to cleave selectively the ester function in position 1 of the cyclopropane, so as to obtain the compound with the formula (IX) :

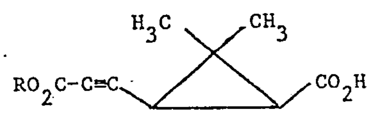

(IX)

in which R is defined as previously, which

— either, if applicable, in the form of a functional derivative, is submitted to the action of an alcohol with the formula (III) :

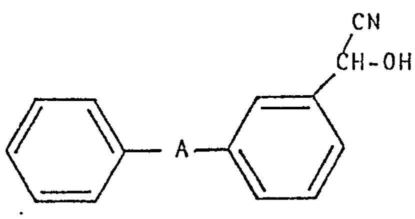

(III)

in which A retains the same significance as previously, so as to obtain a compound with the formula (X) :

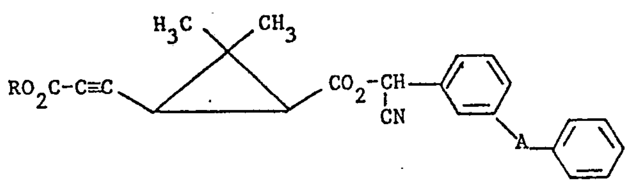

(X)

in which R and A retain the same significance as previously, which is submitted to the action of a mild hydrogenation agent, so as to obtain the compound with the formula (I),

— or is submitted firstly to the action of a mild hydrogenation agent, so as to obtain the compound with the formula (II) :

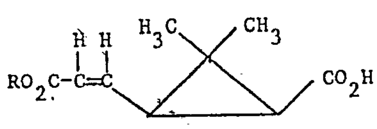

(II)

in which R is defined as previously and the double bond has Z geometry, then, if applicable, in the form of a functional derivative, to the action of an alcohol (III), so as to obtain the compound with the formula (I).

5. Preparation process for the compounds with the formula (I), as defined in Claim 1, characterized in that a compound with the formula (XI) :

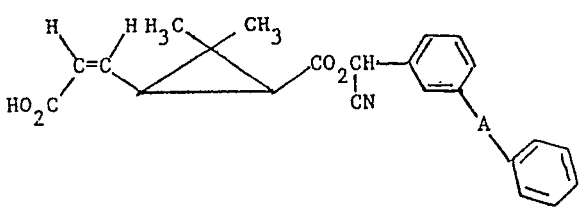

(XI)

in which the double bons has Z geometry and the cyclopropane moiety is of 1R, cis structure, and A is defined as in Claim 1, is submitted to the action of an esterification agent, so as to obtain the corresponding compound with the formula (I) :

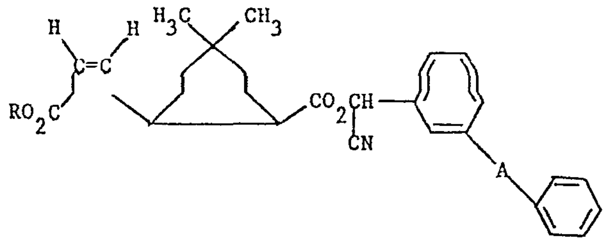

(I)

in which R is defined as in Claim 1.

6. Process according to Claim 5, characterized in that the compound with the formula (XI) is prepared by submitting an acid with the formula (V) :

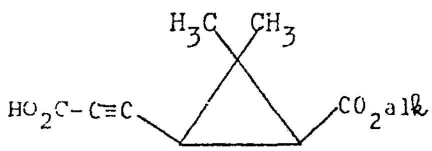

(V)

in which the cyclopropane moiety is of 1R, cis structure and alk represents an alkyl radical containing 1-8 carbon atoms, to the action of 2,2,2-trichloroethanol so as to obtain the compound with the formula (XII) :

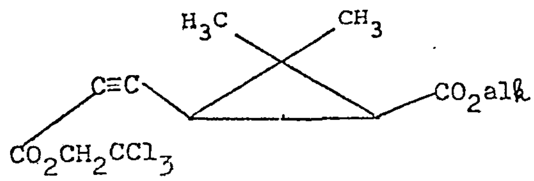

(XII)

which is submitted to the action of an acid hydrolizing agent so as to obtain the compound with the formula (XIII) :

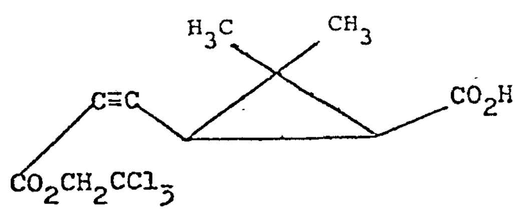

(XIII)

which is submitted to the action of an alcohol with the formula (III) :

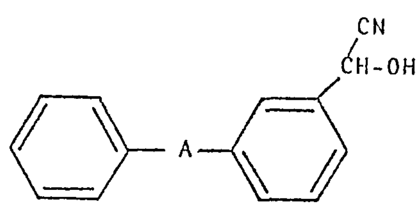

(III)

in which A retains the same significance as in Claim 1, so as to obtain the compound with the formula (XIV) :

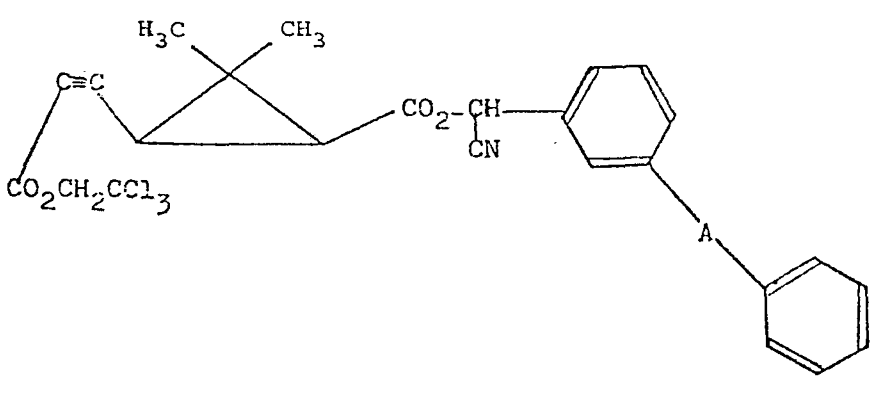

(XIV)

which is submitted to the action of a cleavaging agent of the ester function carried by the acetylene carbon, so as to obtain the compound with the formula (XV) :

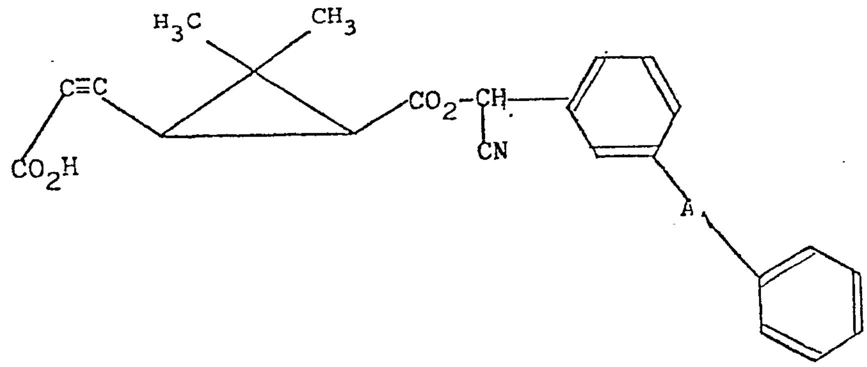

(XV)

which is submitted to the action of a mild hydrogenation agent, so as to obtain the compound with the formula (XI).

7. Process according to Claim 6, characterized in that a compound with the formula (XV) :

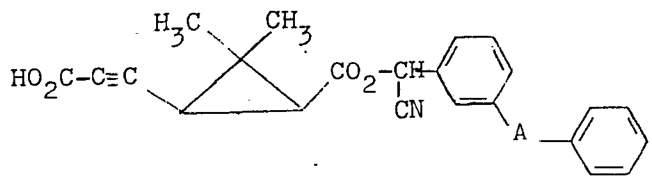

(XV)

in which the cyclopropane moiety is of 1R, cis structure and A is defined as in Claim 1, is submitted to the action of an esterification agent, so as to obtain the compound with the formula (X) :

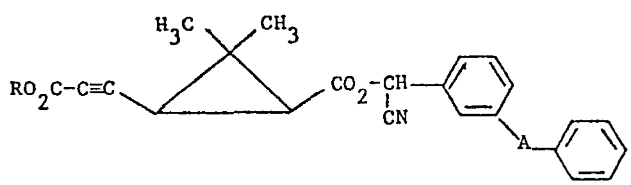

(X)

in which R and A are defined as in Claim 1, which is submitted to the action of a mild hydrogenation agent so as to obtain the compound with the formula (I).

8. Process according to any one of Claims 1 to 7 characterized in that A represents an oxygen atom and the carbon atom bearing the cyano radical in the alcohol moiety is of (S) configuration.

9. Process according to any one of Claims 1 to 7 characterized in that R represents a methyl, ethyl, propyl, isopropyl, butyl or tert-butyl radical.

10. Process according to any one of Claims 1 to 7 characterized in that R represents a cyclopropyl or cyclopropylmethyl radical.

11. Process according to any one of Claims 1 to 7 characterized in that any one of compounds with the formula (I), of which the name follow are prepared :

— (1R, cis) 2,2-dimethyl-3-[(Z) 2-(methoxycarbonyl)-ethenyl] cyclopropane carboxylate of (S) α-cyano-3-phenoxybenzyl.

— (1R, cis) 2,2-dimethyl-3-[(Z) 2-(ethoxycarbonyl)-ethenyl] cyclopropane carboxylate of (S) α-cyano-3-phenoxybenzyl,

— (1R, cis) 2,2-dimethyl-3-[(Z) 2-(isopropyloxycarbonyl)-ethenyl] cyclopropane carboxylate of (S) α-cyano-3-phenoxybenzyl,

— (1R, cis) 2,2-dimethyl-3-[(Z) 2-(cyclopropylmethoxycarbonyl)-ethenyl] cyclopropane carboxylate of (S) α-cyano-3-phenoxybenzyl,

— (1R, cis) 2,2-dimethyl-3-[(Z) 2-(cyclopropyloxycarbonyl)-ethenyl] cyclopropane carboxylate of (S) α-cyano-3-phenoxybenzyl,

— (1R, cis) 2,2-dimethyl-3-[(Z) 2-tertbutoxycarbonyl)-ethenyl] cyclopropane carboxylate of (S) α-cyano-3-phenoxybenzyl.

12. Use of the compounds with the formula (I), as defined in Claim 1, for combatting parasites of vegetation and household parasites.

13. Compositions intended for combatting parasites of vegetation, household parasites and parasites of warm-blooded animals, characterized in that they contain at least one of the products defined in Claim 1.

14. The insecticidal compositions containing as active principle any one of the products defined in Claim 1.

15. The insecticidal compositions containing as active principle at least one of products defined in Claim 11.

16. The compositions intended for combatting parasitic acaridae of vegetation, characterized in that they contain at least one of the products defined in Claim 1.

17. The compositions intended for combatting parasitic acaridae of vegetation, characterized in that they contain as active principle (1R, cis) 2,2-dimethyl-3-[(Z) 2-(tertbutoxycarbonyl)-ethenyl] cyclopropane carboxylate of (S) α-cyano-3-phenoxybenzyl.

18. The compositions intended for animal feeding, containing as active principle at least one of the products defined in Claim 1, combined with a food intended for animal feeding.

19. Combinations endowed with insecticidal, acaricidal or nematocidal activity, characterized in that they contain as active material, for the one part at least one of the compounds with the general formula (I), and for the other part, at least one of the pyrethrinoid esters chosen form the group constituted by the esters of allethrolones, of 3,4,5,6-tetrahydrophthalimidomethylalcohol, of 5-benzyl-3-furylmethyl alcohol, of 3-phenoxybenzyl alcohol and of α-cyano-3-phenoxybenzyl alcohols with chrysanthemic acids, by the esters of 5-benzyl-3-furylmethyl alcohol with 2,2-dimethyl-3-(2-oxo-3-tetrahydrothiophenylidenemethyl) cyclopropane-1-carboxylic acids, by the esters of 3-phenoxybenzyl alcohol and of α-cyano-3-phenoxybenzyl alcohols with 2,2-dimethyl-3-(2,2-dichlorovinyl) cyclopropane-1-carboxylic acids, by the esters of α-cyano-3-phenoxybenzyl alcohols with 2,2-dimethyl-3-(2,2-dibromovinyl) cyclopropane-1-carboxylic acids, by the esters of 3-phenoxybenzyl alcohol with 2-parachlorophenyl-2-isopropyl acetic acids, by the esters of allethrolones, of 3,4,5,6-tetrahydrophthalimidomethyl alcohol, of 5-benzyl-3-furylmethyl alcohol, of 3-phenoxybenzyl alcohol and of α-cyano-3-phenoxybenzyl alcohols with 2,2-dimethyl-3-(1,2,2,2-tetrahalo) cyclopropane-1-carboxylic acids, in which « halo » represents a fluorine, chlorine or bromine atom, it being understood that the compounds (I) may exist in all their possible stereo-isomeric forms, likewise the acid and alcohol copulae of the above pyrethrinoid esters.

**Ansprüche** (für die Vertragsstaaten : BE, CH, DE, FR, GB, IT, LI, LU, NL, SE)

1. Verbindungen der Formel (I)

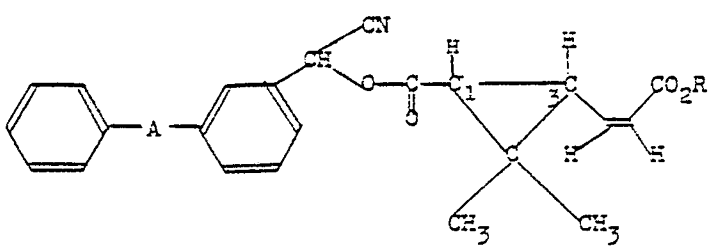

worin A ein Sauerstoffatom, eine Methylengruppe oder eine Carbonylgruppe bedeutet und R einen gesättigten oder ungesättigten, linearen, verzweigten oder cyclischen Alkylrest mit 1 bis 8 Kohlenstoffatomen darstellt, worin die Doppelbindung die Z-Geometrie aufweist und die Cyclopropan-Verknüpfung 1R-cis-Struktur besitzt, in sämtlichen ihrer möglichen Stereoisomeren Formen sowie die Gemische dieser Stereoisomeren.

2. Verbindungen der Formel (I) gemäß Anspruch 1, worin A ein Sauerstoffatom bedeutet.

3. Verbindungen der Formel (I) gemäß einem der Ansprüche 1 und 2, worin die alkoholische Verknüpfungskomponente des Esters in 1-Stellung des Cyclopropans der Rest des (S)-α-Cyano-3-phenoxybenzylalkohols ist.

4. Verbindungen der Formel (I) gemäß einem der Ansprüche 1 bis 3, worin R einen Methyl-, Ethyl-, Propyl-, Isopropyl-, Butyl- oder tert.-Butylrest bedeutet.

5. Verbindungen der Formel (I) gemäß einem der Ansprüche 1 bis 3, worin R einen Cyclopropyl- oder Cyclopropylmethylrest bedeutet.

6. Verbindung der Formel (I) gemäß Anspruch 1 mit der folgenden Bezeichnung :

— (S)-α-Cyano-3-phenoxybenzyl-(1R-cis)-2,2-dimethyl-3-[(Z)-2-(methoxycarbonyl)-ethenyl]-cyclopropancarboxylat.

7. Eine der Verbindungen der Formel (I) gemäß Anspruch 1 mit den folgenden Bezeichnungen :

— (S)-α-Cyano-3-phenoxybenzyl-(1R-cis)-2,2-dimethyl-3-[(Z)-2-(ethoxycarbonyl)-ethenyl)-cyclopropancarboxylat,

— (S)-α-Cyano-3-phenoxybenzyl-(1R-cis)-2,2-dimethyl-3-[(Z)-2-(isopropyloxycarbonyl)-ethenyl]-cyclopropancarboxylat,

— (S)-α-Cyano-3-phenoxybenzyl-(1R-cis)-2,2-dimethyl-3-[(Z)-2-(cyclopropylmethoxycarbonyl)-ethenyl]-cyclopropancarboxylat,

— (S)-α-Cyano-3-phenoxybenzyl-(1R-cis)-2,2-dimethyl-3-[(Z)-2-(cyclopropyloxycarbonyl)-ethenyl]-cyclopropancarboxylat,

— (S)-α-Cyano-3-phenoxybenzyl-(1R-cis)-2,2-dimethyl-3-[(Z)-2-(tert.-butoxycarbonyl)-ethenyl]-cyclopropancarboxylat.

8. Verwendung der Verbindungen der Formel (I) gemäß einem der Ansprüche 1 bis 7 zur Bekämpfung von Parasiten der Pflanzen und Parasiten im häuslichen Bereich.

9. Zusammensetzungen zur Bekämpfung von Parasiten der Pflanzen, Parasiten im häuslichen Bereich und Parasiten warmblütiger Tiere, dadurch gekennzeichnet, daß sie zumindest eines der Produkte gemäß einem der Ansprüche 1 bis 7 enthalten.

10. Insektizide Zusammensetzungen, enthaltend als Wirkstoff eines der Produkte gemäß einem der Ansprüche 1 bis 5.

11. Insektizide Zusammensetzungen, enthaltend als Wirkstoff das Produkt gemäß Anspruch 6.

12. Insektizide Zusammensetzungen, enthaltend als Wirkstoff zumindest eines der Produkte gemäß Anspruch 7.

13. Zusammensetzungen für die Bekämpfung von parasitären Milben der Pflanzen, dadurch gekennzeichnet, daß sie zumindest eines der Produkte gemäß einem der Ansprüche 1 bis 7 enthalten.

14. Zusammensetzungen zur Bekämpfung von parasitären Milben der Pflanzen, dadurch gekennzeichnet, daß sie als Wirkstoff das (S)-α-Cyano-3-phenoxybenzyl-(1R-cis)-2,2-dimethyl-3-[(Z)-2-(tert.-butoxycarbonyl)-ethenyl]-cyclopropancarboxylat enthalten.

15. Zusammensetzungen für die tierische Ernährung, enthaltend als Wirkstoff zumindest eines der Produkte gemäß einem der Ansprüche 1 bis 7 zusammen mit einem für die tierische Ernährung bestimmten Nährmittel.

16. Als Arzneimittel die Verbindungen gemäß einem der Ansprüche 1 bis 7.

17. Mit insektizider, akarizider oder nematizider Aktivität ausgestattete Assoziationen, dadurch gekennzeichnet, daß sie als Wirkstoff einesteils zumindest eine der Verbindungen der allgemeinen Formel (I) und anderenteils zumindest einen der Pyrethrinoidester enthalten, ausgewählt unter den Estern der Allethrolone, des 3,4,5,6-Tetrahydrophthalimido-methylalkohols, des 5-Benzyl-3-furyl-methylalkohols, des 3-Phenoxybenzylalkohols und der α-Cyano-3-phenoxybenzylalkohole der Chrysanthemumsäuren, den Estern des 5-Benzyl-3-furylmethylalkohols der 2,2-Dimethyl-3-(2-oxo-3-tetrahydrothiophenylidenmethyl)-cyclopropan-1-carbonsäuren, den Estern des 3-Phenoxybenzylalkohols und der α-Cyano-3-phenoxybenzylalkohole der 2,2-Dimethyl-3-(2,2-dichlorvinyl)-cyclopropan-1-carbonsäuren, den Estern der α-Cyano-3-phenoxybenzylalkohole der 2,2-Dimethyl-3-(2,2-dibromvinyl)-cyclopropan-1-carbonsäuren, den Estern des 3-Phenoxybenzylalkohols der 2-p-Chlorphenyl-2-isopropylessigsäuren, den Estern der Allethrolone, des 3,4,5,6-Tetrahydrophthalimidomethylalkohols, des 5-Benzyl-3-furylmethylalkohols, des 3-Phenoxybenzylalkohols und der α-Cyano-3-phenoxybenzylalkohole der 2,2-Dimethyl-3-(1,2,2,2-tetrahalo)-cyclopropan-1-carbonsäuren, worin « halo » ein Fluor-, Chlor- oder Bromatom bedeutet, wobei die Verbindungen I in sämtlichen ihrer möglichen stereoisomeren Formen vorliegen können ebenso wie die Säure- und Alkoholkomponenten der vorstehenden Pyrethrinoidester.

18. Verfahren zur Herstellung der Verbindungen der Formel (I) gemäß einem der Ansprüche 1 bis 7, dadurch gekennzeichnet, daß man eine Säure der Formel II

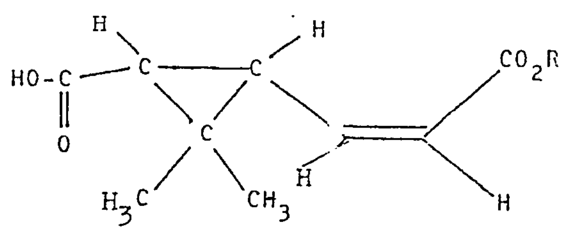

(II)

worin die Doppelbindung die Z-Geometrie besitzt und die Cyclopropan-Verknüpfung 1R-cis-Struktur aufweist und R die in Anspruch 1 angegebene Bedeutung besitzt, oder ein funktionelles Derivat dieser Säure mit einem Alkohol der Formel :

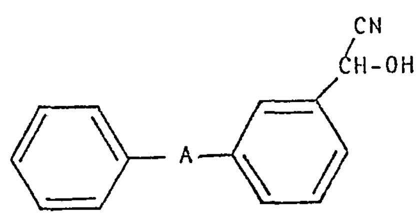

(III)

worin A die in Anspruch 1 angegebene Bedeutung besitzt, umsetzt.

19. Herstellungsverfahren gemäß Anspruch 18, dadurch gekennzeichnet, daß die verwendete Verbindung der Formel (II) hergestellt wird, indem man eine Verbindung der Formel IV :

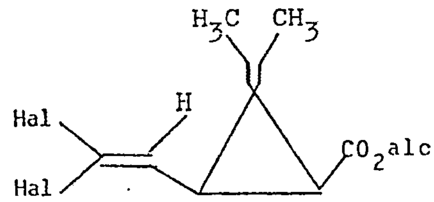

(IV)

worin die Cyclopropan-Verknüpfung 1R-cis Struktur besitzt und Hal ein Halogenatom bedeutet und alc einen Alkylrest mit 1 bis 8 Kohlenstoffatomen darstellt, zu einem ersten Zeitpunkt mit einem alkalischen Mittel umsetzt, das befähigt ist, die Halogenatome zu entreißen, und zu einem zweiten Zeitpunkt

— entweder mit einem Mittel umsetzt, das befähigt ist, die Carboxylgruppe einzuführen, um die Verbindung der Formel V :

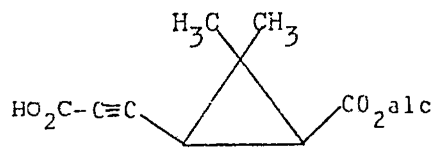

(V)

zu erhalten, die man der Einwirkung eines Veresterungsmittels unterzieht, um die Verbindung der Formel VI :

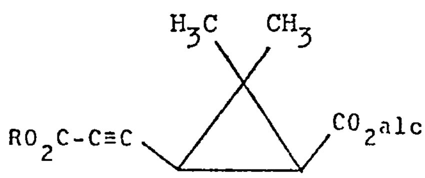

(VI)

zu erhalten, worin R die vorstehend angegebene Bedeutung besitzt,

— oder mit einem Alkylchlorformiat der Formel

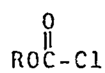

(V')

umsetzt, worin R die vorstehende Bedeutung besitzt, um direkt die Verbindung der Formel VI :

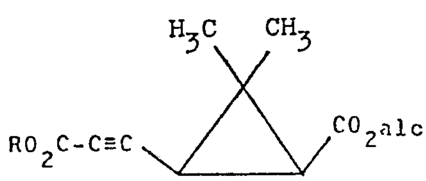

(VI)

zu erhalten, wobei man anschließend die erhaltene Verbindung der Formel (VI) der Einwirkung eines milden Hydrierungsmittels unterzieht, um die Verbindung der Formel :

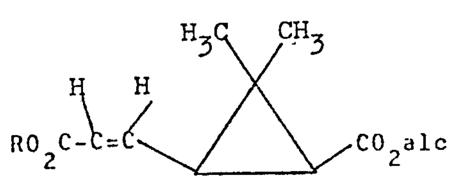

(VII)

zu erhalten, worin die Doppelbindung die Z-Geometrie besitzt, die man der Einwirkung eines sauren Hydrolysemittels unterzieht, das befähigt ist, selektiv die Esterfunktion in 1-Stellung des Cyclopropans zu spalten, um die entsprechende Verbindung der Formel II :

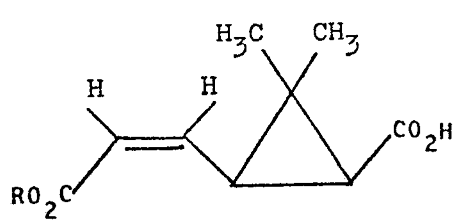

(II)

zu erhalten.

20. Verfahren gemäß Anspruch 19, dadurch gekennzeichnet, daß man eine Verbindung der Formel V :

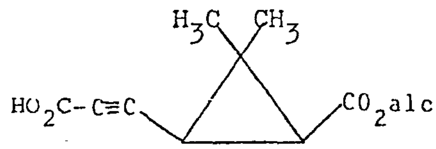

(V)

worin die Cyclopropan-Verknüpfung 1R-cis-Struktur besitzt und alc die vorstehend angegebene Bedeutung besitzt, zunächst der Einwirkung eines milden Hydrierungsmittels unterzieht, um eine Verbindung der Formel :

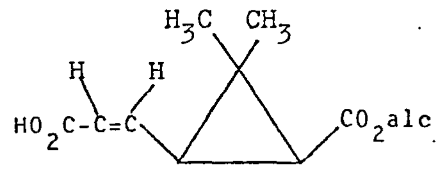

(VIII)

zu erhalten, worin die Doppelbindung die Z-Geometrie besitzt, die man der Einwirkung eines Veresterungsmittels unterzieht, um die entsprechende Verbindung der Formel VII :

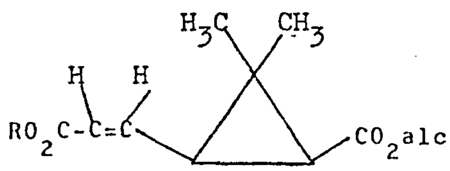

(VII)

zu erhalten, und danach die Synthese, wie in Anspruch 20 beschrieben, fortsetzt.

21. Verfahren gemäß Ansprüche 18 und 19, dadurch gekennzeichnet, daß man eine Verbindung der Formel VI :

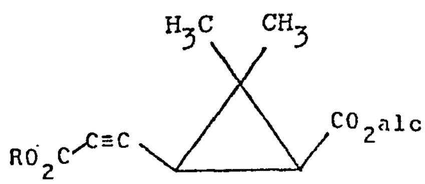

(VI)

worin die Cyclopropan-Verknüpfung 1R-cis-Struktur besitzt und R alc wie vorstehend definiet sind, der Einwirkung eines sauren Hydrolysemittels unterzieht, das befähigt ist, selektiv die Esterfunktion in 1-Stellung des Cyclopropans zu spalten, um die Verbindung der Formel (IX) :

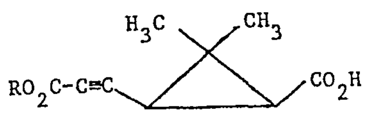

(IX)

zu erhalten, worin R wie vorstehend definiert ist, die

— man entweder gegebenenfalls in Form eines funktionellen Derivats der Einwirkung eines Alkohols der Formel (III) :

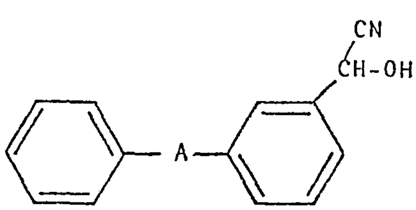

(III)

worin A die vorstehend angegebene Bedeutung besitzt, unterzieht, um eine Verbindung der Formel (X) :

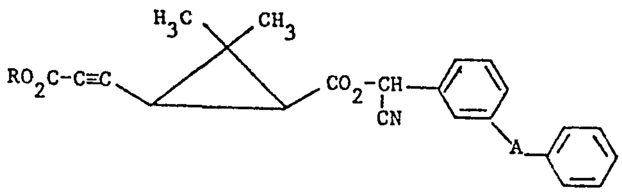

(X)

zu erhalten, worin R und A die vorstehend angegebene Bedeutung besitzen, die man der Einwirkung eines milden Hydrierungsmittels unterzieht, um die Verbindung der Formel (I) zu erhalten,

— oder zunächst der Einwirkung eines milden Hydrierungsmittels unterzieht, um die Verbindung der Formel (II) :

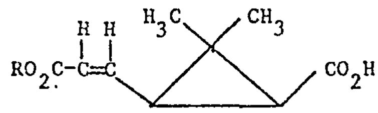

(II)

zu erhalten, worin R wie vorstehend definiert ist und die Doppelbindung die Z-Geometrie aufweist, und danach gegebenenfalls in Form eines funktionellen Derivats der Einwirkung eines Alkohols (III) unterzieht, um die Verbindung der Formel (I) zu erhalten.

22. Verfahren zur Herstellung der Verbindungen der Formel (I) gemäß einem der Ansprüche 1 bis 7, dadurch gekennzeichnet, daß man eine Verbindung der Formel (XI) :

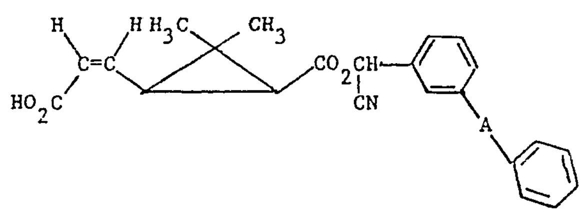

(XI)

worin die Doppelbindung die Z- Geometrie besitzt und die Cyclopropan-Verknüpfung 1R-cis-Struktur aufweist und A wie in Anspruch 1 definiert ist, der Einwirkung eines Veresterungsmittels unterzieht, um die entsprechende Verbindung der Formel (I) :

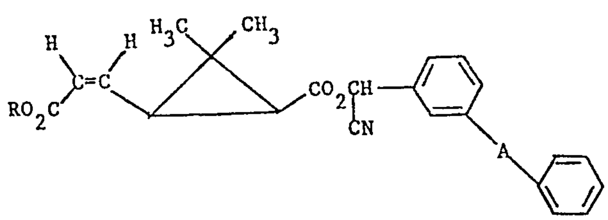

(I)

zu erhalten, worin R wie in Anspruch 1 definiert ist.

23. Verfahren gemäß Anspruch 22, dadurch gekennzeichnet, daß die Verbindung der Formel (XI) hergestellt wird, indem man eine Säure der Formel (V) :

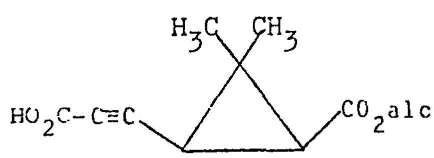

(V)

worin die Cyclopropan-Verknüpfung 1R-cis-Struktur besitzt und alc einen Alkylrest mit 1 bis 8 Kohlenstoffatomen bedeutet, der Einwirkung von 2,2,2-Trichlorethanol unterzieht, um die Verbindung der Formel (XII) :

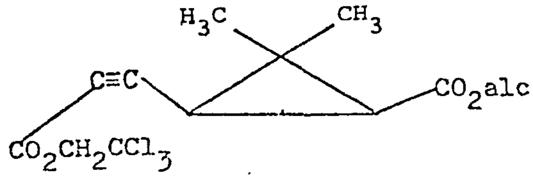

(XII)

zu erhalten, die man der Einwirkung eines sauren Hydrolysemittels unterzieht, um die Verbindung der Formel (XIII) :

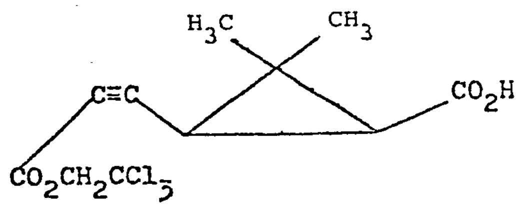

(XIII)

zu erhalten, die man der Einwirkung eines Alkohols der Formel (III) :

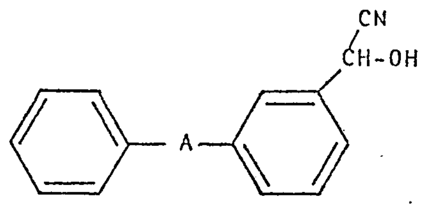

(III)

unterzieht, worin A die in Anspruch 1 angegebene Bedeutung besitzt, um die Verbindung der Formel (XIV) :

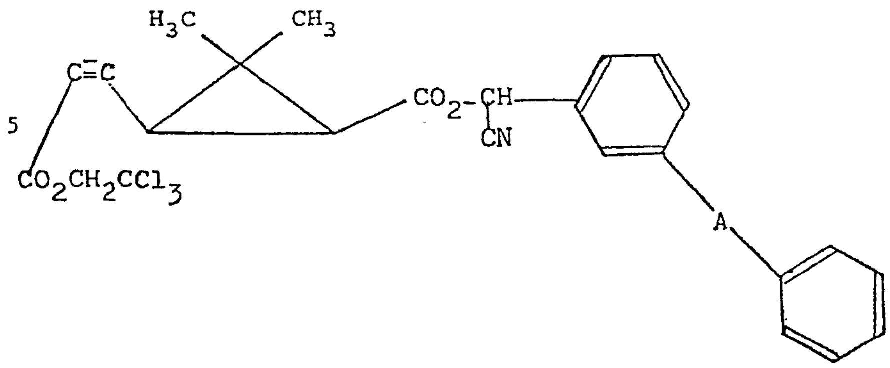

(XIV)

zu erhalten, die man der Einwirkung eines Mittels zur Spaltung der von dem acetylenischen Kohlenstoff getragenen Esterfunktion unterzieht, um die Verbindung der Formel (XV) :

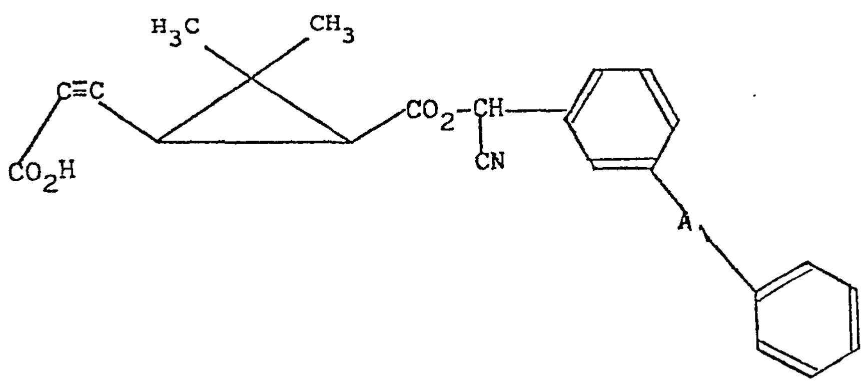

(XV)

zu erhalten, die man der Einwirkung eines milden Hydrierungsmittels unterzieht, um die Verbindung der Formel (XI) zu erhalten.

24. Verfahren gemäß Anspruch 23, dadurch gekennzeichnet, daß man eine Verbindung der Formel (XV) :

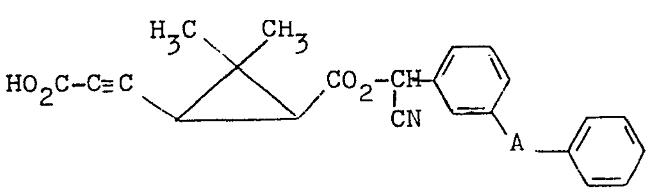

(XV)

worin die Cyclopropan-Verknüpfung 1R-cis-Struktur besitzt und A wie in Anspruch 1 definiert ist, der Einwirkung eines Veresterungsmittels unterzieht, um die Verbindung der Formel (X):

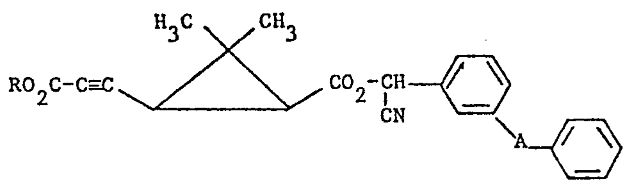

(X)

zu erhalten, worin R und A wie in Anspruch 1 definiert sind, die man der Einwirkung eines milden Hydrierungsmittels unterzieht, um die Verbindung der Formel (I) zu erhalten.

25. Als neue industrielle Produkte und insbesondere als Zwischenprodukte, die für die Durchführung des erfindungsgemäßen Verfahrens gemäß einem der Ansprüche 18 bis 21 erforderlich sind, die Verbindungen der Formeln II, V, VI, VII, VIII, IX und X gemäß den Ansprüchen 18 bis 21.

26. Als neue industrielle Produkte und insbesondere als Zwischenprodukte, die für die Durchführung des erfindungsgemäßen Verfahrens gemäß einem der Ansprüche 22 bis 24 erforderlich sind, die Verbindungen der Formeln XI, XII, XIII, XIV und XV gemäß den Ansprüchen 22 bis 24.

**Ansprüche** (für den Vertragsstaat AT)

1. Verfahren zur Herstellung der Verbindungen der Formel (I) :

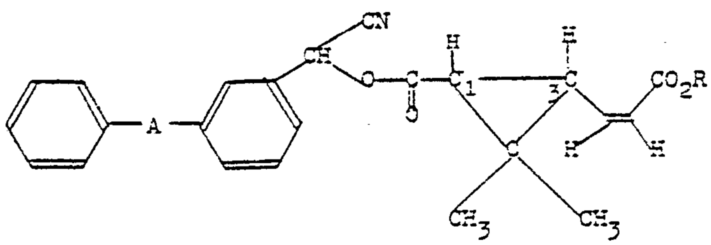

worin A ein Sauerstoffatom, eine Methylengruppe oder eine Carbonylgruppe bedeutet und R einen gesättigten oder ungesättigten, linearen, verzweigten oder cyclischen Alkylrest mit 1 bis 8 Kohlenstoffatomen darstellt, worin die Doppelbindung die Z-Geometrie aufweist und die Cyclopropan-Verknüpfung 1R-cis-Struktur besitzt, in sämtlichen ihrer möglichen stereoisomeren Formen sowie die Gemische dieser Stereoisomeren, dadurch gekennzeichnet daß man eine Säure der Formel II :

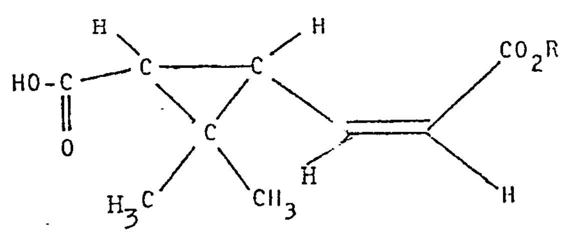

(II)

worin die Doppelbindung die Z-Geometrie besitzt und die Cyclopropan-Verknüpfung 1R-cis-Struktur aufweist und R die gleiche Bedeutung wie vorstehend angegeben besitzt, oder ein funktionelles Derivat dieser Säure mit einem Alkohol der Formel :

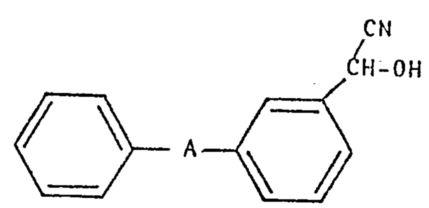

(III)

worin A die vorstehend angegebene Bedeutung besitzt, umsetzt.

2. Verfahren nach Anspruch 1, dadurch gekennzeichnet daß die verwendete Verbindung der Formel (II) hergestellt wir indem man eine Verbindung der Formel (IV) :

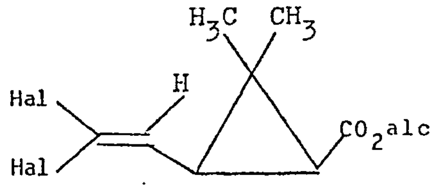

(IV)

worin die Cyclopropan-Verknüpfung 1R-cis-Struktur besitzt, Hal ein Halogenatom bedeutet und alc einen Alkylrest mit 1 bis 8 Kohlenstoffatomen darstellt, zu einem ersten Zeitpunkt mit einem alkalischen Mittel umsetzt, das befähigt ist, die Halogenatome zu entreißen, und zu einem zweiten Zeitpunkt

— entweder mit einem Mittel umsetzt, das befähigt ist, die Carboxylgruppe einzuführen, um die Verbindung der Formel V :

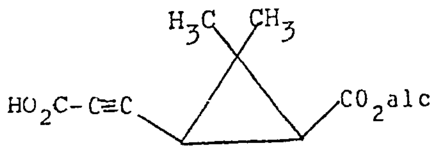

(V)

zu erhalten, die man der Einwirkung eines Veresterungsmittels unterzieht, um die Verbindung der Formel VI :

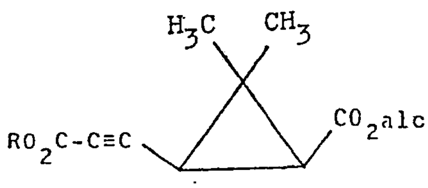

(VI)

zu erhalten, worin R die vorstehend angegebene Bedeutung besitzt,

— oder mit einem Alkylchlorformiat der Formel

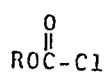

(V')

umsetzt, worin R die vorstehende Bedeutung besitzt, um direkt die Verbindung der Formel VI :

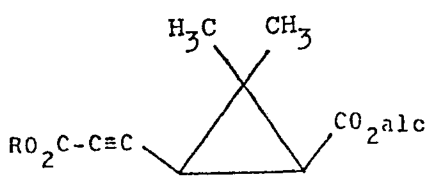

(VI)

zu erhalten, wobei man anschließend die erhaltene Verbindung der Formel (VI) der Einwirkung eines milden Hydrierungsmittels unterzieht, um die Verbindung der Formel :

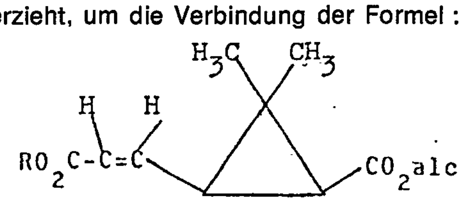

(VII)

zu erhalten, worin die Doppelbindung die Z-Geometrie besitzt, die man der Einwirkung eines sauren Hydrolysemittels unterzieht, das befähigt ist, selektiv die Esterfunktion in 1-Stellung des Cyclopropans zu spalten, um die entsprechende Verbindung der Formel II :

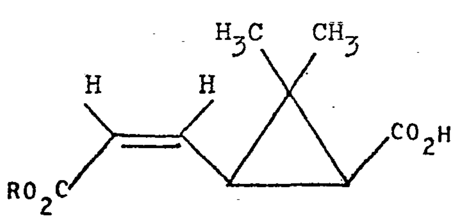

(II)

zu erhalten.

3. Verfahren nach Anspruch 2, dadurch gekennzeichnet daß man eine Verbindung der Formel (V) :

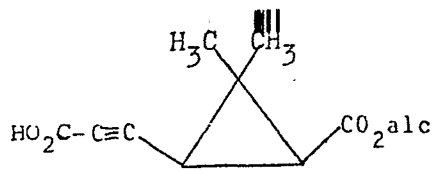

(V)

worin die Cyclopropan-Verknüpfung 1R-cis-Struktur besitzt und alc die vorstehend angegebene Bedeutung besitzt, zunächst der Einwirkung eines milden Hydrierungsmittels unterzieht, um eine Verbindung der Formel :

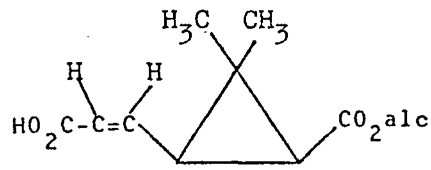

(VIII)

zu erhalten, worin die Doppelbindung die Z-Geometrie besitzt, die man der Einwirkung eines Veresterungsmittels unterzieht, um die entsprechende Verbindung der Formel (VII) :

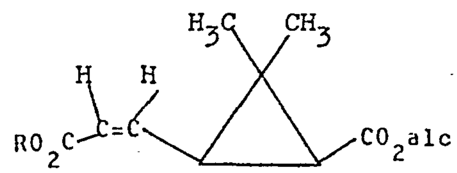

(VII)

zu erhalten, und danach die Synthese, wie in Anspruch 2 beschrieben, fortsetzt.

4. Verfahren gemäß Ansprüche 1 und 2, dadurch gekennzeichnet, daß man eine Verbindung der Formel (VI) :

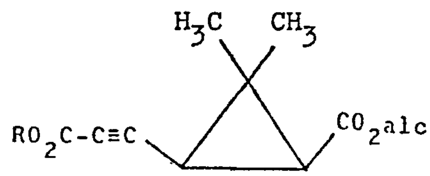

(VI)

worin die Cyclopropan-Verknüpfung 1R-cis-Struktur besitzt und R und alc wie vorstehend definiert sind, der Einwirkung eines sauren Hydrolysemittels unterzieht, das befähigt ist, selektiv die Esterfunktion in 1-Stellung des Cyclopropans zu spalten, um die Verbindung der Formel (IX) :

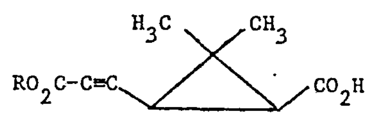

(IX)

zu erhalten, worin R wie vorstehend definiert ist, die

— man entweder gegebenenfalls in Form eines funktionellen Derivats der Einwirkung eines Alkohols der Formel (III) :

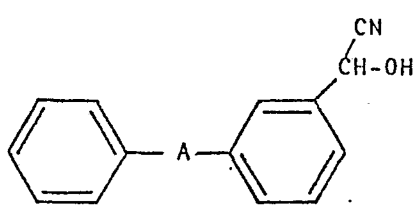

(III)

worin A die vorstehend angegebene Bedeutung besitzt, unterzieht, um eine Verbindung der Formel (X) :

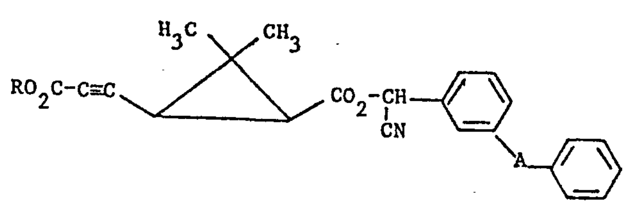

(X)

zu erhalten, worin R und A die vorstehend angegebene Bedeutung besitzen, die man der Einwirkung eines milden Hydrierungsmittels unterzieht, um die Verbindung der Formel (I) zu erhalten,

— oder zunächst der Einwirkung eines milden Hydrierungsmittels unterzieht, um die Verbindung der Formel (II) :

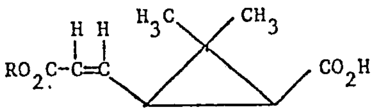

(II)

zu erhalten, worin R wie vorstehend definiert ist und die Doppelbindung die Z-Geometrie aufweist, und danach gegebenenfalls in Form eines funktionellen Derivats der Einwirkung eines Alkohols (III) unterzieht, um die Verbindung der Formel (I) zu erhalten.

5. Verfahren zur Herstellung der Verbindungen der Formel (I) gemaß Anspruch 1, dadurch gekennzeichnet, daß man eine Verbindung der Formel (XI) :

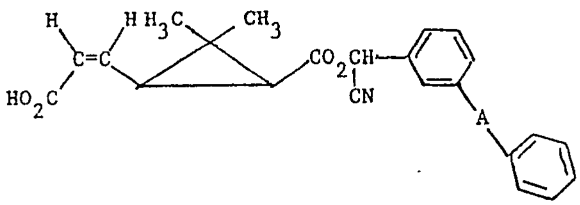

(XI)

worin die Doppelbindung die Z-Geometrie besitzt und die Cyclopropan-Verknüpfung 1R-cis-Struktur aufweist und A wie in Anspruch 1 definiert ist, der Einwirkung eines Veresterungsmittels unterzieht, um die entsprechende Verbindung der Formel (I) :

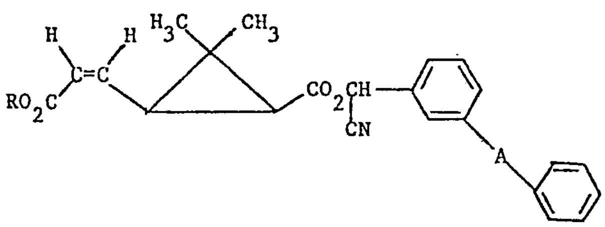

(I)

zu erhalten, worin R wie in Anspruch 1 definiert ist.

6. Verfahren gemäß Anspruch 5, dadurch gekennzeichnet, daß die Verbindung der Formel (XI) hergestellt wird, indem man eine Säure der Formel (V) :

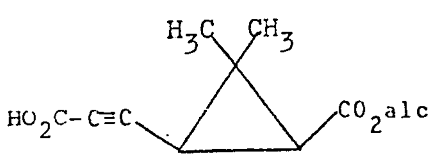

(V)

worin die Cyclopropan-Verknüpfung 1R-cis-Struktur besitzt und alc einen Alkylrest mit 1 bis 8 Kohlenstoffatomen bedeutet, der Einwirkung von 2,2,2-Trichlorethanol unterzieht, um die Verbindung der Formel (XII) :

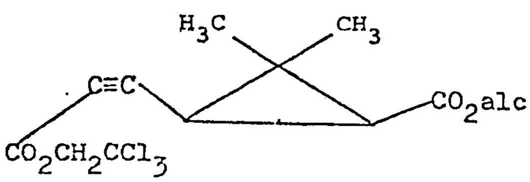

(XII)

zu erhalten, die man der Einwirkung eines sauren Hydrolysemittels unterzieht, um die Verbindung der Formel (XIII) :

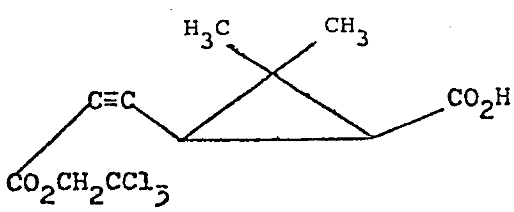

(XIII)

zu erhalten, die man der Einwirkung eines Alkohols der Formel (III) :

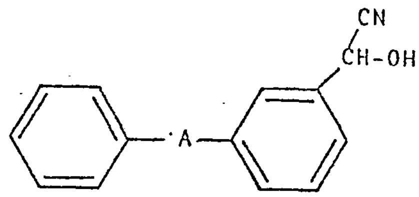

(III)

unterzieht, worin A die in Anspruch 1 angegebene Bedeutung besitzt, um die Verbindung der Formel (XIV) :

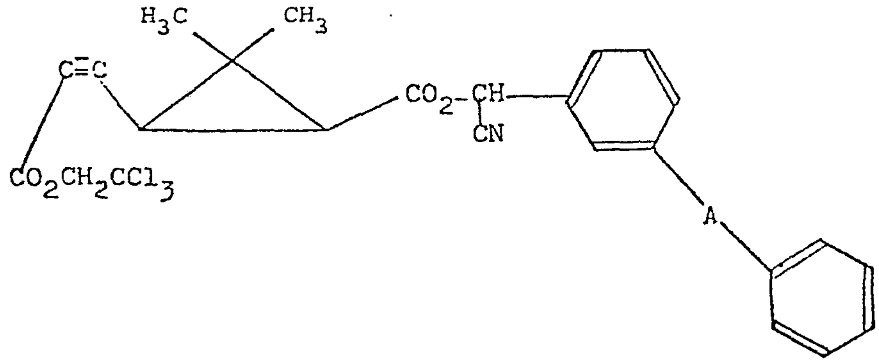

(XIV)

zu erhalten, die man der Einwirkung eines Mittels zur Spaltung der von dem acetylenischen Kohlenstoff getragenen Esterfunktion unterzieht, um die Verbindung der Formel (XV) :

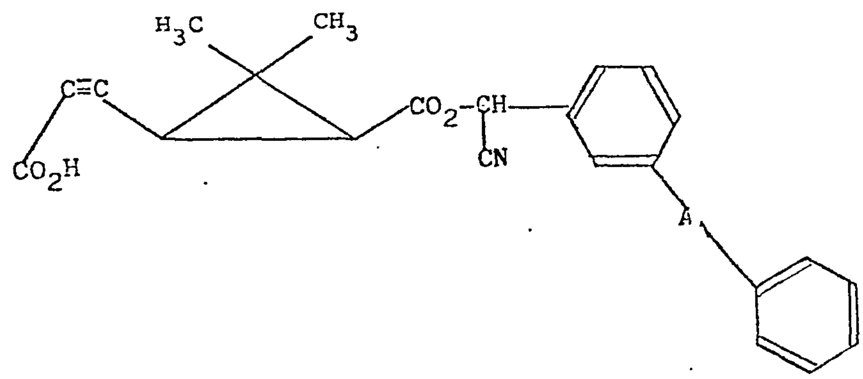

(XV)

zu erhalten, die man der Einwirkung eines milden Hydrierungsmittels unterzieht, um die Verbindung der Formel (XI) zu erhalten.

7. Verfahren gemäß Anspruch 6, dadurch gekennzeichnet, daß man eine Verbindung der Formel (XV) :

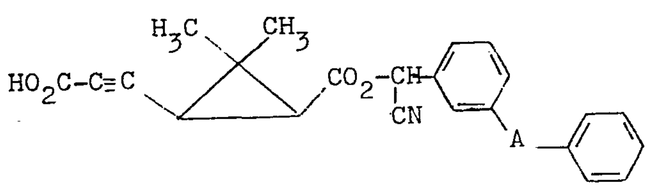

(XV)

worin die Cyclopropan-Verknüpfung 1R-cis-Struktur besitzt und A wie in Anspruch 1 definiert ist, der Einwirkung eines Veresterungsmittels unterzieht, um die Verbindung der Formel (X) :

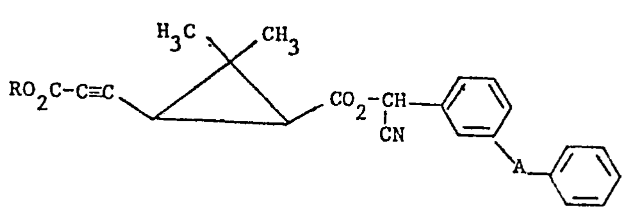

(X)

zu erhalten, worin R und A wie in Anspruch 1 definiert sind, die man der Einwirkung eines milden Hydrierungsmittels unterzieht, um die Verbindung der Formel (I) zu erhalten.

8. Verfahren gemäß einem der Ansprüche 1 bis 7 dadurch gekennzeichnet daß A ein Sauerstoffatom bedeutet und daß der Kohlenstoffträger des Zyanradikals in der alkoholische Verknüpfung (S)-Konfiguration aufweist.

9. Verfahren gemäß einem der Ansprüche 1 bis 7 dadurch gekennzeichnet daß R einen Methyl-, Ethyl-, Propyl-, Isopropyl-, Butyl-, oder tert.-Butylrest bedeutet.

10. Verfahren gemäß einem der Ansprüche 1 bis 7 dadurch gekennzeichnet daß R einen Cyclopropyl- oder Cyclopropylmethylrest bedeutet.

11. Verfahren gemäß einem der Ansprüche 1 bis 7 dadurch gekennzeichnet daß man eine der Verbindungen der Formel (I) mit den folgenden Bezeichnungen, hergestellt :

— (S)-α-Cyano-3-phenoxybenzyl-(1R-cis)-2,2-dimethyl-3-[(Z)-2-(methoxycarbonyl)-ethenyl]-cyclopropancarboxylat,

— (S)-α-Cyano-3-phenoxybenzyl-(1R-cis)-2,2-dimethyl-3-[(Z)-2-(ethoxycarbonyl)-ethenyl)-cyclopropancarboxylat,

— (S)-α-Cyano-3-phenoxybenzyl-(1R-cis)-2,2-dimethyl-3-[(Z)-2-(isopropyloxycarbonyl)-ethenyl]-cyclopropancarboxylat,

— (S)-α-Cyano-3-phenoxybenzyl-(1R-cis)-2,2-dimethyl-3-[(Z)-2-(cyclopropylmethoxycarbonyl)-ethenyl]-cyclopropancarboxylat,

— (S)-α-Cyano-3-phenoxybenzyl-(1R-cis)-2,2-dimethyl-3-[(Z)-2-(cyclopropyloxycarbonyl)-ethenyl]-cyclopropancarboxylat,

— (S)-α-Cyano-3-phenoxybenzyl-(1R-cis)-2,2-dimethyl-3-[(Z)-2-(tert.-butoxycarbonyl)-ethenyl]-cyclopropancarboxylat.

12. Verwendung der Verbindungen der Formel (I) gemäß Anspruch 1 zur Bekämpfung von Parasiten der Pflanzen und Parasiten im häuslichen Bereich.

13. Zusammensetzungen zur Bekämpfung von Parasiten der Pflanzen, parasiten im häuslichen Bereich und Parasiten warmblütiger Tiere, dadurch gekennzeichnet, daß sie zumindest eines der Produkte gemäß Anspruch 1 enthalten.

14. Insektizide Zusammensetzungen, enthaltend als Wirkstoff eines der Produkte gemäß Anspruch 1.

15. Insektizide Zusammensetzungen, enthaltend als Wirkstoff eines der Produkte gemäß Anspruch 11.

16. Zusammensetzungen für die Bekämpfung von parasitären Milben der Pflanzen, dadurch gekennzeichnet, daß sie zumindest eines der Produkte gemäß Anspruch 1 enthalten.

17. Zusammensetzungen zur Bekämpfung von parasitären Milben der Pflanzen, dadurch gekennzeichnet, daß sie als Wirkstoff das (S)-α-Cyano-3-phenoxybenzyl-(1R-cis)-2,2-dimethyl-3-[(Z)-2-(tert.-butoxycarbonyl)-ethenyl]-cyclopropancarboxylat enthalten.

18. Zusammensetzungen für die tierische Ernährung, enthaltend als Wirkstoff zumindest eines der Produkte gemäß Anspruch 1 zusammen mit einen für die tierischen Ernährung bestimmten Nährmittel.

19. Mit insektizider, akarizider oder nematizider Aktivität ausgestattete Assoziationen, dadurch gekennzeichnet, daß sie als Wirkstoff einesteils zumindest eine der Verbindungen der allgemeinen Formel (I) und anderenteils zumindest einen der Pyrethrinoidester enthalten, ausgewählt unter den Estern der Allethrolone, des 3,4,5,6-Tetrahydrophthalimido-methylalkohols, des 5-Benzyl-3-furyl-methylalkohols, des 3-Phenoxybenzylalkohols und der α-Cyano-3-phenoxybenzylalkohole der Chrysanthemumsäuren, den Estern des 5-Benzyl-3-furylmethylalkohols der 2,2-Dimethyl-3-(2-oxo-3-tetrahydrothiophenylidenmethyl)-cyclopropan-1-carbonsäuren, den Estern des 3-Phenoxybenzylalkohols und der α-Cyano-3-phenoxybenzylalkohole der 2,2-Dimethyl-3-(2,2-dichlorvinyl)-cyclopropan-1-carbonsäuren, den Estern der α-Cyano-3-phenoxybenzylalkohole der 2,2-Dimethyl-3-(2,2-dibromvinyl)-cyclopropan-1-carbonsäuren, den Estern des 3-Phenoxybenzylalkohols der 2-p-Chlorphenyl-2-isopropylessigsäuren, den Estern der Allethrolone, des 3,4,5,6-Tetrahydrophthalimidomethylalkohols, des 5-Benzyl-3-furylmethylalkohols, des 3-Phenoxybenzylalkohols und der α-Cyano-3-phenoxybenzylalkohole der 2,2-Dimethyl-3-(1,2,2,2-tetrahalo)-cyclopropan-1-carbonsäuren, worin « halo » ein Fluor-, Chlor- oder Bromatom bedeutet, wobei die Verbindungen I in sämtlichen ihrer möglichen stereoisomeren Formen vorliegen können ebenso wie die Säure- und Alkoholkomponenten der vorstehenden Pyrethrinoidester.